# EUROPEAN PATENT APPLICATION

(11) **EP 4 567 110 A1**
(43) Date of publication of application: **11.06.2025**
(21) Application number: 23850101.9
(22) Date of filing: 02.08.2023
(51) Int. Cl.: C12N 15/11

(54) **METHOD FOR PRODUCING OLIGONUCLEIC ACID**

(30) Priority: 02.08.2022 JP 2022123036
(71) Applicant: AJINOMOTO CO., INC., Chuo-ku Tokyo 104-8315 (JP)
(72) Inventor: KAJIMOTO, Shohei, Kawasaki-shi, Kanagawa 210-8681 (JP); HAGIWARA, Yusuke, Kawasaki-shi, Kanagawa 210-8681 (JP)
(74) Representative: Mewburn Ellis LLP
(86) International application number: PCT/JP2023/028214
(87) International publication number: WO 2024/029547

(57) **Abstract**

A method of producing an oligonucleotide is provided. By using a complementary strand into which a moiety that lowers the stability of a hybrid formed between a complementary strand and a ligation product is introduced, a single-stranded oligonucleotide which is a substrate is enzymatically ligated to produce an oligonucleotide which is the ligation product.

## Description

### Technical Field

The present invention relates to a method of producing an oligonucleotide.

### Background Art

The usefulness of oligonucleotides such as siRNAs and antisense nucleic acids for nucleic acid medicines has been shown, and, in recent years, development of such oligonucleotides has been stepped up. Such oligonucleotides have been primarily produced by chemical synthesis methods. Such an oligonucleotide can be produced by elongation synthesis of nucleic acid residues on a one-by-one basis in a serial manner in turn by, for example, a synthesis method such as a phosphoroamidite method. However, this method has problems in that, for example, the longer chain length of the oligonucleotide results in the lower purity and yield of a product, and production efficiency is low. Thus, a parallel synthesis method by which the short-chain fragments of an oligonucleotide are synthesized and ligated to obtain a long-chain oligonucleotide has been demanded.

An oligonucleotide can be produced by, for example, enzymatically ligating nucleic acid fragments using complementary strands.

Moreover, a technology of introducing destabilizing moieties such as abasic sites into complementary strands or nucleic acid fragments, and using the complementary strands or the nucleic acid fragments in a method of amplifying a DNA sequence, the method including enzymatically ligating the nucleic acid fragments in the presence of the complementary strands, has been reported (Patent Literature 1 and Non Patent Literature 1).

Moreover, a technology of introducing destabilizing elements such as abasic sites and mismatch sites into two complementary strands, and using the complementary strands in a method of detecting a single-stranded DNA, the method including enzymatically ligating the complementary strands which are probes in the presence of a single-stranded DNA, has been reported (Non Patent Literature 2).

### Citation List

### Patent Literature

Patent Literature 1: US 9,193,993 B1

### Non Patent Literature

Non Patent Literature 1: Abu Kausar et al., Tuning DNA stability to achieve turnover in template for an enzymatic ligation reaction, Angew Chem Int Ed Engl. 2011 Sep 12;50(38):8922-6.
Non Patent Literature 2: BS Alladin-Mustan et al., Achieving room temperature DNA amplification by dialling in destabilization, Chem. Commun., 2015, 51, 9101-9104.

### Summary of Invention

### Technical Problem

An objective of the present invention is to provide a method of producing an oligonucleotide.

### Solution to Problem

The present inventors intensively studied to solve the above-described problems, consequently found that a ligation product can be efficiently produced by using a complementary strand into which a moiety that lowers the stability of a hybrid formed between the complementary strand and the ligation product is introduced in the case of enzymatically ligating single-stranded oligonucleotides which are substrates to produce an oligonucleotide which is the ligation product, and completed the present invention.

In other words, the present invention can be exemplified as follows.
[1] A method of producing an oligonucleotide of interest, the method comprising:
   a step of performing enzymatic ligation of N single-stranded substrate oligonucleotides, the N being an integer 2 or more, in presence of M single-stranded complementary oligonucleotides, the M being an integer 1 or more, to generate the oligonucleotide of interest,
   wherein ligation between the substrate oligonucleotides on 5' and 3' sides of each of ligation sites is performed in presence of the one or more complementary oligonucleotides corresponding to the ligation sites,
   wherein each of the complementary oligonucleotides comprises a base sequence in which a destabilizing site is introduced into a first base sequence,
   wherein the first base sequence is a base sequence consisting of a second base sequence and a third base sequence that are ligated in 5' to 3' direction,
   wherein the second base sequence is a complementary sequence of a partial sequence on a 5' side of the substrate oligonucleotide on the 3' side, or a complementary sequence of a full-length sequence of the substrate oligonucleotide on the 3' side,
   wherein the third base sequence is a complementary sequence of a partial sequence on a 3' side of the substrate oligonucleotide on the 5' side, or a complementary sequence of a full-length sequence of the substrate oligonucleotide on the 5' side, and
   wherein the destabilizing site is a moiety that lowers stability of a hybrid formed between each of the complementary oligonucleotide and the oligonucleotide of interest.
[2] The method, wherein the method excludes cases (1) to (5) described below:
   (1) a case in which the N is 2, and the destabilizing site consists of an abasic site at position -1;
   (2) a case in which the N is 2, and the destabilizing site consists of a substitution of a linker between positions -1 and +1 with a nucleic acid residue;
   (3) a case in which the N is 2, and the destabilizing site consists of a substitution of a nucleic acid residue at position -1 with a linker;
   (4) a case in which the N is 2, and the destabilizing site consists of a combination of an abasic site at position -1 and an abasic site at position +4; and
   (5) a case in which the N is 2, and the destabilizing site consists of a combination of an abasic site at position -1 and a mismatch site at position +4.
[3] The method, wherein the destabilizing site comprises an abasic site, a mismatch site, an insertion of a nucleic acid residue, a deletion of a nucleic acid residue, an insertion of a linker, a deletion of a linker, a substitution of a linker, or a combination thereof.
[4] The method, wherein the destabilizing site comprises an insertion of a nucleic acid residue without a deletion of a linker and/or a deletion of a nucleic acid residue without an insertion of a linker.
[5] The method, wherein the destabilizing site consists of one to twenty sets of destabilizing sites.
[6] The method, wherein the destabilizing site consists of one to eight sets of destabilizing sites.
[7] The method, wherein the destabilizing site consists of one to four sets of destabilizing sites.
[8] The method, wherein the destabilizing site consists of destabilizing sites whose number of sets is 30% or less as a proportion with respect to a length of the first base sequence.
[9] The method, wherein the destabilizing site comprises one to eight abasic sites.
[10] The method, wherein the destabilizing site comprises one to two abasic sites.
[11] The method, wherein the destabilizing site comprises abasic sites whose number is 30% or less as a proportion with respect to the length of the first base sequence.
[12] The method, wherein the destabilizing site comprises one to eight mismatch sites.
[13] The method, wherein the destabilizing site comprises one to three mismatch sites.
[14] The method, wherein the destabilizing site comprises mismatch sites whose number is 30% or less as a proportion with respect to the length of the first base sequence.
[15] The method, wherein the destabilizing site comprises deletions of nucleic acid residues whose number is 50% or less as a proportion with respect to the length of the first base sequence.
[16] The method, wherein the destabilizing site comprises one to eight sets of deletions of nucleic acid residues.
[17] The method, wherein the destabilizing site comprises one to three sets of deletions of nucleic acid residues.
[18] The method, wherein each set of the deletions of the nucleic acid residues consists of one to three deletions of nucleic acid residues.
[19] The method, wherein the destabilizing site comprises insertions of nucleic acid residues whose number is 200% or less as a proportion with respect to the length of the first base sequence.
[20] The method, wherein the destabilizing site comprises one to eight sets of insertions of nucleic acid residues.
[21] The method, wherein the destabilizing site comprises one to four sets of insertions of nucleic acid residues.
[22] The method, wherein each set of the insertions of the nucleic acid residues consists of one to ten insertions of nucleic acid residues.
[23] The method, wherein in a case in which each set of the insertions of the nucleic acid residues consists of two or more insertions of nucleic acid residues, a part or all of the nucleic acid residues have self-complementarity.
[24] The method, wherein the destabilizing site comprises one to eight insertions of linkers.
[25] The method, wherein the destabilizing site comprises one to eight deletions of linkers.
[26] The method, wherein the destabilizing site comprises one to eight substitutions of linkers.
[27] The method, wherein the destabilizing site comprises (a), (b), or (c) described below:
   (a) a combination of an abasic site at positions -1 to +1 and a deletion and/or insertion of a nucleic acid residue at a position other than the positions -1 to +1;
   (b) a combination of a mismatch site at positions -1 to +1 and a deletion and/or insertion of a nucleic acid residue at a position other than the positions -1 to +1; and
   (c) a mismatch site at positions -1 to +1.
[28] The method, wherein the destabilizing sites (a), (b), and (c) are (a1), (b1), and (c1) described below, respectively:
   (a1) a combination of an abasic site at positions -1 to +1 and one or two sets of deletions of nucleic acid residues and/or one or two sets of insertions of nucleic acid residues at positions other than the positions -1 to +1;
   (b1) a combination of a mismatch site at positions -1 to +1 and one or two sets of deletions of nucleic acid residues and/or one or two sets of insertions of insertions at positions other than the positions -1 to +1; and
   (c1) a mismatch site at positions -1 to +1.
[29] The method, wherein the destabilizing sites (a), (b), and (c) are (a2), (b2), and (c2) described below, respectively:
   (a2) a combination of an abasic site at positions -1 to +1, one set of insertions of nucleic acid residues at negative positions other than the position -1, and one set of insertions of nucleic acid residues at positive positions other than the position +1;
   (b2) a combination of a mismatch site at positions -1 to +1, one set of insertions of nucleic acid residues at negative positions other than the position -1, and one set of insertions of nucleic acid residues at positive positions other than the position +1; and
   (c2) a mismatch site at positions -1 to +1.
[30] The method, wherein the destabilizing site lowers a melting temperature of the hybrid by 1 to 60°C.
[31] The method, wherein the oligonucleotide of interest has a length of 10 to 200 residues.
[32] The method, wherein each of the substrate oligonucleotides has a length of 5 to 50 residues.
[33] The method, wherein each of the second and third base sequences has a length of 5 to 50 residues.
[34] The method, wherein a length of each of the complementary oligonucleotides is 5 to 300% as a proportion in a case in which the length of the oligonucleotide of interest is taken as 100%.
[35] The method, wherein the length of each of the complementary oligonucleotides is 5 to 300% as a proportion in a case in which a total of the lengths of the substrate oligonucleotides on the 5' side and the 3' side is taken as 100%.
[36] The method, wherein five or more residues of nucleic acid residues included in the second base sequence remain, and five or more residues of nucleic acid residues included in the third base sequence remain in each of the complementary oligonucleotides.
[37] The method, wherein 50% or more of the nucleic acid residues included in the second base sequence remain, and 50% or more of the nucleic acid residues included in the third base sequence remain in each of the complementary oligonucleotides.
[38] The method, wherein three or more consecutive residues of the nucleic acid residues included in the second base sequence remain, and three or more consecutive residues of the nucleic acid residues included in the third base sequence remain in each of the complementary oligonucleotides.
[39] The method, wherein the oligonucleotide of interest consists of a DNA residue, an RNA residue, a nucleic acid residue subjected to a modification, or a combination thereof.
[40] The method, wherein the oligonucleotide of interest is subjected to a modification.
[41] The method, wherein any one or more of the complementary oligonucleotides are subjected to a modification.
[42] The method, wherein the modification comprises a modification of a phosphate portion, a modification of a sugar portion, a modification of a base portion, or a combination thereof.
[43] The method, wherein the modification of the phosphate portion comprises phosphorothioation, boranophosphation, insertion of a linker, or a combination thereof.
[44] The method, wherein the modification of the sugar portion comprises 2'-MOE, 2'-OMe, 2'-F, 4'-thio- 2'-OMe, crosslinkage between positions 2' and 4' of a sugar portion, a modification of a 5' end of an oligonucleotide, a modification of a 3' end of an oligonucleotide, or a combination thereof.
[45] The method, wherein the N is 2.
[46] The method, wherein the N is 3 or more.
[47] The method, wherein the N is 10 or less.
[48] The method, wherein the M is less than N - 1.
[49] The method, wherein the M is N - 1.
[50] The method, wherein the M is more than N - 1.
[51] The method, wherein the step is carried out at 5 to 60°C.
[52] The method, wherein a used amount of each of the complementary oligonucleotides is 50% or less, by molar ratio, of a used amount of the substrate oligonucleotide on the 5' side or a used amount of the substrate oligonucleotide on the 3' side, whichever is less.
[53] The method, wherein a concentration of each of the substrate oligonucleotides in a reaction liquid in the step is 1 to 10000 µM.
[54] The method, wherein an enzyme used in the enzymatic ligation is a T3 DNA ligase.

### Brief Description of Drawings

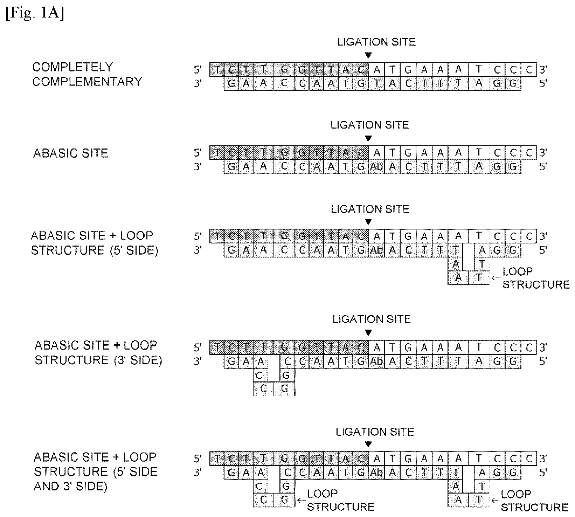

[Figure 1A] Figure 1A is a view illustrating the structures of oligonucleotides (5'-end substrates, 3'-end substrates, and complementary strands) used in Examples and the presumed hybridization structures of the oligonucleotides. In each hybridization structure, substrates are illustrated in the upper side, and a complementary strand is illustrated in the lower side. "Ab" denotes an abasic site.

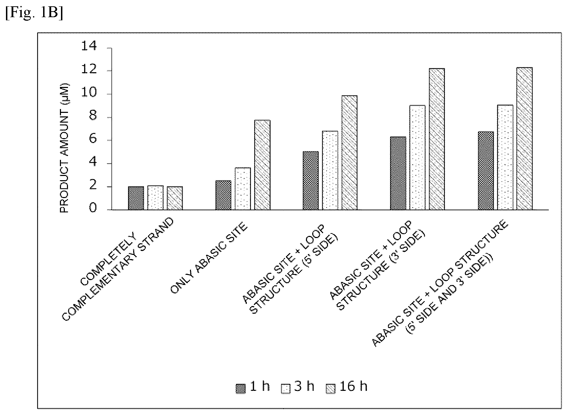

[Figure 1B] Figure 1B is a view illustrating the amount of a product in the case of performing ligation reaction of two substrates using a complementary strand into which an abasic site is introduced, or a complementary strand into which an abasic site and a loop structure are introduced.

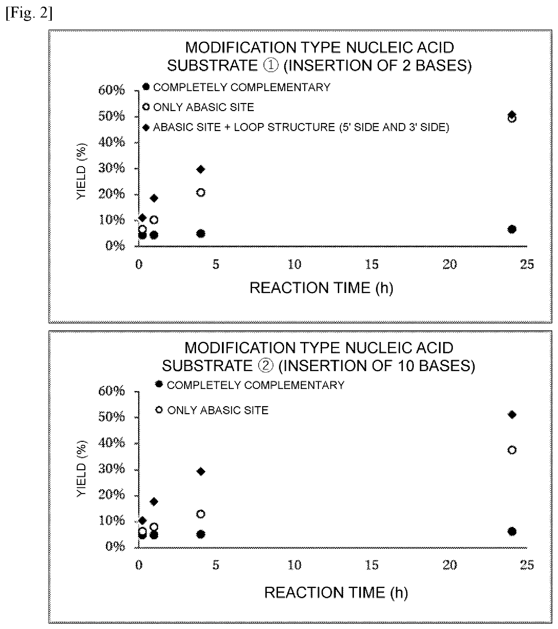

[Figure 2] Figure 2 is a view illustrating yield of a product in the case of performing ligation reaction of two substrates of which the sugar portions are modified using a complementary strand into which an abasic site is introduced, or a complementary strand into which an abasic site and a loop structure are introduced.

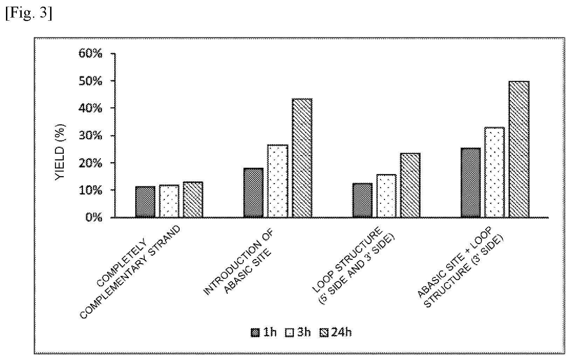

[Figure 3] Figure 3 is a view illustrating the yield of a product in the case of performing ligation reaction of two substrates of which the phosphate portions are modified using a complementary strand into which an abasic site is introduced, a complementary strand into which a loop structure is introduced, or a complementary strand into which an abasic site and a loop structure are introduced.

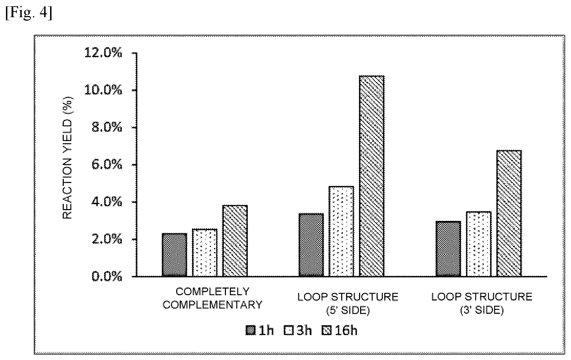

[Figure 4] Figure 4 is a view illustrating the yield of a product in the case of performing ligation reaction of two substrates using a complementary strand into which a loop structure is introduced.

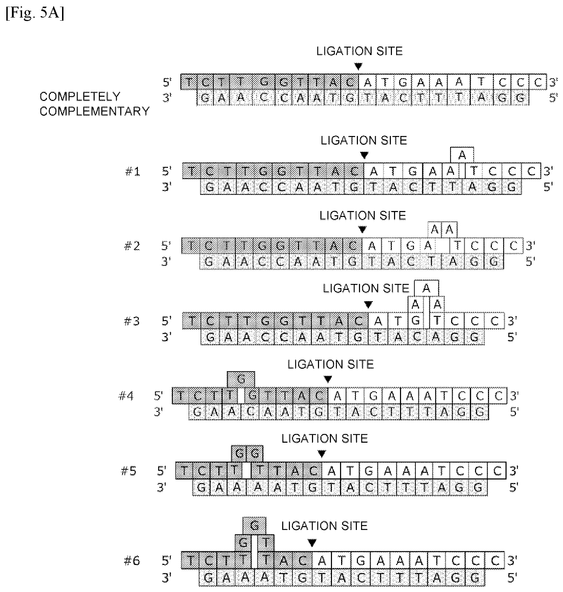

[Figure 5A] Figure 5A is a view illustrating the structures of the oligonucleotides used in Examples and the presumed hybridization structures of substrates and complementary strands. In each hybridization structure, a substrate is illustrated in the upper side, and a complementary strand is illustrated in the lower side.

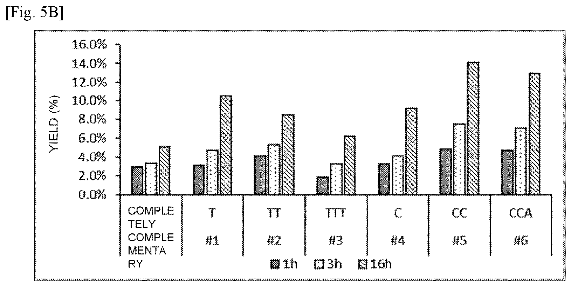

[Figure 5B] Figure 5B is a view illustrating the yield of a product in the case of performing ligation reaction of two substrates using a complementary strand into which one or more deletions of nucleic acid residues are introduced.

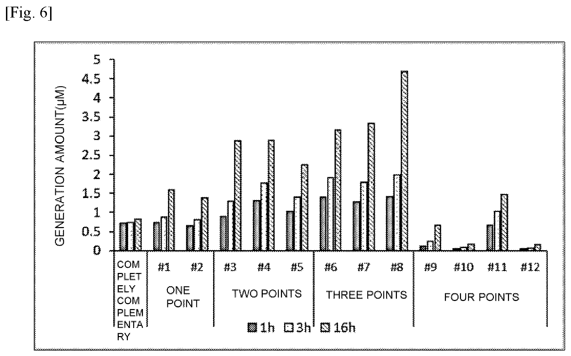

[Figure 6] Figure 6 is a view illustrating the yield of a product in the case of performing ligation reaction of two substrates using a complementary strand into which one or more loop structures are introduced.

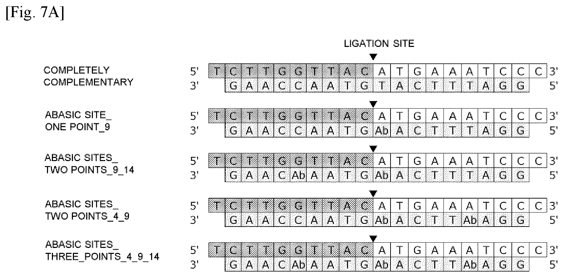

[Figure 7A] Figure 7A is a view illustrating the structures of the oligonucleotides (5'-end substrates, 3'-end substrates, and complementary strands) used in Examples and the presumed hybridization structures of the oligonucleotides. In each hybridization structure, substrates are illustrated in the upper side, and a complementary strand is illustrated in the lower side. "Ab" denotes an abasic site.

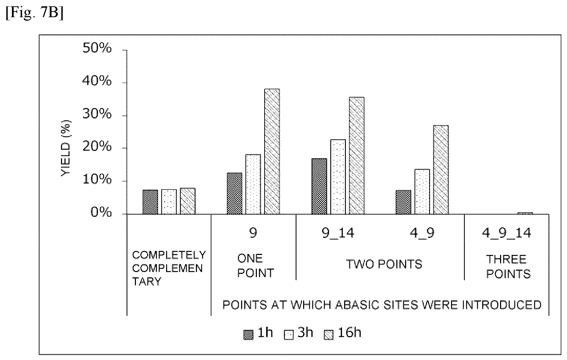

[Figure 7B] Figure 7B is a view illustrating the yield of a product in the case of performing ligation reaction of two substrates using a complementary strand into which one or more abasic sites are introduced.

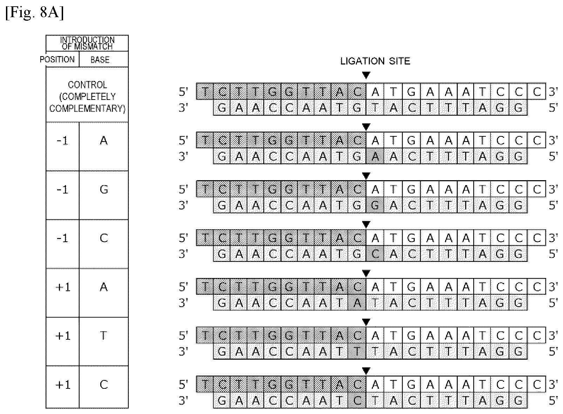

[Figure 8A] Figure 8A is a view illustrating the structures of the oligonucleotides (5'-end substrates, 3'-end substrates, and complementary strands) used in Examples and the presumed hybridization structures of the oligonucleotides. In each hybridization structure, substrates are illustrated in the upper side, and a complementary strand is illustrated in the lower side.

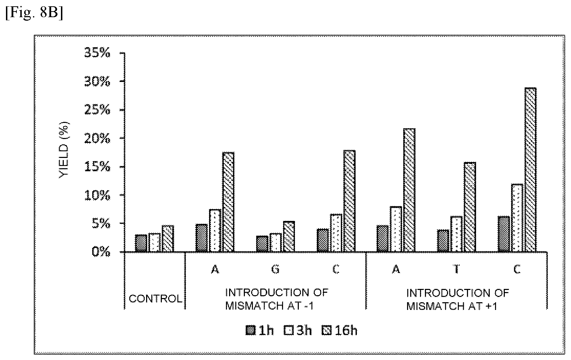

[Figure 8B] Figure 8B is a view illustrating the yield of a product in the case of performing ligation reaction of two substrates using a complementary strand into which a mismatch site is introduced.

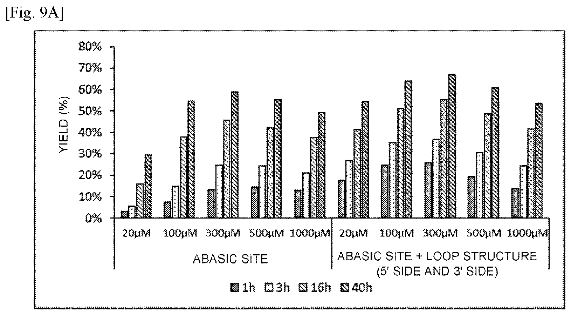

[Figure 9A] Figure 9A is a view illustrating the yield of a product in the case of performing ligation reaction of two substrates using a complementary strand into which an abasic site is introduced, or a complementary strand into which an abasic site and a loop structure are introduced at various substrate concentrations and complementary strand concentrations. A concentration on the horizontal axis indicates such a substrate concentration (common to a 5'-end substrate and a 3'-end substrate). Such a complementary strand concentration is 1/10 of the substrate concentration.

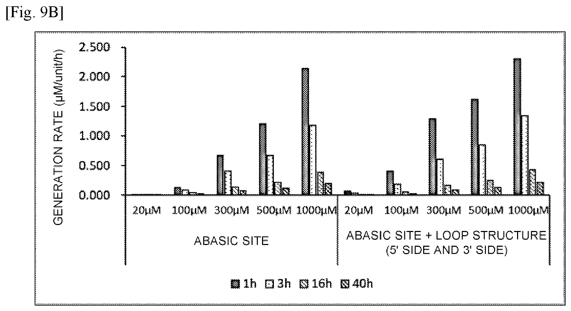

[Figure 9B] Figure 9B is a view illustrating the generation rate of a product per enzymatic activity in the case of performing ligation reaction of two substrates using a complementary strand into which an abasic site is introduced, or a complementary strand into which an abasic site and a loop structure are introduced at various substrate concentrations and complementary strand concentrations. A concentration on the horizontal axis indicates such a substrate concentration (common to a 5'-end substrate and a 3'-end substrate). Such a complementary strand concentration is 1/10 of the substrate concentration.

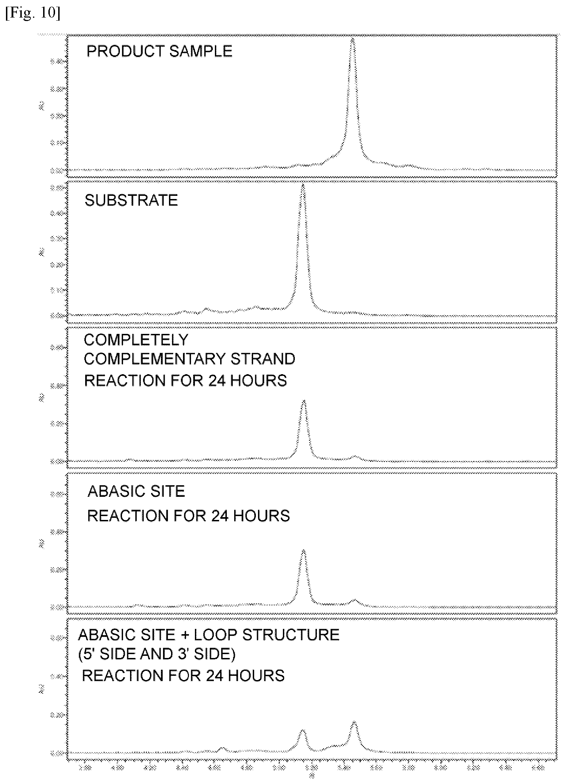

[Figure 10] Figure 10 is a view illustrating generation of a product in the case of performing ligation reaction of two substrates each including 50 residues using a complementary strand into which an abasic site is introduced, or a complementary strand into which an abasic site and a loop structure are introduced.

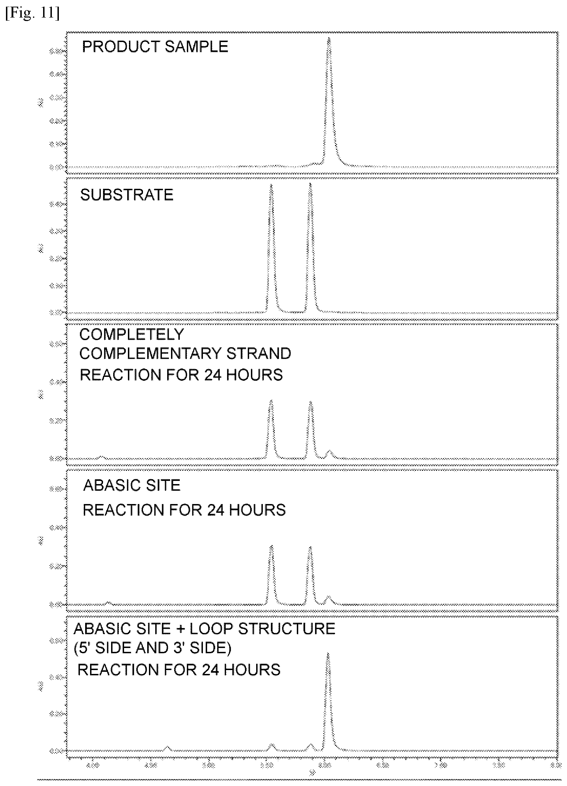

[Figure 11] Figure 11 is a view illustrating generation of a product in the case of performing ligation reaction of two substrates each including 50 residues including modified nucleic acids using a complementary strand into which an abasic site is introduced, or a complementary strand into which an abasic site and a loop structure are introduced.

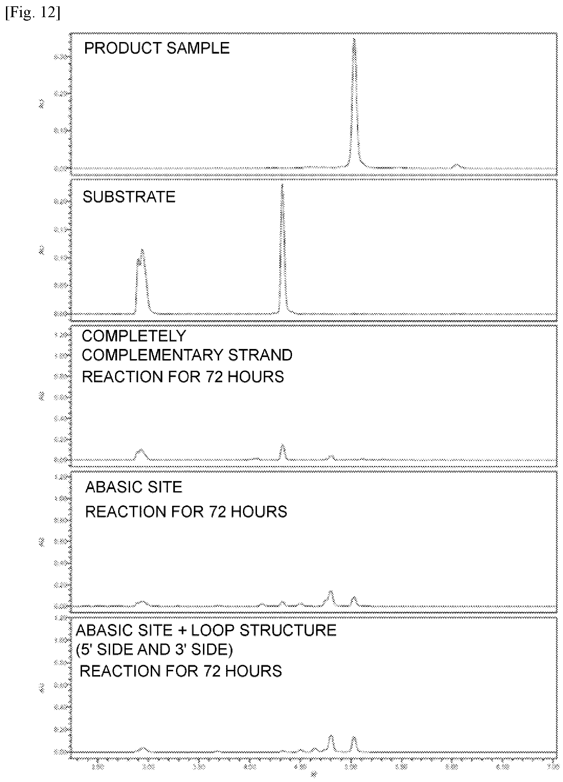

[Figure 12] Figure 12 is a view illustrating generation of a product in the case of performing ligation reaction of three substrates using a complementary strand into which an abasic site is introduced, or a complementary strand into which an abasic site and a loop structure are introduced.

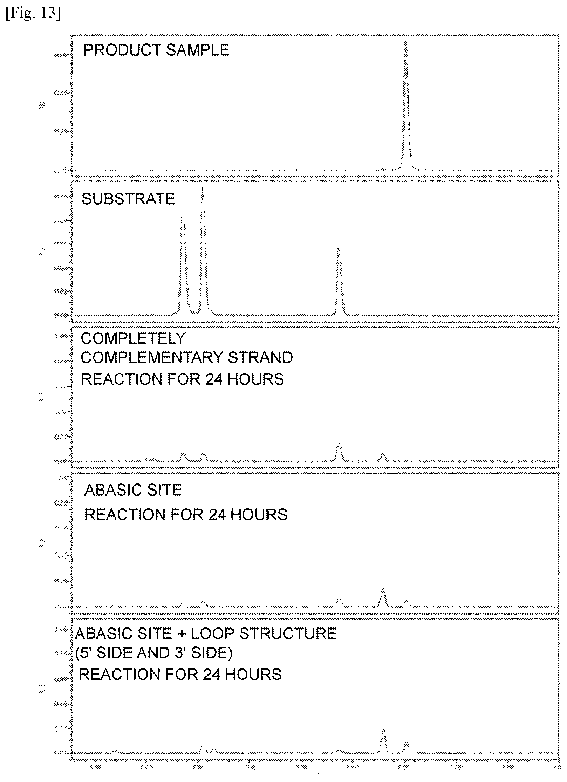

[Figure 13] Figure 13 is a view illustrating generation of a product in the case of performing ligation reaction of three substrates including modified nucleic acids using a complementary strand into which an abasic site is introduced, or a complementary strand into which an abasic site and a loop structure are introduced.

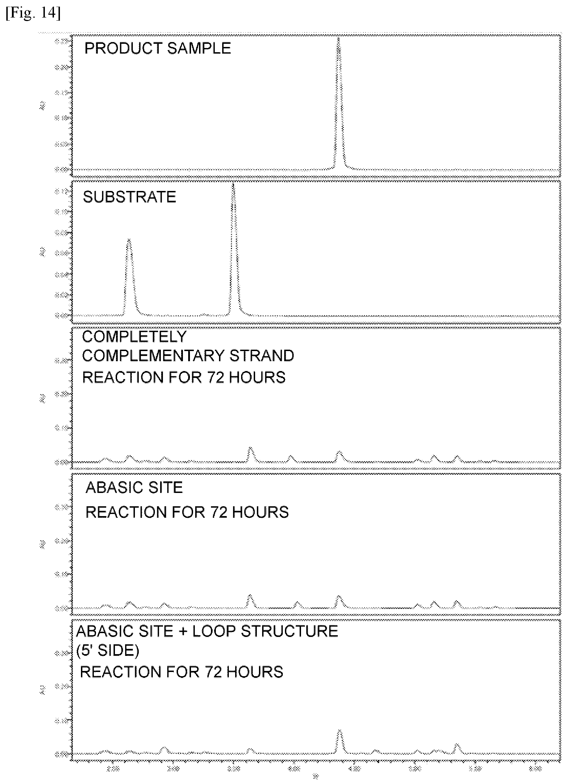

[Figure 14] Figure 14 is a view illustrating generation of a product in the case of performing ligation reaction of two substrates including modified nucleic acids and RNAs using a complementary strand into which an abasic site is introduced, or a complementary strand into which an abasic site and a loop structure are introduced.

### Description of Embodiments

The present invention is described in detail below.

A method of the present invention is a method of ligating single-stranded oligonucleotides. Each of the single-stranded oligonucleotides that are ligated is also referred to as "single-stranded substrate oligonucleotide" or "substrate oligonucleotide".

An oligonucleotide in which the substrate oligonucleotides are ligated is generated by the ligation of the substrate oligonucleotides. The oligonucleotide that is generated is also referred to as "oligonucleotide of interest". Thus, the method of the present invention may be a method of producing an oligonucleotide of interest.

The method of the present invention comprises a step of performing enzymatic ligation of N substrate oligonucleotides, the N being an integer 2 or more. The step is also referred to as "ligation step". The ligation step may be a step of performing enzymatic ligation of N substrate oligonucleotides, the N being an integer 2 or more, to generate an oligonucleotide of interest. The N substrate oligonucleotides, the N being an integer 2 or more, that are ligated in the ligation step are also generically referred to as "set of substrate oligonucleotides".

The ligation step is carried out in the presence of the single-stranded oligonucleotides having a certain structure. Such a single-stranded oligonucleotide having the certain structure is also referred to as "single-stranded complementary oligonucleotide" or "complementary oligonucleotide". Specifically, the ligation step may be carried out in the presence of M complementary oligonucleotides, the M being an integer 1 or more. **In** other words, the ligation step may be a step of performing enzymatic ligation of N substrate oligonucleotides, the N being an integer 2 or more, in the presence of M complementary oligonucleotides, the M being an integer 1 or more. The ligation step may also be a step of performing enzymatic ligation of N substrate oligonucleotides, the N being an integer 2 or more, in the presence of M complementary oligonucleotides, the M being an integer 1 or more, to generate an oligonucleotide of interest. More specifically, the ligation step may be carried out in the presence of complementary oligonucleotides corresponding to respective ligation sites. In other words, each complementary oligonucleotide may be used in ligation of substrate oligonucleotides in a ligation site corresponding to the complementary oligonucleotide. More specifically, ligation between substrate oligonucleotides on 5' and 3' sides in each ligation site may be performed in the presence of a complementary oligonucleotide corresponding to the ligation site. In other words, each complementary oligonucleotide may be used in ligation between substrate oligonucleotides on 5' and 3' sides in a ligation site corresponding to the complementary oligonucleotide.

In accordance with the method of the present invention, the ligation step may be efficiently carried out by, for example, catalytically repeatedly using complementary oligonucleotides in the ligation step. Specifically, for example, the action of a destabilizing site facilitates natural dissociation of complementary oligonucleotides from the ligation product (that is, the oligonucleotide of interest) of substrate oligonucleotides, whereby the complementary oligonucleotides may be repeatedly used in the ligation step. In other words, in accordance with the method of the present invention, for example, the used amount of the complementary oligonucleotides may be reduced.

### <1> Oligonucleotide of Interest

"Oligonucleotide of interest" means an oligonucleotide produced by the method of the present invention.

The oligonucleotide of interest may be present, for example, in the form of a single-stranded oligonucleotide or a double-stranded oligonucleotide. In other words, the oligonucleotide of interest may be produced, for example, in the form of a single-stranded oligonucleotide or a double-stranded oligonucleotide. The oligonucleotide of interest may also be used, for example, in the form of a single-stranded oligonucleotide or a double-stranded oligonucleotide.

"Single-stranded oligonucleotide" means an oligonucleotide consisting of a nucleic acid strand that does not hybridize with another nucleic acid strand. In other words, for example, "oligonucleotide of interest in form of single-stranded oligonucleotide" may mean an oligonucleotide of interest in the form of hybridizing with no other nucleic acid strand. Unless the objective of the present invention is impaired, the single-stranded oligonucleotide may form a double-stranded structure (for example, stem-loop structure) in a molecule, or need not form a double-stranded structure (for example, stem-loop structure) in a molecule.

"Double-stranded oligonucleotide" means an oligonucleotide consisting of at least two nucleic acid strands that hybridize with each other. In other words, for example, "oligonucleotide of interest in the oligonucleotide" may mean an oligonucleotide of interest in the form of hybridizing with another nucleic acid strand. Examples of such other nucleic acid strands include complementary oligonucleotides. In other words, the oligonucleotide of interest may be present, produced, and/or used, for example, in the form of hybridizing with one or more complementary oligonucleotides.

The form (for example, the form as either a single-stranded oligonucleotide or a double-stranded oligonucleotide in the case of presence, production, and/or use) of the oligonucleotide of interest can be set as appropriate depending on, for example, various conditions such as applications of the oligonucleotide of interest. Such an application of the oligonucleotide of interest is not particularly limited. Examples of the applications of the oligonucleotide of interest include applications as antisense nucleic acids, siRNAs, miRNA mimics, decoy nucleic acids, aptamers, or CpG oligonucleotides. In a case in which the oligonucleotide of interest is used as an antisense nucleic acid, an aptamer, or a CpG oligonucleotide, the oligonucleotide of interest may be present, produced, and/or used as, for example, a single-stranded oligonucleotide. In a case in which the oligonucleotide of interest is used as an siRNA, an miRNA mimic, or a decoy nucleic acid, the oligonucleotide of interest may be present, produced, and/or used as, for example, a double-stranded oligonucleotide, or as a single-stranded oligonucleotide in which a double-stranded structure is formed in a molecule.

The kind of a nucleic acid residue included in the oligonucleotide of interest is not particularly limited. The kind of a nucleic acid residue included in the oligonucleotide of interest can be set as appropriate depending on, for example, various conditions such as applications of the oligonucleotide of interest. Examples of such nucleic acid residues include DNA residues, RNA residues, and modified nucleic acid residues. The oligonucleotide of interest may comprise one or more kinds of nucleic acid residues. In other words, the oligonucleotide of interest may comprise, for example, a DNA residue, an RNA residue, a modified nucleic acid residue, or a combination thereof. The oligonucleotide of interest may be a nucleic acid strand consisting of one kind of a nucleic acid residue, or may be a nucleic acid strand consisting of a combination of two or more kinds of nucleic acid residues (that is, a nucleic acid strand comprising two or more kinds of nucleic acid residues in a molecule). In other words, the oligonucleotide of interest may be, for example, a nucleic acid strand consisting of a DNA residue, a nucleic acid strand consisting of an RNA residue, a nucleic acid strand consisting of a modified nucleic acid residue, or a nucleic acid strand consisting of a combination thereof (that is, a nucleic acid strand comprising two or more kinds of nucleic acid residues selected from DNA residues, RNA residues, and modified nucleic acid residues in a molecule).

The base sequence of the oligonucleotide of interest is not particularly limited. The base sequence of the oligonucleotide of interest can be set as appropriate depending on, for example, various conditions such as applications of the oligonucleotide of interest. In a case in which the oligonucleotide of interest is used as an antisense nucleic acid, an siRNA, an miRNA mimic, a decoy nucleic acid, an aptamer, or a CpG oligonucleotide, the base sequence of the oligonucleotide of interest may be set so that the oligonucleotide of interest correspondingly functions as the antisense nucleic acid, the siRNA, the miRNA mimic, the decoy nucleic acid, the aptamer, or the CpG oligonucleotide. In other words, for example, in the case of using the oligonucleotide of interest as an antisense nucleic acid, the base sequence of the oligonucleotide of interest may be set so that the oligonucleotide of interest can sequence-specifically form a double-stranded structure with a target single-stranded RNA. Examples of the target single-stranded RNA for the antisense nucleic acid include mRNAs, pre-mRNAs, and miRNAs. Moreover, for example, in the case of using the oligonucleotide of interest as an siRNA or an miRNA mimic, the base sequence of the oligonucleotide of interest may be set so that the oligonucleotide of interest (specifically, a single strand structure generated by dissociating the oligonucleotide of interest) can sequence-specifically form a double-stranded structure with a target single-stranded RNA. Examples of the target single-stranded RNA for the siRNA or the miRNA mimic include mRNAs. Moreover, for example, in the case of using the oligonucleotide of interest as a decoy nucleic acid, an aptamer, or a CpG oligonucleotide, the base sequence of the oligonucleotide of interest may be set so that the oligonucleotide of interest sequence-specifically binds to a target protein. For example, the base sequence of the oligonucleotide of interest may be set so that the oligonucleotide of interest does not form a double-stranded structure in a molecule, or may be set so that the oligonucleotide of interest forms a double-stranded structure in a molecule.

The length of the oligonucleotide of interest is not particularly limited. The length of the oligonucleotide of interest can be set as appropriate depending on, for example, various conditions such as applications of the oligonucleotide of interest. The length of the oligonucleotide of interest may be, for example, not less than 10 residues, not less than 11 residues, not less than 12 residues, not less than 13 residues, not less than 14 residues, not less than 15 residues, not less than 16 residues, not less than 17 residues, not less than 18 residues, not less than 19 residues, not less than 20 residues, not less than 21 residues, not less than 22 residues, not less than 23 residues, not less than 24 residues, not less than 25 residues, not less than 30 residues, not less than 35 residues, not less than 40 residues, not less than 45 residues, not less than 50 residues, not less than 60 residues, not less than 70 residues, not less than 80 residues, not less than 90 residues, not less than 100 residues, not less than 110 residues, not less than 120 residues, not less than 130 residues, not less than 140 residues, not less than 150 residues, not less than 160 residues, not less than 170 residues, not less than 180 residues, or not less than 190 residues, may be not more than 200 residues, not more than 190 residues, not more than 180 residues, not more than 170 residues, not more than 160 residues, not more than 150 residues, not more than 140 residues, not more than 130 residues, not more than 120 residues, not more than 110 residues, not more than 100 residues, not more than 90 residues, not more than 80 residues, not more than 70 residues, not more than 60 residues, not more than 50 residues, not more than 45 residues, not more than 40 residues, not more than 35 residues, not more than 30 residues, not more than 25 residues, not more than 24 residues, not more than 23 residues, not more than 22 residues, not more than 21 residues, not more than 20 residues, not more than 19 residues, not more than 18 residues, not more than 17 residues, not more than 16 residues, not more than 15 residues, not more than 14 residues, not more than 13 residues, not more than 12 residues, or not more than 11 residues, or may be an uncontradictory combination thereof. Specifically, the length of the oligonucleotide of interest may be, for example, 10 to 11 residues, 11 to 12 residues, 12 to 13 residues, 13 to 14 residues, 14 to 15 residues, 15 to 16 residues, 16 to 17 residues, 17 to 18 residues, 18 to 19 residues, 19 to 20 residues, 20 to 21 residues, 21 to 22 residues, 22 to 23 residues, 23 to 24 residues, 24 to 25 residues, 25 to 30 residues, 30 to 35 residues, 35 to 40 residues, 40 to 45 residues, 45 to 50 residues, 50 to 60 residues, 60 to 70 residues, 70 to 80 residues, 80 to 90 residues, 90 to 100 residues, 100 to 110 residues, 110 to 120 residues, 120 to 130 residues, 130 to 140 residues, 140 to 150 residues, 150 to 160 residues, 160 to 170 residues, 170 to 180 residues, 180 to 190 residues, or 190 to 200 residues. Specifically, the length of the oligonucleotide of interest may be, for example, 10 to 200 residues, 10 to 100 residues, 15 to 80 residues, or 20 to 60 residues. Specifically, the length of the oligonucleotide of interest may be, for example, 10 to 200 residues, 10 to 150 residues, 15 to 120 residues, or 20 to 100 residues.

"DNA" of which a modification is not mentioned may mean a naturally occurring DNA, and may specifically mean a nucleic acid in which a nucleoside segment is deoxyadenosine, deoxyguanosine, deoxycytidine, thymidine, or deoxyuridine.

"RNA" of which a modification is not mentioned may mean a naturally occurring RNA, and may specifically mean a nucleic acid in which a nucleoside segment is adenosine, guanosine, cytidine, 5-methyluridine, or uridine.

"Modified nucleic acid" may mean a nucleic acid except naturally occurring DNAs and naturally occurring RNAs. Examples of such modified nucleic acids include modified DNAs and modified RNAs. "Modified DNA" may mean a nucleic acid having the same structure as the structure of a naturally occurring DNA except that the nucleic acid is modified. "Modified RNA" may mean a nucleic acid having the same structure as the structure of a naturally occurring RNA except that the nucleic acid is modified.

The phrases "oligonucleotide of interest comprises modified nucleic acid residue", "oligonucleotide of interest is modified", and "oligonucleotide of interest has modification" may be used interchangeably with each other.

The phrases "nucleic acid residue is modified nucleic acid residue", "nucleic acid residue is modified", and "nucleic acid residue has modification" may be used interchangeably with each other.

The oligonucleotide of interest may comprise or need not comprise a modified nucleic acid residue. In other words, the oligonucleotide of interest may be, or need not be modified. The modification modes (for example, the kind, position, and amount of modification) of the oligonucleotide of interest are not particularly limited unless the objective of the present invention is impaired. The modification modes of the oligonucleotide of interest can be set as appropriate depending on, for example, various conditions such as applications of the oligonucleotide of interest. Examples of the modification include a modification of a phosphate portion, a modification of a sugar portion, and a modification of a base portion. In other words, the modification may comprise, for example, a modification of a phosphate portion, a modification of a sugar portion, a modification of a base portion, or a combination thereof. The phrase "modification comprises certain modification A" means that at least the modification A is selected as the modification, and encompasses a case in which the modification consists of the modification A, and a case in which the modification consists of a combination of the modification A and another modification. For example, the phrase "modification comprises modification of phosphate portion" means that at least the modification of the phosphate portion is selected as the modification, and encompasses a case in which the modification consists of the modification of the phosphate portion, and a case in which the modification consists of a combination of the modification of the phosphate portion and another modification.

Examples of the phosphate portion include a phosphate group present between nucleic acid residues (that is, a phosphate group that forms a phosphodiester bond between nucleic acid residues). In a case in which a phosphate group between nucleic acid residues is modified, the nucleic acid residue on the 3' sides of the phosphate group (that is, the nucleic acid residue having the phosphate group at position 5') is considered to be modified. Examples of the modification of the phosphate portion include: phosphorothioation (that is, substitution of an oxygen atom of a phosphate group with a sulfur atom; that is, substitution of a phosphate group with a thiophosphate group); and boranophosphation (that is, substitution of an oxygen atom of a phosphate group with borane (BH₃); that is, substitution of a phosphate group with a boranophosphate group). Examples of the modification of the phosphate portion also include an insertion of a linker. "Linker" means a structure other than nucleic acid residues. Examples of the linker include hydrocarbon groups. Examples of the hydrocarbon groups include non-aromatic hydrocarbon groups and aromatic hydrocarbon groups. Examples of the non-aromatic hydrocarbon groups include alkylene groups and alkenylene groups. The alkylene groups may be straight-chain or branched-chain. Examples of the alkylene groups include C₁₋₁₂, C₁₋₈, C₁₋₆, or C₁₋₄ alkylene groups. Examples of the C₁₋₄ alkylene groups include a methylene group, an ethylene group, a propylene group, an isopropylene group, a butylene group, an isobutylene group, a sec-butylene group, and a tert-butylene group. The alkenylene groups may be straight-chain or branched-chain. Examples of the alkenylene groups include C₂₋₁₂, C₂₋₈, C₂₋₆, or C₂₋₄ alkenylene groups. Examples of the C₂₋₄ alkenylene groups include an ethenylene group, a propenylene group, an isopropenylene group, a butenylene group, an isobutenylene group, and a sec-butenylene group. The alkenylene groups may be, for example, cis-type or trans-type. Examples of the aromatic hydrocarbon groups include C₆₋₁₂ aromatic hydrocarbon groups. Examples of the C₆₋₁₂ aromatic hydrocarbon groups include a xylylene group. For example, both ends of the linker may bind to a phosphate group. In other words, the linker may bind to, for example, the phosphate group at position 3' of a nucleic acid residue on the 5' side thereof, and the phosphate group at position 5' of a nucleic acid residue on the 3' side thereof. In other words, an insertion of a linker in a phosphate group present between nucleic acid residues may be substitution of the phosphate group present between the nucleic acid residues with a linker sandwiched between phosphate groups. Each of the phosphate groups to which the linker binds may be or need not be subjected to a modification (for example, phosphorothioation or boranophosphation). For example, in a case in which a complementary oligonucleotide comprises an insertion of a nucleic acid residue as a destabilizing site, the linker may be inserted into a position corresponding to the insertion of the nucleic acid residue in the oligonucleotide of interest.

Examples of the sugar portion include deoxyribose which is the sugar portion of DNA and ribose which is the sugar portion of RNA. Examples of the modification of the sugar portion include 2'-MOE (that is, O-methoxyethylation of position 2' of sugar portion), 2'-OMe (that is, O-methylation of position 2' of sugar portion), 2'-F (that is, fluorination of position 2' of sugar portion), 4'-thio- 2'-OMe (that is, substitution of oxygen atom at position 4' of sugar portion with sulfur atom, and O-methylation of position 2' of sugar portion), and crosslinkage between positions 2' and 4' of a sugar portion. Examples of the crosslinkage between the positions 2' and 4' of the sugar portion include LNA, BNA^{NC}, BNA^{NC} (Me), and AmNA. Examples of the modification of the sugar portion also include a modification of the 5' end of an oligonucleotide and a modification of the 3' end of an oligonucleotide. Examples of the modification of the 5' end of an oligonucleotide include 5'-amination, 5'-thiolation, 5'-dabsylation, 5'-fluoresceination, 5'-tetrafluorofluoresceination, 5'-phosphorylation, 5'-inverted thymidine, 5'-biotin addition, 5'-PEG addition, 5'-N-acetylgalactosamine (GalNAc) addition, 5'-peptide addition, 5'-cholesterol addition, 5'-tocopherol addition, 5'-aliphatic chain addition, 5'-folate addition, 5'-polyamine addition, and 5'-agent addition. Examples of the modification of the 3' end of an oligonucleotide include 3'-amination, 3'-thiolation, 3'-dabsylation, 3'-deoxygenation, 3'-carboxylation, 3'-phosphorylation, 3'-inverted thymidine, 3'-biotin addition, 3'-PEG addition, 3'-N-acetylgalactosamine (GalNAc) addition, 3'-peptide addition, 3'-cholesterol addition, 3'-tocopherol addition, 3'-aliphatic chain addition, 3'-folate addition, 3'-polyamine addition, and 3'-agent addition. Examples of the agents include anticancer agents and other various pharmaceutical components. An adduct such as such an agent may be added, for example, directly, or through an appropriate linker, to an oligonucleotide (Do-Hun Kim et. al., Design and clinical developments of aptamer-drug conjugates for targeted cancer therapy, Biomater Res. 2021 Nov 25;25(1):42.).

Examples of a modification of a phosphate portion and a sugar portion also include morpholination and peptidation of a nucleic acid skeleton. In other words, examples of modified nucleic acids also include morpholino nucleic acids and peptide nucleic acids (PNAs).

Examples of the base portion include adenine (A), thymine (T), guanine (G), cytosine (C), and uracil (U). Examples of the modification of the base portion include alkylation (methylation or the like), O-alkylation (O-methylation or the like), N-alkylation (N-methylation or the like), aminoalkylation (aminopropylation or the like), alkoxylation (methoxylation or the like), halogenation (bromination, fluorination, iodization, chlorization, or the like), acylation, amination, carboxylation, hydroxylation, oxonation, nitration, thiation, azation, deazation, and substitution with other various analogs. Specific examples of modified bases include 8-haloadenine (halo is bromo, fluoro, iodine, or chloro; hereinafter, the same applies), 8-haloguanine, 5-halothymine, 5-halocytosine, 5-halouracil, 1-, 3-, or 7-deazaadenine, 1-, 3-, or 7-deazaguanine, 3-deazathymine, 3-deazacytosine, 3-deazauracil, 8-azaadenine, 8-azaguanine, 5- or 6-azathymine, 5- or 6-azacytosine, 5- or 6-azauracil, 3-methyladenine, N6-methyladenine, O6-methylguanine, O4-methylthymine, 5-methylcytosine, 5-(2-amino)propyluracil, 2-aminoadenine, 8-oxoadenine, 8-oxoguanine, 2- or 4-thiothymine, 2- or 4-thiocytosine, 2- or 4-thiouracil, 2-aminopurine, 2,6-diaminopurine, hypoxanthine, and 7-deaza-8-azahypoxanthine. Specific examples of the modified bases also include artificial bases such as x, y, s, Ds, Pa, PxR, PICS, 5SICS, NaM, MMO2, TPT3, iG, iC, P, and Z (Hirao et al., For New Research Area, Xenobiology, from Artificial Base Pair Technology for Expanding Genetic Information, KAGAKU TO SEIBUTSU Vol. 54, No. 11, 2016, pp. 835-840). Examples of the modification of the base portion also include a deficit in a base portion. In other words, the modified nucleic acid may be a nucleic acid in which no base portion is present (that is, abasic nucleic acid).

The presence or absence, and kind of the modification can be independently set for each nucleic acid residue included in the oligonucleotide of interest. The oligonucleotide of interest may have one kind of a modification, or may have two or more kinds of modifications in combination. The oligonucleotide of interest may, or need not comprise, for example, each modification described as an example above. Each nucleic acid residue included in the oligonucleotide of interest may have one kind of a modification, or may have two or more kinds of modifications in combination. Only some of the nucleic acid residues included in the oligonucleotide of interest may be modified nucleic acid residues, or all of the nucleic acid residues included in the oligonucleotide of interest may be modified nucleic acid residues.

The proportion of the modified nucleic acid residues in the oligonucleotide of interest (that is, the proportion of the number of the modified nucleic acid residues to the number of the nucleic acid residues included in the oligonucleotide of interest) may be, for example, 0% or more, 5% or more, 10% or more, 20% or more, 30% or more, 40% or more, 50% or more, 60% or more, 70% or more, 80% or more, 90% or more, or 95% or more, may be 100% or less, 95% or less, 90% or less, 80% or less, 70% or less, 60% or less, 50% or less, 40% or less, 30% or less, 20% or less, 10% or less, or 5% or less, or may be an uncontradictory combination thereof. Specifically, the proportion of the modified nucleic acid residues in the oligonucleotide of interest may be, for example, 0 to 5%, 5 to 10%, 10 to 20%, 20 to 30%, 30 to 40%, 40 to 50%, 50 to 60%, 60 to 70%, 70 to 80%, 80 to 90%, 90 to 95%, or 95 to 100%. The proportion, described as an example above, of the modified nucleic acid residues in the oligonucleotide of interest can also be independently applied to nucleic acid residues having an optionally selected modification (for example, any modification described as an example above). In other words, for example, the proportion of nucleic acid residues having a modification in a phosphate portion in the oligonucleotide of interest (that is, the proportion of the number of nucleic acid residues having a modification in a phosphate portion to the number of the nucleic acid residues included in the oligonucleotide of interest) may be set at the proportion, described as an example above, of the modified nucleic acid residues in the oligonucleotide of interest. For example, the proportion of nucleic acid residues having a modification of a sugar portion in the oligonucleotide of interest (that is, the proportion of the number of nucleic acid residues having a modification of a sugar portion to the number of the nucleic acid residues included in the oligonucleotide of interest) may be set at the proportion, described as an example above, of the modified nucleic acid residues in the oligonucleotide of interest. For example, the proportion of nucleic acid residues having a modification of a base portion in the oligonucleotide of interest (that is, the proportion of the number of nucleic acid residues having a modification of a base portion to the number of the nucleic acid residues included in the oligonucleotide of interest) may be set at the proportion, described as an example above, of the modified nucleic acid residues in the oligonucleotide of interest.

The number of the modified nucleic acid residues in the oligonucleotide of interest may be, for example, 0 or more, 5 or more, 10 or more, 15 or more, 20 or more, 25 or more, 30 or more, 40 or more, 50 or more, 60 or more, 80 or more, 100 or more, 120 or more, 140 or more, 160 or more, or 180 or more, may be 200 or less, 180 or less, 160 or less, 140 or less, 120 or less, 100 or less, 80 or less, 60 or less, 50 or less, 40 or less, 30 or less, 25 or less, 20 or less, 15 or less, 10 or less, or 5 or less, or may be an uncontradictory combination thereof. Specifically, the number of the modified nucleic acid residues in the oligonucleotide of interest may be, for example, 0 to 5, 5 to 10, 10 to 15, 15 to 20, 20 to 25, 25 to 30, 30 to 40, 40 to 50, 50 to 60, 60 to 80, 80 to 100, 100 to 120, 120 to 140, 140 to 160, 160 to 180, or 180 to 200. The number, described as an example above, of the modified nucleic acid residues in the oligonucleotide of interest can also be independently applied to nucleic acid residues having an optionally selected modification (for example, any modification described as an example above). In other words, for example, the number of nucleic acid residues having a modification of a phosphate portion in the oligonucleotide of interest may be set at the number, described as an example above, of the modified nucleic acid residues in the oligonucleotide of interest. For example, the number of nucleic acid residues having a modification of a sugar portion in the oligonucleotide of interest may be set at the number, described as an example above, of the modified nucleic acid residues in the oligonucleotide of interest. For example, the number of nucleic acid residues having a modification of a base portion in the oligonucleotide of interest may be set at the number, described as an example above, of the modified nucleic acid residues in the oligonucleotide of interest.

Unless otherwise specified, modified nucleic acid residues of which the structure of the position 2' of the sugar portion is identical with that of deoxyribose are considered to correspond to modified DNA residues. Unless otherwise specified, modified nucleic acid residues of which the structure of the position 2' of the sugar portion is identical with that of ribose are considered to correspond to modified RNA residues. Unless otherwise specified, modified nucleic acid residues having a modification of the position 2' of a sugar portion are considered to correspond to both modified DNA residues and modified RNA residues.

For example, the oligonucleotide of interest may be modified in such a modification mode as described as an example above at the time of ligation of substrate oligonucleotides, and may be modified to achieve such a modification mode as described above after ligation of substrate oligonucleotides. In other words, for example, the modified oligonucleotide of interest may be produced by ligating substrate oligonucleotides that are modified according to the modification mode of the oligonucleotide of interest, or may be produced by ligating substrate oligonucleotides that are not modified (for example, may be unmodified) according to the modification mode of the oligonucleotide of interest, and modifying the ligation product after the ligation.

### <2> Substrate Oligonucleotides and Set Thereof

"Substrate oligonucleotide" means each of single-stranded oligonucleotides that are ligated in the ligation step. In other words, "substrate oligonucleotide" means each of nucleic acid fragments included in the oligonucleotide of interest.

"Single strand" in the substrate oligonucleotides shows the form of the substrate oligonucleotides at the time of starting the method (specifically, ligation step) of the present invention. The substrate oligonucleotides can be in the form of double-stranded oligonucleotides depending on the progression of the method (specifically, ligation step) of the present invention. For example, the substrate oligonucleotides can hybridize with complementary oligonucleotides to be in the form of double-stranded oligonucleotides depending on the progression of the method (specifically, ligation step) of the present invention.

"Set of substrate oligonucleotides" is the generic name of substrate oligonucleotides that are ligated in the ligation step. A set of substrate oligonucleotides consists of N substrate oligonucleotides, the N being an integer 2 or more,.

The number (N) of the substrate oligonucleotides is 2 or more. "Number of substrate oligonucleotides" means the number of substrate oligonucleotides that are ligated in the ligation step. In other words, "number of substrate oligonucleotides" means the number of substrate oligonucleotides included in the oligonucleotide of interest. In other words, "number of substrate oligonucleotides" means the number of substrate oligonucleotides included in a set of the substrate oligonucleotides. The number (N) of the substrate oligonucleotides may be 2, or may be 3 or more. For example, the number (N) of the substrate oligonucleotides may be 2 or more, 3 or more, 4 or more, 5 or more, 6 or more, 7 or more, 8 or more, or 9 or more, may be 10 or less, 9 or less, 8 or less, 7 or less, 6 or less, 5 or less, 4 or less, or 3 or less, or may be an uncontradictory combination thereof. Specifically, the number (N) of the substrate oligonucleotides may be, for example, 2 to 3, 3 to 4, 4 to 5, 5 to 6, 6 to 7, 7 to 8, 8 to 9, or 9 to 10. Specifically, the number (N) of the substrate oligonucleotides may be, for example, 2 to 10, 2 to 7, 2 to 5, 2 to 4, or 2 to 3. The number (N) of the substrate oligonucleotides may be particularly 2 to 3. The number (N) of the substrate oligonucleotides may be still more particularly 2.

A ligation site between substrate oligonucleotides is also simply referred to as "ligation site". Two substrate oligonucleotides that are ligated through each ligation site are also referred to as "substrate oligonucleotide on 5' side" and "substrate oligonucleotide on 3' side", respectively. The phrase "5' side" may be used interchangeably with the phrase "5' end side". The phrase "3' side" may be used interchangeably with the phrase "3' end side". The 3' end of a substrate oligonucleotide on a 5' side and the 5' end of a substrate oligonucleotide on a 3' side are ligated to each other at each ligation site. It is to be noted that "5' side" and "3' side" for a substrate oligonucleotide show the same directions as "5' side" and "3' side" for the oligonucleotide of interest, respectively. Moreover, "5' side" and "3' side" for a substrate oligonucleotide show directions reverse to "5' side" and "3' side" for a complementary oligonucleotide that hybridizes with the substrate oligonucleotide, respectively. Moreover, "5' side" and "3' side" for a substrate oligonucleotide show relative directions based on each ligation site. In other words, for example, in a case in which three substrate oligonucleotides A, B, and C are ligated in a 5' to 3' direction, A and B are substrate oligonucleotides on 5' and 3' sides, respectively, in the ligation of A and B, and B and C are substrate oligonucleotides on 5' and 3' sides, respectively, in the ligation of B and C. The number of ligation sites is determined depending on the number of substrate oligonucleotides. In other words, in a case in which the number of the substrate oligonucleotides is N, the N being an integer 2 or more, the number of the ligation sites is N - 1. In other words, the ligation step is carried out at the N - 1 ligation sites. In other words, the oligonucleotide of interest consists of N substrate oligonucleotides that are ligated at N - 1 positions. In a case in which the number of a ligation site is 1, "each ligation site" means the ligation site. In a case in which the number of ligation sites is 2 or more, "each ligation site" means each of the ligation sites.

The structure (for example, the kind, base sequence, length, and modification mode of nucleic acid residues) of each substrate oligonucleotide can be set as appropriate depending on the structure of the oligonucleotide of interest. In other words, the structure of each substrate oligonucleotide may be the same as the structure of a segment corresponding to each substrate oligonucleotide in the oligonucleotide of interest.

The kind of a nucleic acid residue is as described above. Each substrate oligonucleotide may comprise one or more kinds of nucleic acid residues. In other words, each substrate oligonucleotide may comprise, for example, a DNA residue, an RNA residue, a modified nucleic acid residue, or a combination thereof. Each substrate oligonucleotide may be a nucleic acid strand consisting of one kind of nucleic acid residues, or may be a nucleic acid strand consisting of a combination of two or more kinds of nucleic acid residues (that is, a nucleic acid strand comprising two or more kinds of nucleic acid residues in a molecule). In other words, for example, each substrate oligonucleotide may be a nucleic acid strand consisting of DNA residues, may be a nucleic acid strand consisting of RNA residues, may be a nucleic acid strand consisting of modified nucleic acid residues, or may be a nucleic acid strand consisting of a combination thereof (that is, a nucleic acid strand comprising, in a molecule, two or more kinds of nucleic acid residues selected from DNA residues, RNA residues, and modified nucleic acid residues).

The base sequence of each substrate oligonucleotide consisting of the partial sequence of the base sequence of the oligonucleotide of interest. Moreover, the base sequence of each substrate oligonucleotide is set so that the ligation sequence of the substrate oligonucleotides consists of the base sequence of the oligonucleotide of interest. For example, in a case in which the number of substrate oligonucleotides is two, the base sequence of a substrate oligonucleotide on a 5' side consists of the partial sequence on the 5' side of the oligonucleotide of interest, the base sequence of a substrate oligonucleotide on a 3' side consists of the partial sequence on the 3' side of the oligonucleotide of interest, and the ligation sequence of the substrate oligonucleotide on the 5' side and the substrate oligonucleotide on the 3' side consists of the base sequence of the oligonucleotide of interest.

The length of each substrate oligonucleotide is not particularly limited unless the objective of the present invention is impaired. In other words, the length of each substrate oligonucleotide is set so that the substrate oligonucleotides can be ligated by the method of the present invention. The length of each substrate oligonucleotide may be set, for example, so that each substrate oligonucleotide can hybridize with a corresponding complementary oligonucleotide. "Complementary oligonucleotide corresponding to substrate oligonucleotide" means a complementary oligonucleotide that is used in a ligation site between the substrate oligonucleotide and a substrate oligonucleotide that is ligated thereto. The length of each substrate oligonucleotide can be set so that the length of a ligation sequence to which substrate oligonucleotides are ligated is equal to the length of the oligonucleotide of interest.

For example, the length of each substrate oligonucleotide may be not less than 5 residues, not less than 6 residues, not less than 7 residues, not less than 8 residues, not less than 9 residues, not less than 10 residues, not less than 11 residues, not less than 12 residues, not less than 13 residues, not less than 14 residues, not less than 15 residues, not less than 17 residues, not less than 20 residues, not less than 25 residues, not less than 30 residues, not less than 35 residues, not less than 40 residues, not less than 45 residues, not less than 50 residues, not less than 55 residues, not less than 60 residues, or not less than 65 residues, may be not more than 70 residues, not more than 65 residues, not more than 60 residues, not more than 55 residues, not more than 50 residues, not more than 45 residues, not more than 40 residues, not more than 35 residues, not more than 30 residues, not more than 25 residues, not more than 20 residues, not more than 17 residues, not more than 15 residues, not more than 14 residues, not more than 13 residues, not more than 12 residues, not more than 11 residues, not more than 10 residues, not more than 9 residues, not more than 8 residues, not more than 7 residues, or not more than 6 residues, or may be an uncontradictory combination thereof. Specifically, the length of each substrate oligonucleotide may be, for example, 5 to 6 residues, 6 to 7 residues, 7 to 8 residues, 8 to 9 residues, 9 to 10 residues, 10 to 11 residues, 11 to 12 residues, 12 to 13 residues, 13 to 14 residues, 14 to 15 residues, 15 to 17 residues, 17 to 20 residues, 20 to 25 residues, 25 to 30 residues, 30 to 35 residues, 35 to 40 residues, 40 to 45 residues, 45 to 50 residues, 50 to 55 residues, 55 to 60 residues, 60 to 65 residues, or 65 to 70 residues. Specifically, the length of each substrate oligonucleotide may be, for example, 5 to 70 residues, 5 to 50 residues, 5 to 30 residues, 5 to 20 residues, or 5 to 15 residues.

For example, the length of each substrate oligonucleotide may be 3% or more, 5% or more, 10% or more, 20% or more, 30% or more, 40% or more, 50% or more, 60% or more, 70% or more, 80% or more, 90% or more, or 95% or more, may be 97% or less, 95% or less, 90% or less, 80% or less, 70% or less, 60% or less, 50% or less, 40% or less, 30% or less, 20% or less, 10% or less, or 5% or less, or may be an uncontradictory combination thereof, as a proportion in a case in which the length of the oligonucleotide of interest is taken as 100%. Specifically, the length of each substrate oligonucleotide may be, for example, 3 to 5%, 5 to 10%, 10 to 20%, 20 to 30%, 30 to 40%, 40 to 50%, 50 to 60%, 60 to 70%, 70 to 80%, 80 to 90%, 90 to 95%, or 95 to 97% as a proportion in a case in which the length of the oligonucleotide of interest is taken as 100%. Specifically, the length of each substrate oligonucleotide may be, for example, 3 to 97%, 10 to 90%, 20 to 80%, 30 to 70%, or 40 to 60% as a proportion in a case in which the length of the oligonucleotide of interest is taken as 100%.

The phrases "substrate oligonucleotide comprises modified nucleic acid residue", "substrate oligonucleotide is modified", and "substrate oligonucleotide has modification" may be used interchangeably with each other.

Each substrate oligonucleotide may comprise or need not comprise modified nucleic acid residues. In other words, each substrate oligonucleotide may be modified or need not be modified. Each substrate oligonucleotide may be modified or need not be modified depending on the modification mode of the oligonucleotide of interest. For example, in the case of modifying a ligation product after ligation of substrate oligonucleotides, the modification mode of the substrate oligonucleotides and the modification mode of the oligonucleotide of interest can be different from each other. The modification of the nucleic acid residues is as described above. Each substrate oligonucleotide may have one kind of a modification or may have two or more kinds of modifications in combination. Each substrate oligonucleotide may comprise or need not comprise, for example, each modification described as an example above. Each nucleic acid residue included in each substrate oligonucleotide may have one kind of a modification or may have two or more kinds of modifications in combination. Only some of nucleic acid residues included in each substrate oligonucleotide may be modified nucleic acid residues, or all of the nucleic acid residues included in each substrate oligonucleotide may be modified nucleic acid residues.

The proportion of modified nucleic acid residues in each substrate oligonucleotide (that is, the proportion of the number of modified nucleic acid residues to the number of nucleic acid residues included in each substrate oligonucleotide) can be set so that the proportion of modified nucleic acid residues in a ligation sequence to which substrate oligonucleotides are ligated is equal to the proportion of the modified nucleic acid residues in the oligonucleotide of interest. For example, the proportion of the modified nucleic acid residues in each substrate oligonucleotide may be 0% or more, 5% or more, 10% or more, 20% or more, 30% or more, 40% or more, 50% or more, 60% or more, 70% or more, 80% or more, 90% or more, or 95% or more, may be 100% or less, 95% or less, 90% or less, 80% or less, 70% or less, 60% or less, 50% or less, 40% or less, 30% or less, 20% or less, 10% or less, or 5% or less, or may be an uncontradictory combination thereof. Specifically, the proportion of the modified nucleic acid residues in each substrate oligonucleotide may be, for example, 0 to 5%, 5 to 10%, 10 to 20%, 20 to 30%, 30 to 40%, 40 to 50%, 50 to 60%, 60 to 70%, 70 to 80%, 80 to 90%, 90 to 95%, or 95 to 100%. The proportion, described as an example above, of the modified nucleic acid residues in each substrate oligonucleotide can also be independently applied to nucleic acid residues having an optionally selected modification (for example, any modification described as an example above). In other words, for example, the proportion of nucleic acid residues having a modification of a phosphate portion in each substrate oligonucleotide (that is, the proportion of the number of the nucleic acid residues having the modification of the phosphate portion to the number of nucleic acid residues included in each substrate oligonucleotide) may be set at the proportion, described as an example above, of the modified nucleic acid residues in each substrate oligonucleotide. For example, the proportion of nucleic acid residues having a modification of a sugar portion in each substrate oligonucleotide (that is, the proportion of the number of the nucleic acid residues having the modification of the sugar portion to the number of nucleic acid residues included in each substrate oligonucleotide) may be set at the proportion, described as an example above, of the modified nucleic acid residues in each substrate oligonucleotide. For example, the proportion of nucleic acid residues having a modification of a base portion in each substrate oligonucleotide (that is, the proportion of the number of the nucleic acid residues having the modification of the base portion to the number of nucleic acid residues included in each substrate oligonucleotide) may be set at the proportion, described as an example above, of the modified nucleic acid residues in each substrate oligonucleotide.

The number of the modified nucleic acid residues in each substrate oligonucleotide can be set so that the number of modified nucleic acid residues in a ligation sequence to which substrate oligonucleotides are ligated is equal to the number of the modified nucleic acid residues in the oligonucleotide of interest. For example, the number of the modified nucleic acid residues in each substrate oligonucleotide may be 0 or more, 5 or more, 10 or more, 15 or more, 20 or more, 25 or more, 30 or more, 35 or more, 40 or more, 45 or more, 50 or more, 55 or more, 60 or more, or 65 or more, may be 70 or less, 65 or less, 60 or less, 55 or less, 50 or less, 45 or less, 40 or less, 35 or less, 30 or less, 25 or less, 20 or less, 15 or less, 10 or less, or 5 or less, or may be an uncontradictory combination thereof. Specifically, the length of each substrate oligonucleotide may be, for example, 0 to 5 residues, 5 to 10 residues, 10 to 15 residues, 15 to 20 residues, 20 to 25 residues, 25 to 30 residues, 30 to 35 residues, 35 to 40 residues, 40 to 45 residues, 45 to 50 residues, 50 to 55 residues, 55 to 60 residues, 60 to 65 residues, or 65 to 70 residues. The number, described as an example above, of the modified nucleic acid residues in each substrate oligonucleotide can also be independently applied to nucleic acid residues having an optionally selected modification (for example, any modification described as an example above). In other words, for example, the number of nucleic acid residues having a modification of a phosphate portion in each substrate oligonucleotide may be set at the number, described as an example above, of the modified nucleic acid residues in each substrate oligonucleotide. For example, the number of nucleic acid residues having a modification of a sugar portion in each substrate oligonucleotide may be set at the number, described as an example above, of the modified nucleic acid residues in each substrate oligonucleotide. For example, the number of nucleic acid residues having a modification of a base portion in each substrate oligonucleotide may be set at the number, described as an example above, of the modified nucleic acid residues in each substrate oligonucleotide.

Each substrate oligonucleotide may have, for example, a configuration useful or necessary for ligation between substrate oligonucleotides. For example, the 5' end of a substrate oligonucleotide on a 3' side may be phosphorylated. In other words, the ligation between substrate oligonucleotides may be, for example, ligation between the phosphate group of the 5' end of a substrate oligonucleotide on a 3' side and the hydroxyl group of the 3' end of a substrate oligonucleotide on a 5' side. For example, the 3' end of a substrate oligonucleotide on a 5' side may be phosphorylated. In other words, the ligation between substrate oligonucleotides may be, for example, ligation between the hydroxyl group of the 5' end of a substrate oligonucleotide on a 3' side and the phosphate group of the 3' end of a substrate oligonucleotide on a 5' side. The phosphate portion of the 5' end or the 3' end may be modified, or need not be modified. Examples of the modification of the phosphate portion of the 5' end or the 3' end include phosphorothioation (that is, substitution of the oxygen atom of the phosphate group with a sulfur atom), and boranophosphation (that is, substitution of the oxygen atom of the phosphate group with borane (BH₃)). Examples of the modification of the phosphate portion of the 5' end or the 3' end also include an insertion of a linker. The insertion of the linker is as described above.

A method of producing each substrate oligonucleotide is not particularly limited. Each substrate oligonucleotide can be produced by, for example, in turn ligating nucleic acid residues included in each substrate oligonucleotide. For example, the nucleic acid residues can be ligated by a phosphoroamidite method. Each substrate oligonucleotide can be produced by, for example, ligating nucleic acid fragments included in each substrate oligonucleotide. The nucleic acid fragments can be enzymatically ligated by, for example, using a complementary strand. The nucleic acid fragments may be ligated by, for example, the method of the present invention.

### <3> Complementary Oligonucleotide

"Complementary oligonucleotide" means a single-stranded oligonucleotide that is used in the ligation step and has a particular structure. In the ligation step, a complementary oligonucleotide corresponding to each ligation site is used.

"Single strand" for a complementary oligonucleotide shows the form of a complementary oligonucleotide at the start of the method (specifically, ligation step) of the present invention. The complementary oligonucleotide can be in the form of a double-stranded oligonucleotide depending on the progression of the method (specifically, ligation step) of the present invention. For example, the complementary oligonucleotide can hybridize with a substrate oligonucleotide to be in the form of a double-stranded oligonucleotide depending on the progression of the method (specifically, ligation step) of the present invention.

The structure (for example, the kind, base sequence, length, and modification mode of nucleic acid residues) of a complementary oligonucleotide can be set as appropriate depending on various conditions such as the structures of substrate oligonucleotides on 5' and 3' sides, ligated through a ligation site corresponding to the complementary oligonucleotide.

The kind of a nucleic acid residue is as described above. Each complementary oligonucleotide may comprise one or more kinds of nucleic acid residues. In other words, each complementary oligonucleotide may comprise, for example, DNA residues, RNA residues, modified nucleic acid residues, or a combination thereof. Each complementary oligonucleotide may be a nucleic acid strand consisting of one kind of nucleic acid residues, or may be a nucleic acid strand consisting of a combination of two or more kinds of nucleic acid residues (that is, a nucleic acid strand comprising two or more kinds of nucleic acid residues in a molecule). In other words, for example, each complementary oligonucleotide may be a nucleic acid strand consisting of DNA residues, may be a nucleic acid strand consisting of RNA residues, may be a nucleic acid strand consisting of modified nucleic acid residues, or may be a nucleic acid strand consisting of a combination thereof (that is, a nucleic acid strand comprising, in a molecule, two or more kinds of nucleic acid residues selected from the DNA residues, the RNA residues, and the modified nucleic acid residues).

The complementary oligonucleotide comprises a base sequence in which a destabilizing site is introduced into a first base sequence. The base sequence in which a destabilizing site is introduced into a first base sequence is also referred to as "destabilizing base sequence".

"Base sequence in which destabilizing site is introduced into first base sequence" means a base sequence that is the modified sequence of a first base sequence and is the same as the first base sequence except that a destabilizing site is introduced (that is, the destabilizing site is included). In other words, the first base sequence is a base sequence prior to the introduction of the destabilizing site (that is, including no destabilizing site). "Introduction of destabilizing site" referred to herein is an expression for specifying the structure of a destabilizing base sequence, and not for specifying a method of producing a destabilizing base sequence.

The first base sequence is a base sequence consisting of a second base sequence and a third base sequence that are ligated in 5' to 3' direction. "Base sequence consisting of second base sequence and third base sequence that are ligated in 5' to 3' direction" means the ligation sequence of the second base sequence and the third base sequence, in which the 3' end of the second base sequence and the 5' end of the third base sequence are ligated to each other. "Ligation of second base sequence and third base sequence" referred to herein is an expression for specifying the structure of the first base sequence, and not for specifying a method of producing the first base sequence.

The second base sequence is the complementary sequence of the partial sequence on the 5' side of the substrate oligonucleotide on the 3' side, or the complementary sequence of the full-length sequence of the substrate oligonucleotide on the 3' side. "Partial sequence on 5' side of substrate oligonucleotide on 3' side" means the partial sequence of the substrate oligonucleotide on the 3' side, the partial sequence consisting of consecutive nucleic acid residues comprising nucleic acid residues on the 5' end (which are nucleic acid residues that are ligated to the 3' end of the substrate oligonucleotide on the 5' side).

The third base sequence is the complementary sequence of the partial sequence on the 3' side of the substrate oligonucleotide on the 5' side, or the complementary sequence of the full-length sequence of the substrate oligonucleotide on the 5' side. "Partial sequence on 3' side of substrate oligonucleotide on 5' side" is the partial sequence of the substrate oligonucleotide on the 5' side, the partial sequence consisting of consecutive nucleic acid residues comprising nucleic acid residues on the 3' end (which are nucleic acid residues that are ligated to the 5' end of the substrate oligonucleotide on the 3' side).

"Complementary" between base sequences may mean that a base portion can form a Watson-Crick base pair or a base pair having a strength that is equivalent to or more than that of the Watson-Crick base pair between base sequences. Examples of base pairs considered to be complementary include Watson-Crick base pairs selected from the bases (which may be, or need not be modified) described as examples above, and base pairs including combinations enabling formation of base pairs having strengths that are equivalent or more than those of the Watson-Crick base pairs. Specific examples of the base pairs considered to be complementary include Watson-Crick base pairs such as A-T base pairs, A-U base pairs, and G-C base pairs. Specific examples of the base pairs considered to be complementary also include artificial base pairs such as x-y base pairs, s-y base pairs, Ds-Pa base pairs, Ds-PxR base pairs, PICS-PICS base pairs, 5SICS-NaM base pairs, 5SICS-MMO2 base pairs, TPT3-NaM base pairs, iG-iC base pairs, and P-Z base pairs.

All of the first, second, and third base sequences may be virtual base sequences for specifying a destabilizing base sequence, and need not actually exist. For example, in a case in which a substrate oligonucleotide comprises a nucleic acid residue A1 that is not able to form a Watson-Crick base pair or a base pair having a strength that is equivalent to or more than that of the Watson-Crick base pair with any base portion, the first, second, or third base sequence may be a virtual base sequence comprising a nucleic acid residue A2 having a virtual base portion that can form a Watson-Crick base pair or a virtual base pair having a strength that is equivalent to or more than that of the Watson-Crick base pair with the nucleic acid residue A1. Specifically, for example, in a case in which a substrate oligonucleotide comprises an abasic nucleic acid residue B1, the abasic nucleic acid residue B1 is not able to form a base pair with any base portion, but the first, second, or third base sequence may be a virtual base sequence comprising a nucleic acid residue B2 having a virtual base portion that can form a Watson-Crick base pair or a virtual base pair having a strength that is equivalent to or more than that of the Watson-Crick base pair with the abasic nucleic acid residue B1. Unless otherwise specified, such a virtual base pair is regarded as a base pair having the same strength as that of an AT base pair in calculation of a melting temperature (Tm).

The lengths of the first, second, and third base sequences are not particularly limited unless the objective of the present invention is impaired. In other words, the lengths of the first, second, and third base sequences are set so that substrate oligonucleotides can be ligated by the method of the present invention. The length of the first base sequence is the total of the lengths of the second and third base sequences. The length of the second base sequence may be set so that, for example, a complementary oligonucleotide can hybridize with a substrate oligonucleotide on a 3' side. The length of the third base sequence may be set so that, for example, a complementary oligonucleotide can hybridize with a substrate oligonucleotide on a 5' side.

For example, the length of the first base sequence may be not less than 10 residues, not less than 11 residues, not less than 12 residues, not less than 13 residues, not less than 14 residues, not less than 15 residues, not less than 16 residues, not less than 17 residues, not less than 20 residues, not less than 25 residues, not less than 30 residues, not less than 35 residues, not less than 40 residues, not less than 45 residues, not less than 50 residues, not less than 60 residues, not less than 70 residues, not less than 80 residues, or not less than 90 residues, may be not more than 100 residues, not more than 90 residues, not more than 80 residues, not more than 70 residues, not more than 60 residues, not more than 50 residues, not more than 45 residues, not more than 40 residues, not more than 35 residues, not more than 30 residues, not more than 25 residues, not more than 20 residues, not more than 17 residues, not more than 16 residues, not more than 15 residues, not more than 14 residues, not more than 13 residues, not more than 12 residues, or not more than 11 residues, or may be an uncontradictory combination thereof. Specifically, the length of the first base sequence may be, for example, 10 to 11 residues, 11 to 12 residues, 12 to 13 residues, 13 to 14 residues, 14 to 15 residues, 15 to 16 residues, 16 to 17 residues, 17 to 20 residues, 20 to 25 residues, 25 to 30 residues, 30 to 35 residues, 35 to 40 residues, 40 to 45 residues, 45 to 50 residues, 50 to 60 residues, 60 to 70 residues, 70 to 80 residues, 80 to 90 residues, or 90 to 100 residues. Specifically, the length of the first base sequence may be, for example, 10 to 100 residues, 10 to 50 residues, 10 to 40 residues, 12 to 30 residues, or 15 to 25 residues.

For example, each of the lengths of the second and third base sequences may be not less than 4 residues, not less than 5 residues, not less than 6 residues, not less than 7 residues, not less than 8 residues, not less than 9 residues, not less than 10 residues, not less than 11 residues, not less than 12 residues, not less than 13 residues, not less than 14 residues, not less than 15 residues, not less than 17 residues, not less than 20 residues, not less than 25 residues, not less than 30 residues, not less than 35 residues, not less than 40 residues, or not less than 45 residues, may be not more than 50 residues, not more than 45 residues, not more than 40 residues, not more than 35 residues, not more than 30 residues, not more than 25 residues, not more than 20 residues, not more than 17 residues, not more than 15 residues, not more than 14 residues, not more than 13 residues, not more than 12 residues, not more than 11 residues, not more than 10 residues, not more than 9 residues, not more than 8 residues, not more than 7 residues, not more than 6 residues, or not more than 5 residues, or may be an uncontradictory combination thereof. Specifically, each of the lengths of the second and third base sequences may be, for example, 4 to 5 residues, 5 to 6 residues, 6 to 7 residues, 7 to 8 residues, 8 to 9 residues, 9 to 10 residues, 10 to 11 residues, 11 to 12 residues, 12 to 13 residues, 13 to 14 residues, 14 to 15 residues, 15 to 17 residues, 17 to 20 residues, 20 to 25 residues, 25 to 30 residues, 30 to 35 residues, 35 to 40 residues, 40 to 45 residues, or 45 to 50 residues. Specifically, each of the lengths of the second and third base sequences may be, for example, 4 to 50 residues, 4 to 25 residues, 4 to 20 residues, 5 to 15 residues, or 7 to 13 residues.

For example, the length of the first base sequence may be 5% or more, 10% or more, 20% or more, 30% or more, 40% or more, 50% or more, 60% or more, 70% or more, 80% or more, or 90% or more, may be 100% or less, 90% or less, 80% or less, 70% or less, 60% or less, 50% or less, 40% or less, 30% or less, 20% or less, or 10% or less, or may be an uncontradictory combination thereof, as a proportion in a case in which the total of the lengths of substrate oligonucleotides on 5' and 3' sides is taken as 100%. Specifically, the length of the first base sequence may be, for example, 5 to 10%, 10 to 20%, 20 to 30%, 30 to 40%, 40 to 50%, 50 to 60%, 60 to 70%, 70 to 80%, 80 to 90%, or 90 to 100% as a proportion in a case in which the total of the lengths of substrate oligonucleotides on 5' and 3' sides is taken as 100%. Specifically, the length of the first base sequence may be, for example, 5 to 100%, 5 to 50%, 5 to 20%, 20 to 100%, 20 to 50%, or 50 to 100%. as a proportion in a case in which the total of the lengths of substrate oligonucleotides on 5' and 3' sides is taken as 100%.

For example, the length of the second base sequence may be 5% or more, 10% or more, 20% or more, 30% or more, 40% or more, 50% or more, 60% or more, 70% or more, 80% or more, or 90% or more, may be 100% or less, 90% or less, 80% or less, 70% or less, 60% or less, 50% or less, 40% or less, 30% or less, 20% or less, or 10% or less, or may be an uncontradictory combination thereof, as a proportion in a case in which the length of a substrate oligonucleotide on a 3' side is taken as 100%. Specifically, the length of the second base sequence may be, for example, 5 to 10%, 10 to 20%, 20 to 30%, 30 to 40%, 40 to 50%, 50 to 60%, 60 to 70%, 70 to 80%, 80 to 90%, or 90 to 100% as a proportion in a case in which the length of a substrate oligonucleotide on a 3' side is taken as 100%. Specifically, the length of the second base sequence may be, for example, 5 to 100%, 5 to 50%, 5 to 20%, 20 to 100%, 20 to 50%, or 50 to 100% as a proportion in a case in which the length of a substrate oligonucleotide on a 3' side is taken as 100%.

For example, the length of the third base sequence may be 5% or more, 10% or more, 20% or more, 30% or more, 40% or more, 50% or more, 60% or more, 70% or more, 80% or more, or 90% or more, may be 100% or less, 90% or less, 80% or less, 70% or less, 60% or less, 50% or less, 40% or less, 30% or less, 20% or less, or 10% or less, or may be an uncontradictory combination thereof, as a proportion in a case in which the length of a substrate oligonucleotide on a 5' side is taken as 100%. Specifically, the length of the third base sequence may be, for example, 5 to 10%, 10 to 20%, 20 to 30%, 30 to 40%, 40 to 50%, 50 to 60%, 60 to 70%, 70 to 80%, 80 to 90%, or 90 to 100% as a proportion in a case in which the length of a substrate oligonucleotide on a 5' side is taken as 100%. Specifically, the length of the third base sequence may be, for example, 5 to 100%, 5 to 50%, 5 to 20%, 20 to 100%, 20 to 50%, or 50 to 100% as a proportion in a case in which the length of a substrate oligonucleotide on a 5' side is taken as 100%.

In a case in which a substrate oligonucleotide comprises a linker, the first base sequence comprises a linker at a corresponding position. In a case in which a substrate oligonucleotide comprises a linker between nucleic acid residues A and B, "corresponding position" referred to herein means a position between nucleic acid residues a and b in the first base sequence, and the nucleic acid residues a and b are complementary nucleic acid residues of the nucleic acid residues A and B, respectively. The linker included in the substrate oligonucleotide may be the same as the linker included in the corresponding position of the first base sequence.

"Destabilizing site" means a moiety that lowers the stability of a hybrid that is formed between a complementary oligonucleotide and the oligonucleotide of interest. In other words, the stability of the hybrid that is formed between the complementary oligonucleotide and the oligonucleotide of interest is lowered by introducing a destabilizing site into the first base sequence in comparison with that before the introduction of the destabilizing site into the first base sequence. In other words, the stability of the hybrid that is formed between the complementary oligonucleotide and the oligonucleotide of interest is lower than the stability of a hybrid that is formed between a completely complementary oligonucleotide (here, meaning an oligonucleotide in which a complementary oligonucleotide is the same except to include a first base sequence instead of a destabilizing base sequence) and the oligonucleotide of interest. Examples of the lowering of the stability of the hybrid include lowering of the melting temperature (Tm) of the hybrid. For example, the amount of the lowering of the Tm due to the introduction of the destabilizing site may be 1°C or more, 2°C or more, 3°C or more, 4°C or more, 5°C or more, 6°C or more, 7°C or more, 8°C or more, 9°C or more, 10°C or more, 11°C or more, 12°C or more, 13°C or more, 14°C or more, 15°C or more, 16°C or more, 17°C or more, 18°C or more, 19°C or more, 20°C or more, 25°C or more, 30°C or more, 35°C or more, 40°C or more, 45°C or more, 50°C or more, or 55°C or more, may be 60°C or less, 55°C or less, 50°C or less, 45°C or less, 40°C or less, 35°C or less, 30°C or less, 25°C or less, 20°C or less, 19°C or less, 18°C or less, 17°C or less, 16°C or less, 15°C or less, 14°C or less, 13°C or less, 12°C or less, 11°C or less, 10°C or less, 9°C or less, 8°C or less, 7°C or less, 6°C or less, 5°C or less, 4°C or less, 3°C or less, or 2°C or less, or may be an uncontradictory combination thereof. Specifically, the amount of the lowering of Tm due to the introduction of the destabilizing site may be, for example, 1 to 2°C, 2 to 3°C, 3 to 4°C, 4 to 5°C, 5 to 6°C, 6 to 7°C, 7 to 8°C, 8 to 9°C, 9 to 10°C, 10 to 11°C, 11 to 12°C, 12 to 13°C, 13 to 14°C, 14 to 15°C, 15 to 16°C, 16 to 17°C, 17 to 18°C, 18 to 19°C, 19 to 20°C, 20 to 25°C, 25 to 30°C, 30 to 35°C, 35 to 40°C, 40 to 45°C, 45 to 50°C, 50 to 55°C, or 55 to 60°C. Specifically, the amount of the lowering of Tm due to the introduction of the destabilizing site may be, for example, 1 to 60°C, 3 to 60°C, 5 to 60°C, 10 to 60°C, 1 to 40°C, 3 to 40°C, 5 to 40°C, 10 to 40°C, 1 to 20°C, 3 to 20°C, 5 to 20°C, or 10 to 20°C.

The destabilizing site may be, for example, a destabilizing site that lowers the stability of a hybrid formed between a complementary oligonucleotide and the oligonucleotide of interest, but completely inhibits neither a hybridize between a complementary oligonucleotide and a substrate oligonucleotide on a 5' side nor a hybridize between a complementary oligonucleotide and a substrate oligonucleotide on a 3' side.

The destabilizing site is not particularly limited as long as the destabilizing site improves the efficiency of generating the oligonucleotide of interest. Examples of improvement in the efficiency of generating the oligonucleotide of interest include an increase in the molar ratio of the amount of the generated oligonucleotide of interest to the used amount of a complementary oligonucleotide.

Examples of the destabilizing site include abasic sites, mismatch sites, insertions of nucleic acid residues, deletions of nucleic acid residues, insertions of linkers, deletions of linkers, and substitutions of linkers.

### "Abasic site" means the following (1) or (2):

(1) nucleic acid residues in which no base portion is present (that is, abasic nucleic acid residues); or
(2) nucleic acid residues which have an optional base portion and in which the nucleic acid residues of a corresponding substrate oligonucleotide are abasic nucleic acid residues.

In other words, the nucleic acid residues of either or both of a complementary oligonucleotide and a substrate oligonucleotide may be abasic nucleic acid residues in the abasic site. In particular, the nucleic acid residues of at least a complementary oligonucleotide may be abasic nucleic acid residues in the abasic site.

"Mismatch site" means nucleic acid residues having a base portion in which a base pair is mismatched with a corresponding substrate oligonucleotide. The phrase "base pair is mismatched" may mean that a base portion is incapable of forming a base pair between base sequences, or that the strength of a base pair formed between base sequences by a base portion is equivalent to or less than that of a Watson-Crick base pair. Examples of combinations of bases considered to be mismatches include combinations of bases that are selected from the bases (which may be modified) described as examples above and are incapable of forming base pairs, and combinations of bases that are selected from bases (which may be modified) described as examples above and are capable of base pairs having strengths that are equivalent to or less than that of a Watson-Crick base pair. Specific examples of combinations of bases considered to be mismatches include a combination of A and A, a combination of A and G, a combination of A and C, a combination of T and T, a combination of T and G, a combination of T and C, a combination of T and U, a combination of U and U, a combination of U and G, a combination of U and C, a combination of G and G, a combination of C and C, a combination of I and U, a combination of I and A, and a combination of I and C (I is hypoxanthine). Particular examples of combinations of bases considered to be mismatches include combinations of bases, other than combinations of bases forming wobble base pairs. Examples of the combinations of the bases forming wobble base pairs include a combination of U and G, a combination of I and U, a combination of I and A, and a combination of I and C. Examples of the combinations of the bases, other than the combinations of the bases forming wobble base pairs include a combination A and A, a combination of A and G, a combination of A and C, a combination of T and T, a combination of T and G, a combination of T and C, a combination of T and U, a combination of U and U, a combination of U and C, a combination of G and G, and a combination of C and C.

"Insertion of nucleic acid residue" means that a nucleic acid residue is inserted between nucleic acid residues.

"Deletion of nucleic acid residue" means that a nucleic acid residue is deleted.

"Insertion of linker" means that a linker is inserted between nucleic acid residues. Such a linker is as described above in a modification of a phosphate portion. For example, both ends of the linker may bind to a phosphate group. In other words, the linker may bind to, for example, the phosphate group at position 3' of a nucleic acid residue on the 5' side thereof, and the phosphate group at position 5' of a nucleic acid residue on the 3' side thereof. An insertion of a linker may be combined with, for example, a deletion of a nucleic acid residue. In other words, for example, a nucleic acid residue may be substituted with a linker. In other words, for example, nucleic acid residues on both the ends of a deleted nucleic acid residue may be directly ligated, or may be ligated through a linker. In other words, a deletion of a nucleic acid residue may occur or need not occur together with an insertion of a linker. "Deletion of nucleic acid residue occurring together with insertion of linker" means a substitution of a nucleic acid residue with a linker. "Deletion of nucleic acid residue occurring without insertion of linker" means a deletion of a nucleic acid residue, other than a substitution of a nucleic acid residue with a linker. An insertion of a linker may also be combined with a destabilizing site other than a deletion of a nucleic acid residue, such as, for example, an insertion of a nucleic acid residue. In other words, for example, a linker and a nucleic acid residue ligated thereto may also be collectively inserted.

"Deletion of linker" means that a linker is deleted. A deletion of a linker can be selected in a case in which a first base sequence comprises a linker. A deletion of a linker may be combined with, for example, an insertion of a nucleic acid residue. In other words, for example, a linker may be substituted with a nucleic acid residue. In other words, an insertion of a nucleic acid residue may occur or need not occur together with a deletion of a linker. "Insertion of nucleic acid residue occurring together with deletion of linker" means a substitution of a linker with a nucleic acid residue. "Insertion of nucleic acid residue occurring without deletion of linker" means an insertion of a nucleic acid residue, other than a substitution of a linker with a nucleic acid residue. An insertion of a linker may also be combined with a destabilizing site other than an insertion of a nucleic acid residue, such as, for example, a deletion of a nucleic acid residue. In other words, for example, a linker and a nucleic acid residue ligated thereto may also be collectively deleted.

"Substitution of linker" means that a linker is substituted with another kind of a linker. A substitution of a linker can be selected in a case in which a first base sequence comprises a linker. For example, the length of the linker with which the substitution is performed may be different from the length of the substituted linker (that is, may be more or less than the length of the substituted linker).

Each of such destabilizing sites as described in examples above may function singly as a destabilizing site. However, in a case in which two or more destabilizing sites are used in combination, each destabilizing site included in the combination may function or need not function singly as a destabilizing site as long as the combination functions as a destabilizing site as a whole.

As the destabilizing site, one kind of a destabilizing site may be used, or two or more kinds of destabilizing sites may be used in combination. For example, the destabilizing site may comprise, or need not comprise each destabilizing site described as an example above. The destabilizing site may comprise, for example, one or more destabilizing sites selected from the destabilizing sites described as examples above. Specifically, the destabilizing site may comprise, for example, an abasic site, a mismatch site, an insertion of a nucleic acid residue, a deletion of a nucleic acid residue, an insertion of a linker, a deletion of a linker, a substitution of a linker, or a combination thereof. The phrase "destabilizing site comprises certain destabilizing site A" means that at least the destabilizing site A is selected as the destabilizing site, and encompasses a case in which the destabilizing site consists of the destabilizing site A, and a case in which the destabilizing site consists of a combination of the destabilizing site A and another destabilizing site. For example, the phrase "destabilizing site comprises abasic site" means that at least the abasic site is selected as the destabilizing site, and encompasses a case in which the destabilizing site consists of the abasic site, and a case in which the destabilizing site consists of a combination of the abasic site and another destabilizing site. The phrase "destabilizing site comprises certain destabilizing sites A" means that at least the destabilizing sites A are selected as destabilizing sites, and the number of, or the number of sets of the destabilizing sites A included in the destabilizing site is limited to the specified number or the specified number of the sets in a case in which the number of, or the number of the sets of the destabilizing sites A are specified (here, the indefinite article (for example, "a" or "an") is not considered to specify the number or the number of the sets). For example, the phrase "destabilizing site comprises an abasic site" means that at least the abasic site is selected as the destabilizing site, and the number of the abasic site included in the destabilizing site is limited to one, and encompasses a case in which the destabilizing site consists of the abasic site whose number is one, and a case in which the destabilizing site consists of a combination of the abasic site whose number is one and another destabilizing site.

For example, the destabilizing site may comprise, or need not comprise each destabilizing site described as an example above. For example, the destabilizing site may comprise, or need not comprise an abasic site. For example, the destabilizing site may comprise, or need not comprise a mismatch site. For example, the destabilizing site may comprise, or need not comprise an insertion of a nucleic acid residue. For example, the destabilizing site may comprise, or need not comprise a deletion of a nucleic acid residue. For example, the destabilizing site may comprise, or need not comprise an insertion of a linker. For example, the destabilizing site may comprise, or need not comprise a deletion of a linker. For example, the destabilizing site may comprise, or need not comprise a substitution of a linker.

The introduction modes (for example, the kind, number, number of sets, and introduction position) of the destabilizing sites are not particularly limited as long as the destabilizing sites improve the efficiency of generating the oligonucleotide of interest. The introduction modes of the destabilizing sites can be set as appropriate depending on, for example, various conditions such as the structure of a substrate oligonucleotide.

"Number of destabilizing sites" means the number of nucleic acid residues and linkers corresponding to destabilizing sites included in a destabilizing base sequence. The number of optionally selected destabilizing sites (for example, any one or more kinds of the destabilizing sites described as examples above) with respect to the total number of destabilizing sites may be, for example, 10% or more, 20% or more, 30% or more, 40% or more, 50% or more, 60% or more, 70% or more, 80% or more, 90% or more, 95% or more, or 100%.

"Number of sets of destabilizing sites" means the number of sets of destabilizing sites included in a destabilizing base sequence. "Set of destabilizing sites" means a destabilizing site that is not consecutive to a destabilizing site of which the kind is the same as that of the destabilizing site, or two or more consecutive destabilizing sites of which the kinds are the same. With regard to "set of destabilizing sites", the kinds of abasic sites are considered to be the same, the kinds of mismatch sites are considered to be the same, the kinds of insertions of nucleic acid residues are considered to be the same, the kinds of deletions of nucleic acid residues are considered to be the same, the kinds of insertions of linkers are considered to be the same, the kinds of deletions of linkers are considered to be the same, and the kinds of substitutions of linkers are considered to be the same. "Two or more insertions of linkers, two or more deletions of linkers, or two or more substitutions of linkers are consecutive" means that two or more nucleic acid residues into which insertions of linkers, deletions of linkers, or substitutions of linkers are introduced on 5' sides are consecutive. The number of sets of optionally selected destabilizing sites (for example, any one or more kinds of the destabilizing sites described as examples above) with respect to the total number of sets of destabilizing sites may be, for example, 10% or more, 20% or more, 30% or more, 40% or more, 50% or more, 60% or more, 70% or more, 80% or more, 90% or more, 95% or more, or 100%.

For example, the number of sets of destabilizing sites may be 1 or more, 2 or more, 3 or more, 4 or more, 5 or more, 6 or more, 7 or more, 8 or more, 9 or more, 10 or more, 12 or more, or 15 or more, may be 20 or less, 15 or less, 12 or less, 10 or less, 9 or less, 8 or less, 7 or less, 6 or less, 5 or less, 4 or less, 3 or less, or 2 or less, or may be an uncontradictory combination thereof. Specifically, the number of sets of destabilizing sites may be, for example, 1 to 2, 2 to 3, 3 to 4, 4 to 5, 5 to 6, 6 to 7, 7 to 8, 8 to 9, 9 to 10, 10 to 12, 12 to 15, or 15 to 20. Specifically, the number of sets of destabilizing sites may be, for example, 1 to 20, 1 to 10, 1 to 8, 1 to 7, 1 to 6, 1 to 5, 1 to 4, 1 to 3, 1 to 2, or 1.

For example, the number of sets of destabilizing sites (or, for example, the number of sets of destabilizing sites, other than insertions of linkers, deletions of linkers, and substitutions of linkers) may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of sets of destabilizing sites (or, for example, the number of sets of destabilizing sites, other than insertions of linkers, deletions of linkers, and substitutions of linkers) may be, for example, 1 to 2, 2 to 3, 3 to 4, 4 to 5, 5 to 6, 6 to 7, or 7 to 8. Specifically, the number of sets of destabilizing sites (or, for example, the number of sets of destabilizing sites, other than insertions of linkers, deletions of linkers, and substitutions of linkers) may be, for example, 1 to 8, 1 to 7, 1 to 6, 1 to 5, 1 to 4, 1 to 3, 1 to 2, or 1. The number of sets of destabilizing sites (or, for example, the number of sets of destabilizing sites, other than insertions of linkers, deletions of linkers, and substitutions of linkers) may be, particularly, 1, 2, or 3. In accordance with one aspect, the number of sets of destabilizing sites (or, for example, the number of sets of destabilizing sites, other than insertions of linkers, deletions of linkers, and substitutions of linkers) may be still more particularly 1 or 2. In accordance with one aspect, the number of sets of destabilizing sites (or, for example, the number of sets of destabilizing sites, other than insertions of linkers, deletions of linkers, and substitutions of linkers) may be still more particularly 3. For example, the proportion of the number of sets of destabilizing sites (or, for example, the number of sets of destabilizing sites, other than insertions of linkers, deletions of linkers, and substitutions of linkers) to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of sets of destabilizing sites (or, for example, the number of sets of destabilizing sites, other than insertions of linkers, deletions of linkers, and substitutions of linkers) to the length of a first base sequence (the number of residues) may be, for example, 5 to 10%, 10 to 15%, 15 to 20%, 20 to 25%, or 25 to 30%. Specifically, the proportion of the number of sets of destabilizing sites (or, for example, the number of sets of destabilizing sites, other than insertions of linkers, deletions of linkers, and substitutions of linkers) to the length of a first base sequence (the number of residues) may be, for example, 5 to 30%, 5 to 20%, or 5 to 15%.

For example, in a case in which, as destabilizing sites, a nucleic acid residue at the position A of a first base sequence is substituted with a linker, a nucleic acid residue to which a linker is ligated is inserted into the position B of the first base sequence, and four consecutive nucleic acid residues to which any linker is not ligated are inserted into the position C of the first base sequence (here, the positions A, B, and C are not adjacent to each other), the number of the destabilizing sites is 7 (specifically, the number of a deletion of the nucleic acid residue is 1, the number of insertions of the nucleic acid residues is 5, and the number of insertions of the linkers is 2), and the number of sets of the destabilizing sites is 5 (specifically, the number of a set of the deletion of the nucleic acid residue is 1, the number of sets of the insertions of the nucleic acid residues is 2, and the number of sets of the insertions of the linker is 2).

Unless otherwise specified, the introduction modes of destabilizing sites are interpreted based on the following criteria.
(1) Such a mode is interpreted so that the number of destabilizing sites is minimum (but 1 or more).
(2) Such a mode is interpreted so that the number of sets of destabilizing sites is minimum (but 1 or more) in a case in which the numbers of destabilizing sites are equal.
(3) Such a mode is interpreted so that in a case in which the numbers of destabilizing sites and the numbers of sets of the destabilizing sites are equal, the introduction positions of the destabilizing sites are set at positions -X or positions X (X is a positive integer; but X may be zero in the case of inserting nucleic acid residues), and the total of X is minimum.

The introduction positions of destabilizing sites are specified as positions in a first base sequence unless otherwise specified. With respect to a ligation site between second and third base sequences, the positions in the first base sequence on the 5' side of the ligation site (that is, a second base sequence side) are denoted by consecutive negative numbers from -1, and the positions in the first base sequence on the 3' side of the ligation site (that is, a third base sequence side) are denoted by consecutive positive numbers from +1, unless otherwise specified. With regard to the ligation site between the second and third base sequences, a position on the 5' side of the ligation site (that is, the second base sequence side) is also referred to as "negative position" or "position on second base sequence side". With regard to the ligation site between the second and third base sequences, a position on the 3' side of the ligation site (that is, the third base sequence side) is also referred to as "positive position" or "position on third base sequence side". In other words, for example, in a case in which each of the second and third base sequences is a base sequence with 10 residues, the nucleic acid residue on the 5' end of the second base sequence is at position -10, the nucleic acid residue on the 3' end of the second base sequence is at position -1, the nucleic acid residue on the 5' end of the third base sequence is at position +1, the nucleic acid residue on the 3' end of the third base sequence is at position +10, and the nucleic acid residues at position -1 and position +1 are ligated to each other. In the case of inserting a nucleic acid residue, the phrase "insertion position of nucleic acid residue is position -X" (X is a positive integer) means that the nucleic acid residue is inserted between a nucleic acid residue at position -X and a nucleic acid residue at position -(X + 1), unless otherwise specified. In the case of inserting a nucleic acid residue, the phrase "insertion position of nucleic acid residue is position +X" (X is a positive integer) means that the nucleic acid residue is inserted between a nucleic acid residue at position +X and a nucleic acid residue at position +(X + 1), unless otherwise specified. In the case of inserting a nucleic acid residue, the phrase "insertion position of nucleic acid residue is position 0" means that the nucleic acid residue is inserted between a nucleic acid residue at position -1 and a nucleic acid residue at position +1, unless otherwise specified. In the case of an insertion of a linker, a deletion of a linker, or a substitution of a linker, the introduction position of a destabilizing site is specified as the position of a nucleic acid residue having a 5' side into which the insertion of the linker, the deletion of the linker, or the substitution of the linker is introduced, unless otherwise specified. In other words, for example, the phrase "insertion position of linker is position +X" (X is positive integer) means that a linker is inserted into the 5' side of a nucleic acid residue at position +X (that is, between a nucleic acid residue at position +X and a nucleic acid residue at position +(X - 1)), unless otherwise specified.

For example, a destabilizing site may be introduced into either or both of second and third base sequences. In a case in which the number of destabilizing sites is 2 or more, the introduction positions of the destabilizing sites may be independently selected for each destabilizing site, unless otherwise specified. The introduction positions of the destabilizing sites may be, for example, position -1, position +1, the nucleic acid residue on the 5' end of a first base sequence, the nucleic acid residue on the 3' end of the first base sequence, or other positions. The introduction positions of the destabilizing sites may be, for example, positions -25 to +25, positions -20 to +20, positions -17 to +17, positions -15 to +15, positions -14 to +14, positions -13 to +13, positions -12 to +12, positions -11 to +11, positions -10 to +10, positions -9 to +9, positions -8 to +8, positions -7 to +7, positions -6 to +6, positions -5 to +5, positions -4 to +4, positions -3 to +3, positions -2 to +2, or positions -1 to +1. The introduction positions of the destabilizing sites may be, for example, positions -1 to -4, positions -2 to -5, positions -3 to -6, positions -4 to -7, positions -5 to -8, positions -6 to -9, positions -7 to -10, positions -8 to -11, positions -9 to -12, positions -10 to -13, positions -11 to -14, positions -12 to -15, positions -13 to -16, positions -14 to -17, positions -15 to -18, positions -16 to -19, positions -17 to -20, positions -18 to -21, positions -19 to -22, positions -20 to -23, positions -21 to - 24, or positions -22 to -25. The introduction positions of the destabilizing sites may be, for example, positions +1 to +4, positions +2 to +5, positions +3 to +6, positions +4 to +7, positions +5 to +8, positions +6 to +9, positions +7 to +10, positions +8 to +11, positions +9 to +12, positions +10 to +13, positions +11 to +14, positions +12 to +15, positions +13 to +16, positions +14 to +17, positions +15 to +18, positions +16 to +19, positions +17 to +20, positions +18 to +21, positions +19 to +22, positions +20 to +23, positions +21 to +24, positions +22 to +25, positions +23 to +26, or positions +24 to +27. The introduction position of the destabilizing site may be, for example, position -25, position -24, position -23, position -22, position -21, position - 20, position -19, position -18, position -17, position -16, position -15, position -14, position -13, position -12, position -11, position -10, position -9, position -8, position -7, position -6, position -5, position -4, position -3, position -2, position -1, position +1, position +2, position +3, position +4, position +5, position +6, position +7, position +8, position +9, position +10, position +11, position +12, position +13, position +14, position +15, position +16, position +17, position +18, position +19, position +20, position +21, position +22, position +23, position +24, or position +25.

For example, not less than 4, not less than 5, not less than 6, not less than 7, not less than 8, not less than 9, not less than 10, not less than 11, not less than 12, not less than 13, not less than 14, not less than 15, not less than 16, not less than 17, not less than 18, not less than 19, or not less than 20 nucleic acid residues of nucleic acid residues included in a second base sequence may remain in a destabilizing base sequence. For example, 50% or more, 60% or more, 70% or more, 80% or more, 90% or more, or 95% or more of nucleic acid residues of the nucleic acid residues included in the second base sequence may remain in the destabilizing base sequence. For example, not less than 2, not less than 3, not less than 4, not less than 5, or not less than 6 consecutive nucleic acid residues of the nucleic acid residues included in the second base sequence may remain in the destabilizing base sequence.

For example, not less than 4, not less than 5, not less than 6, not less than 7, not less than 8, or not less than 9 nucleic acid residues of the nucleic acid residues at positions -1 to -10 of the second base sequence may remain in the destabilizing base sequence. For example, not less than 2, not less than 3, not less than 4, not less than 5, or not less than 6 consecutive nucleic acid residues of the nucleic acid residues at positions -1 to -10 of the second base sequence may remain in the destabilizing base sequence.

For example, not less than 4, not less than 5, not less than 6, not less than 7, not less than 8, not less than 9, not less than 10, not less than 11, not less than 12, not less than 13, not less than 14, not less than 15, not less than 16, not less than 17, not less than 18, not less than 19, or not less than 20 nucleic acid residues of nucleic acid residues included in a third base sequence may remain in the destabilizing base sequence. For example, 50% or more, 60% or more, 70% or more, 80% or more, 90% or more, or 95% or more of nucleic acid residues of the nucleic acid residues included in the third base sequence may remain in the destabilizing base sequence. For example, not less than 2, not less than 3, not less than 4, not less than 5, or not less than 6 consecutive nucleic acid residues of the nucleic acid residues included in the third base sequence may remain in the destabilizing base sequence.

For example, not less than 4, not less than 5, not less than 6, not less than 7, not less than 8, or not less than 9 nucleic acid residues of the nucleic acid residues at positions +1 to +10 of the third base sequence may remain in the destabilizing base sequence. For example, not less than 2, not less than 3, not less than 4, not less than 5, or not less than 6 consecutive nucleic acid residues of the nucleic acid residues at positions +1 to +10 of the third base sequence may remain in the destabilizing base sequence.

The phrase "nucleic acid residue remains in destabilizing base sequence" means that the nucleic acid residue is not substituted with a destabilizing site. None of an insertion of a nucleic acid residue, an insertion of a linker, a deletion of a linker, and a substitution of a linker changes an original nucleic acid residue, and therefore influences the number of nucleic acid residues remaining in the second or third base sequences, and the number of consecutive nucleic acid residues remaining in the second or third base sequences.

For example, the number of abasic sites may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the abasic sites may be, for example, 1 to 2, 2 to 3, 3 to 4, 4 to 5, 5 to 6, 6 to 7, or 7 to 8. Specifically, the number of the abasic sites may be, for example, 1 to 8, 1 to 7, 1 to 6, 1 to 5, 1 to 4, 1 to 3, 1 to 2, or 1. The number of the abasic sites may be particularly 1, 2, or 3. The number of the abasic sites may be still more particularly 1 or 2. For example, the proportion of the number of the abasic sites to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the abasic sites to the length of the first base sequence (the number of residues) may be, for example, 5 to 10%, 10 to 15%, 15 to 20%, 20 to 25%, or 25 to 30%. Specifically, the proportion of the number of the abasic sites to the length of the first base sequence (the number of residues) may be, for example, 5 to 30%, 5 to 20%, or 5 to 15%.

For example, the number of sets of abasic sites may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the sets of the abasic sites may be, for example, 1 to 2, 2 to 3, 3 to 4, 4 to 5, 5 to 6, 6 to 7, or 7 to 8. Specifically, the number of the sets of the abasic sites may be, for example, 1 to 8, 1 to 7, 1 to 6, 1 to 5, 1 to 4, 1 to 3, 1 to 2, or 1. The number of the sets of the abasic sites may be particularly 1, 2, or 3. The number of the sets of the abasic sites may be still more particularly 1 or 2. For example, the proportion of the number of the sets of the abasic sites to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the sets of the abasic sites to the length of the first base sequence (the number of residues) may be, for example, 5 to 10%, 10 to 15%, 15 to 20%, 20 to 25%, or 25 to 30%. Specifically, the proportion of the number of the sets of the abasic sites to the length of the first base sequence (the number of residues) may be, for example, 5 to 30%, 5 to 20%, or 5 to 15%.

For example, the number of abasic sites included in a set of abasic sites may be 1 or more, 2 or more, 3 or more, or 4 or more, may be 5 or less, 4 or less, 3 or less, or 2 or less, or may be an uncontradictory combination thereof. Specifically, the number of abasic sites included in a set of abasic sites may be, for example, 1 to 2, 2 to 3, 3 to 4, or 4 to 5. Specifically, the number of abasic sites included in a set of abasic sites may be, for example, 1 to 5, 1 to 4, 1 to 3, 1 to 2, or 1. The number of abasic sites included in a set of abasic sites may be particularly 1 or 2. The number of abasic sites included in a set of abasic sites may be still more particularly 1. In a case in which the number of sets of abasic sites is 2 or more, the number of abasic sites included in a set of abasic sites may be independently selected for each set of abasic sites, unless otherwise specified.

In a case in which the number of abasic sites is 2 or more, the introduction positions of the abasic sites may be independently selected for each abasic site, unless otherwise specified. The introduction positions of the abasic sites may be, for example, the introduction positions of the destabilizing sites described as examples above. In particular, the introduction positions of the abasic sites may be positions -1 to +1, or may be other positions. In accordance with an aspect, a first abasic site may be introduced into position -1 or position +1, and optionally, a second abasic site may be further introduced into a position (for example, positions -5 to -7 or positions +5 to +7) that is not adjacent to the first abasic nucleic acid.

For example, the number of mismatch sites may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the mismatch sites may be, for example, 1 to 2, 2 to 3, 3 to 4, 4 to 5, 5 to 6, 6 to 7, or 7 to 8. Specifically, the number of the mismatch sites may be, for example, 1 to 8, 1 to 7, 1 to 6, 1 to 5, 1 to 4, 1 to 3, 1 to 2, or 1. The number of the mismatch sites may be particularly 1, 2, or 3. The number of a mismatch site may be still more particularly 1. For example, the proportion of the number of the mismatch sites to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the mismatch sites to the length of the first base sequence (the number of residues) may be, for example, 5 to 10%, 10 to 15%, 15 to 20%, 20 to 25%, or 25 to 30%. Specifically, the proportion of the number of the mismatch sites to the length of the first base sequence (the number of residues) may be, for example, 5 to 30%, 5 to 20%, or 5 to 15%.

For example, the number of sets of mismatch sites may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the sets of the mismatch sites may be, for example, 1 to 2, 2 to 3, 3 to 4, 4 to 5, 5 to 6, 6 to 7, or 7 to 8. Specifically, the number of the sets of the mismatch sites may be, for example, 1 to 8, 1 to 7, 1 to 6, 1 to 5, 1 to 4, 1 to 3, 1 to 2, or 1. The number of the sets of the mismatch sites may be particularly 1, 2, or 3. The number of a set of a mismatch site may be still more particularly 1. For example, the proportion of the sets of the number of the mismatch sites to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the sets of the mismatch sites to the length of the first base sequence (the number of residues) may be, for example, 5 to 10%, 10 to 15%, 15 to 20%, 20 to 25%, or 25 to 30%. Specifically, the proportion of the number of the sets of the mismatch sites to the length of the first base sequence (the number of residues) may be, for example, 5 to 30%, 5 to 20%, or 5 to 15%.

For example, the number of mismatch sites included in a set of mismatch sites may be 1 or more, 2 or more, 3 or more, or 4 or more, may be 5 or less, 4 or less, 3 or less, or 2 or less, or may be an uncontradictory combination thereof. Specifically, the number of mismatch sites included in a set of mismatch sites may be, for example, 1 to 2, 2 to 3, 3 to 4, or 4 to 5. Specifically, the number of mismatch sites included in a set of mismatch sites may be, for example, 1 to 5, 1 to 4, 1 to 3, 1 to 2, or 1. The number of mismatch sites included in a set of mismatch sites may be particularly 1 or 2. The number of mismatch sites included in a set of mismatch sites may be still more particularly 1. In a case in which the number of sets of mismatch sites is 2 or more, the number of mismatch sites included in a set of mismatch sites may be independently selected for each set of mismatch sites, unless otherwise specified.

In a case in which the number of mismatch sites is 2 or more, the introduction positions of the mismatch sites may be independently selected for each mismatch site, unless otherwise specified. The introduction positions of the mismatch sites may be, for example, the introduction positions of the destabilizing sites described as examples above. The introduction positions of the mismatch sites may be particularly positions -2 to +2. The introduction positions of the mismatch sites may be still more particularly positions -1 to +1.

For example, the number of insertions of nucleic acid residues (that is, the number of inserted nucleic acid residues) may be 1 or more, 2 or more, 3 or more, 4 or more, 5 or more, 6 or more, 7 or more, 8 or more, 9 or more, 10 or more, 12 or more, 15 or more, 20 or more, 25 or more, 30 or more, 40 or more, or 50 or more, may be 60 or less, 50 or less, 40 or less, 30 or less, 25 or less, 20 or less, 15 or less, 12 or less, 10 or less, 9 or less, 8 or less, 7 or less, 6 or less, 5 or less, 4 or less, 3 or less, or 2 or less, or may be an uncontradictory combination thereof. Specifically, the number of the insertions of the nucleic acid residues may be, for example, 1 to 2, 2 to 3, 3 to 4, 4 to 5, 5 to 6, 6 to 7, 7 to 8, 8 to 9, 9 to 10, 10 to 12, 12 to 15, 15 to 20, 20 to 25, 25 to 30, 30 to 40, 40 to 50, or 50 to 60. Specifically, the number of the insertions of the nucleic acid residues may be, for example, 1 to 60, 1 to 50, 1 to 40, 1 to 30, 1 to 20, 1 to 10, 1 to 8, 1 to 7, 1 to 6, 1 to 5, 1 to 4, 1 to 3, 1 to 2, or 1. The number of the insertions of the nucleic acid residues may be particularly 1, 2, 3, 4, 5, 6, 7, 8, 9, 10, 11, or 12. For example, the proportion of the number of the insertions of the nucleic acid residues to the length of a first base sequence (the number of residues) may be 5% or more, 10% or more, 20% or more, 30% or more, 40% or more, 50% or more, 60% or more, 70% or more, 80% or more, 90% or more, 100% or more, 110% or more, 120% or more, 130% or more, 140% or more, 150% or more, 170% or more, 200% or more, or 250% or more, may be 300% or less, 250% or less, 200% or less, 170% or less, 150% or less, 140% or less, 130% or less, 120% or less, 110% or less, 100% or less, 90% or less, 80% or less, 70% or less, 60% or less, 50% or less, 40% or less, 30% or less, 20% or less, or 10% or less, or may be an uncontradictory combination thereof. Specifically, the proportion of the number of the insertions of the nucleic acid residues to the length of the first base sequence (the number of residues) may be, for example, 5 to 10%, 10 to 20%, 20 to 30%, 30 to 40%, 40 to 50%, 50 to 60%, 60 to 70%, 70 to 80%, 80 to 90%, 90 to 100%, 100 to 110%, 110 to 120%, 120 to 130%, 130 to 140%, 140 to 150%, 150 to 170%, 170 to 200%, 200 to 250%, or 250 to 300%. Specifically, the proportion of the number of the insertions of the nucleic acid residues to the length of the first base sequence (the number of residues) may be, for example, 5 to 300%, 5 to 200%, 5 to 150%, 5 to 100%, or 5 to 50%.

For example, the number of sets of insertions of nucleic acid residues may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the sets of the insertions of the nucleic acid residues may be, for example, 1 to 2, 2 to 3, 3 to 4, 4 to 5, 5 to 6, 6 to 7, or 7 to 8. Specifically, the number of the sets of the insertions of the nucleic acid residues may be, for example, 1 to 8, 1 to 7, 1 to 6, 1 to 5, 1 to 4, 1 to 3, 1 to 2, or 1. The number of the sets of the insertions of the nucleic acid residues may be particularly 1, 2, or 3. The number of the sets of the insertions of the nucleic acid residues may be still more particularly 2. For example, the proportion of the number of the sets of the insertions of the nucleic acid residues to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the sets of the insertions of the nucleic acid residues to the length of the first base sequence (the number of residues) may be, for example, 5 to 10%, 10 to 15%, 15 to 20%, 20 to 25%, or 25 to 30%. Specifically, the proportion of the number of the sets of the insertions of the nucleic acid residues to the length of the first base sequence (the number of residues) may be, for example, 5 to 30%, 5 to 20%, or 5 to 15%.

For example, the number of insertions of nucleic acid residues included in a set of insertions of nucleic acid residues may be 1 or more, 2 or more, 3 or more, 4 or more, 5 or more, 6 or more, 7 or more, 8 or more, 9 or more, 10 or more, 12 or more, 15 or more, 20 or more, or 25 or more, may be 30 or less, 25 or less, 20 or less, 15 or less, 12 or less, 10 or less, 9 or less, 8 or less, 7 or less, 6 or less, 5 or less, 4 or less, 3 or less, or 2 or less, or may be an uncontradictory combination thereof. The number of insertions of nucleic acid residues included in a set of insertions of nucleic acid residues may be particularly 2 or more. Specifically, the number of insertions of nucleic acid residues included in a set of insertions of nucleic acid residues may be, for example, 1 to 2, 2 to 3, 3 to 4, 4 to 5, 5 to 6, 6 to 7, 7 to 8, 8 to 9, 9 to 10, 10 to 12, 12 to 15, 15 to 20, 20 to 25, or 25 to 30. Specifically, the number of insertions of nucleic acid residues included in a set of insertions of nucleic acid residues may be, for example, 1 to 30, 1 to 20, 1 to 10, 1 to 8, 1 to 7, 1 to 6, 1 to 5, 1 to 4, 1 to 3, 1 to 2, or 1. The number of insertions of nucleic acid residues included in a set of insertions of nucleic acid residues may be particularly 1, 2, 3, 4, 5, 6, 7, or 8. In a case in which the number of sets of insertions of nucleic acid residues is 2 or more, the number of insertions of nucleic acid residues included in a set of insertions of nucleic acid residues may be independently selected for each set of insertions of nucleic acid residues, unless otherwise specified.

In a case in which the number of insertions of nucleic acid residues is 2 or more, the introduction positions of the insertions of the nucleic acid residues may be independently selected for an insertion of each nucleic acid residue, unless otherwise specified. The introduction positions of the insertions of the nucleic acid residues may be, for example, the introduction positions of the destabilizing sites described as examples above. The introduction positions of the insertions of the nucleic acid residues may be particularly positions -3 to -6 or positions +3 to +6. The introduction position of the insertion of the nucleic acid residue may be still more particularly position -5 or position +5.

In a case in which the number of the insertions of the nucleic acid residues is 2 or more, the kinds of the bases of the inserted nucleic acid residues may be independently selected for each nucleic acid residue, unless otherwise specified. The kinds of the bases of the inserted nucleic acid residues are not particularly limited. The bases of the inserted nucleic acid residues may be any bases, and the nucleic acid residues may be abasic. In the case of inserting two or more consecutive nucleic acid residues (hereinafter also referred to as "consecutive inserted residues"), a part or all of the consecutive inserted residues may have self-complementarity. For example, 50% or more, 60% or more, 70% or more, 80% or more, or 90% or more of the nucleic acid residues included in the consecutive inserted residues may have self-complementarity. For example, at least the 5'-end nucleic acid residue and 3'-end nucleic acid residue of the consecutive inserted residues may have, or need not have self-complementarity. For example, one, two, three, or four or more nucleic acid residues from the 5' end of the consecutive inserted residues and one, two, three, or four or more nucleic acid residues from the 3' end of the consecutive inserted residues may have self-complementarity. Specifically, for example, in a case in which the consecutive inserted residues are TTGAA, 80% (= four residues/five residues) of the nucleic acid residues included in the consecutive inserted residues have self-complementarity, and the two nucleic acid residues from the 5' end of the consecutive inserted residues and the two nucleic acid residues from the 3' end have self-complementarity. For example, a part or all of the consecutive inserted residues may form a double-stranded structure on the basis of self-complementarity. Inserted nucleic acid residues (particularly, consecutive inserted residues) are also referred to as "loop" or "loop structure".

For example, the number of deletions of nucleic acid residues (that is, the number of deleted nucleic acid residues) may be 1 or more, 2 or more, 3 or more, 4 or more, 5 or more, 6 or more, 7 or more, 8 or more, 9 or more, 10 or more, 12 or more, 15 or more, 20 or more, 25 or more, 30 or more, 40 or more, or 50 or more, may be 60 or less, 50 or less, 40 or less, 30 or less, 25 or less, 20 or less, 15 or less, 12 or less, 10 or less, 9 or less, 8 or less, 7 or less, 6 or less, 5 or less, 4 or less, 3 or less, or 2 or less, or may be an uncontradictory combination thereof. Specifically, the number of the deletions of the nucleic acid residues may be, for example, 1 to 2, 2 to 3, 3 to 4, 4 to 5, 5 to 6, 6 to 7, 7 to 8, 8 to 9, 9 to 10, 10 to 12, 12 to 15, 15 to 20, 20 to 25, 25 to 30, 30 to 40, 40 to 50, or 50 to 60. Specifically, the number of the deletions of the nucleic acid residues may be, for example, 1 to 60, 1 to 50, 1 to 40, 1 to 30, 1 to 20, 1 to 10, 1 to 8, 1 to 7, 1 to 6, 1 to 5, 1 to 4, 1 to 3, 1 to 2, or 1. The number of the deletions of the nucleic acid residues may be particularly 1, 2, 3, 4, 5, 6, 7, 8, 9, 10, 11, or 12. The number of the deletions of the nucleic acid residues may be still more particularly 1, 2, or 3. For example, the proportion of the number of the deletions of the nucleic acid residues to the length of a first base sequence (the number of residues) may be 5% or more, 10% or more, 15% or more, 20% or more, 25% or more, 30% or more, 40% or more, 50% or more, 60% or more, or 70% or more, may be 80% or less, 70% or less, 60% or less, 50% or less, 40% or less, 30% or less, 25% or less, 20% or less, 15% or less, or 10% or less, or may be an uncontradictory combination thereof. Specifically, the proportion of the number of the deletions of the nucleic acid residues to the length of the first base sequence (the number of residues) may be, for example, 5 to 10%, 10 to 15%, 15 to 20%, 20 to 25%, 25 to 30%, 30 to 40%, 40 to 50%, 50 to 60%, 60 to 70%, or 70 to 80%. Specifically, the proportion of the number of the deletions of the nucleic acid residues to the length of the first base sequence (the number of residues) may be, for example, 5 to 80%, 5 to 50%, 5 to 30%, or 5 to 20%.

For example, the number of sets of deletions of nucleic acid residues may be 1 or more, 2 or more, or 3 or more, may be 4 or less, 3 or less, or 2 or less, or may be an uncontradictory combination thereof. Specifically, the number of the sets of the deletions of the nucleic acid residues may be, for example, 1 to 2, 2 to 3, or 3 to 4. Specifically, the number of the sets of the deletions of the nucleic acid residues may be, for example, 1 to 4, 1 to 3, 1 to 2, or 1. The number of the sets of the deletions of the nucleic acid residues may be particularly 1, 2, or 3. In accordance with an aspect, the number of the sets of the deletions of the nucleic acid residues may be still more particularly 1. In accordance with an aspect, the number of the sets of the deletions of the nucleic acid residues may be still more particularly 2. For example, the proportion of the number of the sets of the deletions of the nucleic acid residues to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the sets of the deletions of the nucleic acid residues to the length of the first base sequence (the number of residues) may be, for example, 5 to 10%, 10 to 15%, 15 to 20%, 20 to 25%, or 25 to 30%. Specifically, the proportion of the number of the sets of the deletions of the nucleic acid residues to the length of the first base sequence (the number of residues) may be, for example, 5 to 30%, 5 to 20%, or 5 to 15%.

For example, the number of deletions of nucleic acid residues included in a set of deletions of nucleic acid residues may be 1 or more, 2 or more, 3 or more, 4 or more, 5 or more, 6 or more, 7 or more, 8 or more, 9 or more, 10 or more, 12 or more, 15 or more, 20 or more, or 25 or more, may be 30 or less, 25 or less, 20 or less, 15 or less, 12 or less, 10 or less, 9 or less, 8 or less, 7 or less, 6 or less, 5 or less, 4 or less, 3 or less, or 2 or less, or may be an uncontradictory combination thereof. Specifically, the number of deletions of nucleic acid residues included in a set of deletions of nucleic acid residues may be, for example, 1 to 2, 2 to 3, 3 to 4, 4 to 5, 5 to 6, 6 to 7, 7 to 8, 8 to 9, 9 to 10, 10 to 12, 12 to 15, 15 to 20, 20 to 25, or 25 to 30. Specifically, the number of deletions of nucleic acid residues included in a set of deletions of nucleic acid residues may be, for example, 1 to 30, 1 to 20, 1 to 10, 1 to 8, 1 to 7, 1 to 6, 1 to 5, 1 to 4, 1 to 3, 1 to 2, or 1. The number of deletions of nucleic acid residues included in a set of deletions of nucleic acid residues may be particularly 1, 2, 3, 4, 5, 6, 7, or 8. The number of deletions of nucleic acid residues included in a set of deletions of nucleic acid residues may be still more particularly 1, 2, or 3. In a case in which the number of sets of deletions of nucleic acid residues is 2 or more, the number of deletions of nucleic acid residues included in a set of deletions of nucleic acid residues may be independently selected for each set of deletions of nucleic acid residues, unless otherwise specified.

In a case in which the number of deletions of nucleic acid residues is 2 or more, the introduction positions of the deletions of the nucleic acid residues may be independently selected for a deletion of each nucleic acid residue, unless otherwise specified. The introduction positions of the deletions of the nucleic acid residues may be, for example, the introduction positions of the destabilizing sites described as examples above. The introduction positions of the deletions of the nucleic acid residues may be particularly positions -3 to -6 or positions +3 to +6.

For example, the number of insertions, deletions, or substitutions of linkers (that is, the number of inserted, deleted, or substituted linkers) may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the insertions, deletions, or substitutions of the linkers may be, for example, 1 to 2, 2 to 3, 3 to 4, 4 to 5, 5 to 6, 6 to 7, or 7 to 8. Specifically, the number of the insertions, deletions, or substitutions of the linkers may be, for example, 1 to 8, 1 to 7, 1 to 6, 1 to 5, 1 to 4, 1 to 3, 1 to 2, or 1. The number of the insertions, deletions, or substitutions of the linkers may be particularly 1, 2, or 3. The number of the insertions, deletions, or substitutions of the linkers may be still more particularly 1. For example, the proportion of the number of the insertions, deletions, or substitutions of the linkers to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the insertions, deletions, or substitutions of the linkers to the length of the first base sequence (the number of residues) may be, for example, 5 to 10%, 10 to 15%, 15 to 20%, 20 to 25%, or 25 to 30%. Specifically, the proportion of the number of the insertions, deletions, or substitutions of the linkers to the length of the first base sequence (the number of residues) may be, for example, 5 to 30%, 5 to 20%, or 5 to 15%.

For example, the number of sets of insertions, deletions, or substitutions of linkers may be 1 or more, 2 or more, 3 or more, 4 or more, 5 or more, 6 or more, or 7 or more, may be 8 or less, 7 or less, 6 or less, 5 or less, 4 or less, 3 or less, or 2 or less, or may be an uncontradictory combination thereof. Specifically, the number of the sets of the insertions, deletions, or substitutions of the linkers may be, for example, 1 to 2, 2 to 3, 3 to 4, 4 to 5, 5 to 6, 6 to 7, or 7 to 8. Specifically, the number of the sets of the insertions, deletions, or substitutions of the linkers may be, for example, 1 to 8, 1 to 7, 1 to 6, 1 to 5, 1 to 4, 1 to 3, 1 to 2, or 1. The number of the sets of the insertions, deletions, or substitutions of the linkers may be particularly 1, 2, or 3. The number of the sets of the insertions, deletions, or substitutions of the linkers may be still more particularly 1. For example, the proportion of the number of the sets of the insertions, deletions, or substitutions of the linkers to the length of a first base sequence (the number of residues) may be 5% or more, 10% or more, 15% or more, 20% or more, or 25% or more, may be 30% or less, 25% or less, 20% or less, 15% or less, or 10% or less, or may be an uncontradictory combination thereof. Specifically, the proportion of the number of the sets of the insertions, deletions, or substitutions of the linkers to the length of the first base sequence (the number of residues) may be, for example, 5 to 10%, 10 to 15%, 15 to 20%, 20 to 25%, or 25 to 30%. Specifically, the proportion of the number of the sets of the insertions, deletions, or substitutions of the linkers to the length of the first base sequence (the number of residues) may be, for example, 5 to 30%, 5 to 20%, or 5 to 15%.

For example, the number of insertions, deletions, or substitutions of linkers included in a set of insertions, deletions, or substitutions of linkers may be 1 or more, 2 or more, 3 or more, or 4 or more, may be 5 or less, 4 or less, 3 or less, or 2 or less, or may be an uncontradictory combination thereof. Specifically, the number of insertions, deletions, or substitutions of linkers included in a set of insertions, deletions, or substitutions of linkers may be, for example, 1 to 2, 2 to 3, 3 to 4, or 4 to 5. Specifically, the number of insertions, deletions, or substitutions of linkers included in a set of insertions, deletions, or substitutions of linkers may be, for example, 1 to 5, 1 to 4, 1 to 3, 1 to 2, or 1. The number of insertions, deletions, or substitutions of linkers included in a set of insertions, deletions, or substitutions of linkers may be particularly 1 or 2. The number of insertions, deletions, or substitutions of linkers included in a set of insertions, deletions, or substitutions of linkers may be still more particularly 1. In a case in which the number of sets of insertions, deletions, or substitutions of linkers is 2 or more, the number of insertions, deletions, or substitutions of linkers included in a set of insertions, deletions, or substitutions of linkers may be independently selected for each set of insertions, deletions, or substitutions of linkers, unless otherwise specified.

In a case in which the number of insertions, deletions, or substitutions of linkers is 2 or more, the introduction positions of the insertions, deletions, or substitutions of the linkers may be independently selected for an insertion, deletion, or substitution of each linker, unless otherwise specified. The introduction positions of the insertions, deletions, or substitutions of the linkers may be, for example, the introduction positions of the destabilizing sites described as examples above.

Such a destabilizing site may comprise, for example, an insertion of a nucleic acid residue without a deletion of a linker and/or a deletion of a nucleic acid residue without an insertion of a linker.

The destabilizing site may comprise, for example, insertions of two or more consecutive nucleic acid residues (that is, a set of insertions of nucleic acid residues, consisting of insertions of two or more nucleic acid residues) and/or deletions of two or more consecutive nucleic acid residues (that is, a set of deletions of nucleic acid residues, consisting of deletions of two or more nucleic acid residues).

The destabilizing site may comprise, for example, the following (A):
(A) a combination of an abasic site and a deletion and/or insertion of a nucleic acid residue.

The abasic sites in the destabilizing site (A) described above may be one abasic site, or may be two or more abasic sites. For example, the abasic sites in the destabilizing site (A) described above may be one of abasic sites at positions -1 to +1, or may be a combination of one of the abasic sites at positions -1 to +1 and one or more abasic sites at positions other than the positions -1 to +1. Such an abasic site in the destabilizing site (A) described above may be particularly one of the abasic sites at the positions -1 to +1.

The destabilizing site may comprise, for example, the following (B):
(B) a combination of a mismatch site and a deletion and/or insertion of a nucleic acid residue.

The mismatch sites in the destabilizing site (B) described above may be one mismatch site, or may be two or more mismatch sites. For example, the mismatch sites in the destabilizing site (B) described above may be one of mismatch sites at positions -1 to +1, or may be a combination of one of the mismatch sites at the positions -1 to +1 and one or more mismatch sites at positions other than the positions -1 to +1. Such a mismatch site in the destabilizing site (B) described above may be particularly one of the mismatch sites at the positions -1 to +1.

Such a deletion of a nucleic acid residue in the destabilizing site (A) or (B) described above may be, for example, one or more deletions of nucleic acid residues at positions other than positions -1 to +1. The deletion of the nucleic acid residue in the destabilizing site (A) or (B) described above may be, for example, one or more sets of deletions of nucleic acid residues at positions other than the positions -1 to +1. Specifically, the deletion of the nucleic acid residue in the destabilizing site (A) or (B) described above may be, for example, one or two sets of deletions of nucleic acid residues at positions other than the positions -1 to +1.

Such an insertion of a nucleic acid residue in the destabilizing site (A) or (B) described above may be, for example, one or more insertions of nucleic acid residues at positions other than positions -1 to +1. The insertion of the nucleic acid residue in the destabilizing site (A) or (B) described above may be, for example, one or more sets of insertions of nucleic acid residues at the positions other than the positions -1 to +1. Specifically, the insertion of the nucleic acid residue in the destabilizing site (A) or (B) described above may be, for example, one or two sets of insertions of nucleic acid residues at positions other than the positions -1 to +1. Such insertions of the nucleic acid residues in the destabilizing site (A) or (B) described above may be particularly two sets of insertions of nucleic acid residues at positions other than the positions -1 to +1. The insertions of the nucleic acid residues in the destabilizing site (A) or (B) described above may be still more particularly a combination of a set of insertions of nucleic acid residues at negative positions other than position -1 and a set of insertions of nucleic acid residues at positive positions other than position +1.

The destabilizing site may comprise, for example, the following (a), (b), or (c):
(a) a combination of an abasic site at positions -1 to +1 and a deletion and/or insertion of a nucleic acid residue at a position other than the positions -1 to +1;
(b) a combination of a mismatch site at positions -1 to +1 and a deletion and/or insertion of a nucleic acid residue at a position other than the positions -1 to +1; and
(c) a mismatch site at positions -1 to +1.

The destabilizing site (a) described above may be an example of the destabilizing site (A). The destabilizing site (b) described above may be an example of the destabilizing site (B).

The destabilizing site may particularly comprise the destabilizing site (A) described above such as the destabilizing site (a) described above or the destabilizing sites (B) described above such as the destabilizing site (b) described above. The destabilizing site may still more particularly comprise the destabilizing site (A) described above such as the destabilizing site (a) described above.

The destabilizing site (a) described above may be, for example, a combination of an abasic site at positions -1 to +1 and deletions of one or more nucleic acid residues and/or insertions of one or more nucleic acid residues at positions other than the positions -1 to +1. The destabilizing site (a) described above may be, for example, a combination of an abasic site at positions -1 to +1 and deletions of one or more sets of nucleic acid residues and/or insertions of one or more sets of nucleic acid residues at positions other than the positions -1 to +1. Specifically, the destabilizing site (a) described above may be, for example, a combination of an abasic site at positions -1 to +1 and deletions of one or two sets of nucleic acid residues and/or insertions of one or two sets of nucleic acid residues at positions other than the positions -1 to +1. The destabilizing site (a) described above may be particularly a combination of an abasic site at positions -1 to +1 and insertions of one or two sets of nucleic acid residues at positions other than the positions -1 to +1. The destabilizing site (a) described above may be still more particularly a combination of an abasic site at positions -1 to +1 and insertions of two sets of nucleic acid residues at positions other than the positions -1 to +1. The destabilizing site (a) described above may be still more particularly a combination of an abasic site at positions -1 to +1, an insertion of a set of nucleic acid residues at negative positions other than position -1, and an insertion of a set of nucleic acid residues at positive positions other than position +1.

The destabilizing site (b) described above may be, for example, a combination of a mismatch site at positions -1 to +1 and deletions of one or more nucleic acid residues and/or insertions of one or more nucleic acid residues at positions other than the positions -1 to +1. The destabilizing site (b) described above may be, for example, a combination of a mismatch site at positions -1 to +1 and deletions of one or more sets of nucleic acid residues and/or insertions of one or more sets of nucleic acid residues at positions other than the positions -1 to +1. Specifically, the destabilizing site (b) described above may be, for example, a combination of a mismatch site at positions -1 to +1 and deletions of one or two sets of nucleic acid residues and/or insertions of one or two sets of nucleic acid residues at positions other than the positions -1 to +1. The destabilizing site (b) described above may be particularly a combination of a mismatch site at positions -1 to +1 and insertions of one or two sets of nucleic acid residues at positions other than the positions -1 to +1. The destabilizing site (b) described above may be still more particularly a combination of a mismatch site at positions -1 to +1 and insertions of two sets of nucleic acid residues at positions other than the positions -1 to +1. The destabilizing site (b) described above may be still more particularly a combination of a mismatch site at positions -1 to +1, an insertion of a set of nucleic acid residues at negative positions other than position -1, and an insertion of a set of nucleic acid residues at positive positions other than position +1.

Examples of positions other than positions -1 to +1 include positions other than positions -1 to +1, selected from the introduction positions of the m destabilizing sites described as examples above. Particular examples of the positions other than the positions -1 to +1 include positions -5 to -7, positions +5 to +7, positions -3 to -6, and positions +3 to +6. Examples of negative positions other than position -1 include negative positions other than position -1, selected from the introduction positions of the destabilizing sites described as examples above. Particular examples of the negative positions other than the position -1 include positions -5 to -7 and positions -3 to -6. Still more particular examples of the negative positions other than the position -1 include positions -3 to -6. Examples of positive positions other than position +1 include positive positions other than position +1, selected from the introduction positions of the destabilizing sites described as examples above. Particular examples of the positive positions other than the position +1 include positions +5 to +7 and positions +3 to +6. Still more particular examples of the positive positions other than the position +1 include positions +3 to +6.

In accordance with an aspect, a case in which the destabilizing site consists of an abasic site, a substitution of a linker with a nucleic acid residue, or a substitution of a nucleic acid residue with a linker may be excluded from the present invention. In accordance with an aspect, a case in which the destabilizing site consists of an abasic site at position -1, a substitution of a linker between position -1 and position +1 with a nucleic acid residue, or a substitution of a nucleic acid residue at position -1 with a linker may be excluded from the present invention. In accordance with an aspect, a case in which the number (N) of substrate oligonucleotides is two, and the destabilizing site consists of an abasic site at -1, a substitution of a linker between position -1 and position +1 with a nucleic acid residue, or a substitution of a nucleic acid residue at position -1 with a linker may be excluded from the present invention. In accordance with an aspect, a case in which the number (N) of substrate oligonucleotides is two, the number (M) of a complementary oligonucleotide is one, and the destabilizing site consists of an abasic site at -1, a substitution of a linker between position -1 and position +1 with a nucleic acid residue, or a substitution of a nucleic acid residue at position -1 with a linker may be excluded from the present invention.

In accordance with an aspect, a case in which the destabilizing site consists of an abasic site, two abasic sites, or a combination of an abasic site and a mismatch site may be excluded from the present invention. In accordance with an aspect, a case in which the destabilizing site consists of an abasic site at position -1, a combination of an abasic site at position -1 and an abasic site at a position (for example, position +4) other than the position -1, or a combination of an abasic site at position -1 and a mismatch site at a position (for example, position +4) other than position -1 may be excluded from the present invention. In accordance with an aspect, a case in which the number (N) of substrate oligonucleotides is two, and the destabilizing site consists of an abasic site at position -1, a combination of an abasic site at position -1 and an abasic site at a position (for example, position +4) other than the position -1, or a combination of an abasic site at position -1 and a mismatch site at a position (for example, position +4) other than the position -1 may be excluded from the present invention. In accordance with an aspect, a case in which the number (N) of substrate oligonucleotides is two, the number (M) of a complementary oligonucleotide is one, and the destabilizing site consists of an abasic site at position -1, a combination of an abasic site at position -1 and an abasic site at a position (for example, position +4) other than the position -1, or a combination of an abasic site at position -1 and a mismatch site at a position (for example, position +4) other than the position -1 may be excluded from the present invention.

The complementary oligonucleotide may consist of, or need not consist of a destabilizing base sequence. In other words, the complementary oligonucleotide may further comprise base sequences (hereinafter also referred to as "additional sequences") on the 5' side and/or 3' side of the destabilizing base sequence. The complementary oligonucleotide may particularly consist of a destabilizing base sequence.

The length of the additional sequence is not particularly limited unless the objective of the present invention is impaired.

For example, each of the lengths of the additional sequences on the 5' side and the 3' side may be not less than 0 residues, not less than 1 residue, not less than 2 residues, not less than 3 residues, not less than 4 residues, not less than 5 residues, not less than 7 residues, not less than 10 residues, not less than 15 residues, or not less than 20 residues, may be not more than 25 residues, not more than 20 residues, not more than 15 residues, not more than 10 residues, not more than 7 residues, not more than 5 residues, not more than 4 residues, not more than 3 residues, not more than 2 residues, or not more than 1 residue, or may be an uncontradictory combination thereof. Specifically, each of the lengths of the additional sequences on the 5' side and the 3' side may be, for example, 0 to 1 residue, 1 to 2 residues, 2 to 3 residues, 3 to 4 residues, 4 to 5 residues, 5 to 7 residues, 7 to 10 residues, 10 to 15 residues, 15 to 20 residues, or 20 to 25 residues. Specifically, each of the lengths of the additional sequences on the 5' side and the 3' side may be, for example, 0 to 25 residues, 0 to 15 residues, or 0 to 10 residues.

For example, each of the lengths of the additional sequences on the 5' side and the 3' side may be 0% or more, 5% or more, 10% or more, 20% or more, 30% or more, 40% or more, 50% or more, 60% or more, 70% or more, 80% or more, or 90% or more, may be 100% or less, 90% or less, 80% or less, 70% or less, 60% or less, 50% or less, 40% or less, 30% or less, 20% or less, 10% or less, or 5% or less, or may be an uncontradictory combination thereof, as a proportion in a case in which the total of the lengths of substrate oligonucleotides on a 5' side and a 3' side is taken as 100%. Each of the lengths of the additional sequences on the 5' side and the 3' side may be, for example, 0 to 5%, 5 to 10%, 10 to 20%, 20 to 30%, 30 to 40%, 40 to 50%, 50 to 60%, 60 to 70%, 70 to 80%, 80 to 90%, or 90 to 100% as a proportion in a case in which the total of the lengths of the substrate oligonucleotides on the 5' side and the 3' side is taken as 100%. Specifically, each of the lengths of the additional sequences on the 5' side and the 3' side may be, for example, 0 to 100%, 0 to 50%, or 0 to 20% as a proportion in a case in which the total of the lengths of the substrate oligonucleotides on the 5' side and the 3' side is taken as 100%.

The length of the complementary oligonucleotide may be, for example, not less than 10 residues, not less than 11 residues, not less than 12 residues, not less than 13 residues, not less than 14 residues, not less than 15 residues, not less than 16 residues, not less than 17 residues, not less than 18 residues, not less than 19 residues, not less than 20 residues, not less than 21 residues, not less than 22 residues, not less than 23 residues, not less than 24 residues, not less than 25 residues, not less than 30 residues, not less than 35 residues, not less than 40 residues, not less than 45 residues, not less than 50 residues, not less than 60 residues, not less than 70 residues, not less than 80 residues, not less than 90 residues, not less than 100 residues, not less than 110 residues, not less than 120 residues, not less than 130 residues, not less than 140 residues, not less than 150 residues, not less than 160 residues, not less than 170 residues, not less than 180 residues, or not less than 190 residues, may be not more than 200 residues, not more than 190 residues, not more than 180 residues, not more than 170 residues, not more than 160 residues, not more than 150 residues, not more than 140 residues, not more than 130 residues, not more than 120 residues, not more than 110 residues, not more than 100 residues, not more than 90 residues, not more than 80 residues, not more than 70 residues, not more than 60 residues, not more than 50 residues, not more than 45 residues, not more than 40 residues, not more than 35 residues, not more than 30 residues, not more than 25 residues, not more than 24 residues, not more than 23 residues, not more than 22 residues, not more than 21 residues, not more than 20 residues, not more than 19 residues, not more than 18 residues, not more than 17 residues, not more than 16 residues, not more than 15 residues, not more than 14 residues, not more than 13 residues, not more than 12 residues, or not more than 11 residues, or may be an uncontradictory combination thereof. Specifically, the length of the complementary oligonucleotide may be, for example, 10 to 11 residues, 11 to 12 residues, 12 to 13 residues, 13 to 14 residues, 14 to 15 residues, 15 to 16 residues, 16 to 17 residues, 17 to 18 residues, 18 to 19 residues, 19 to 20 residues, 20 to 21 residues, 21 to 22 residues, 22 to 23 residues, 23 to 24 residues, 24 to 25 residues, 25 to 30 residues, 30 to 35 residues, 35 to 40 residues, 40 to 45 residues, 45 to 50 residues, 50 to 60 residues, 60 to 70 residues, 70 to 80 residues, 80 to 90 residues, 90 to 100 residues, 100 to 110 residues, 110 to 120 residues, 120 to 130 residues, 130 to 140 residues, 140 to 150 residues, 150 to 160 residues, 160 to 170 residues, 170 to 180 residues, 180 to 190 residues, or 190 to 200 residues. Specifically, the length of the complementary oligonucleotide may be, for example, 10 to 200 residues, 10 to 100 residues, 15 to 80 residues, or 20 to 60 residues.

For example, the length of the complementary oligonucleotide may be 5% or more, 10% or more, 20% or more, 30% or more, 40% or more, 50% or more, 60% or more, 70% or more, 80% or more, 90% or more, 100% or more, 110% or more, 120% or more, 130% or more, 140% or more, 150% or more, 170% or more, 200% or more, 250% or more, 300% or more, or 350% or more, may be 400% or less, 350% or less, 300% or less, 250% or less, 200% or less, 170% or less, 150% or less, 140% or less, 130% or less, 120% or less, 110% or less, 100% or less, 90% or less, 80% or less, 70% or less, 60% or less, 50% or less, 40% or less, 30% or less, 20% or less, or 10% or less, or may be an uncontradictory combination, as a proportion in a case in which the total of the lengths of the substrate oligonucleotides on the 5' side and the 3' side is taken as 100%. Specifically, the length of the complementary oligonucleotide may be, for example, 5 to 10%, 10 to 20%, 20 to 30%, 30 to 40%, 40 to 50%, 50 to 60%, 60 to 70%, 70 to 80%, 80 to 90%, 90 to 100%, 100 to 110%, 110 to 120%, 120 to 130%, 130 to 140%, 140 to 150%, 150 to 170%, 170 to 200%, 200 to 250%, 250 to 300%, 300 to 350%, or 350 to 400% as a proportion in a case in which the total of the lengths of the substrate oligonucleotides on the 5' side and the 3' side is taken as 100%. Specifically, the length of the complementary oligonucleotide may be, for example, 5 to 400%, 5 to 300%, 5 to 200%, 5 to 150%, 5 to 100%, 5 to 50%, 5 to 20%, 20 to 150%, 20 to 100%, 20 to 50%, 50 to 150%, or 50 to 100% as a proportion in a case in which the total of the lengths of the substrate oligonucleotides on the 5' side and the 3' side is taken as 100%.

For example, the length of the complementary oligonucleotide may be 5% or more, 10% or more, 20% or more, 30% or more, 40% or more, 50% or more, 60% or more, 70% or more, 80% or more, 90% or more, 100% or more, 110% or more, 120% or more, 130% or more, 140% or more, 150% or more, 170% or more, 200% or more, 250% or more, 300% or more, or 350% or more, may be 400% or less, 350% or less, 300% or less, 250% or less, 200% or less, 170% or less, 150% or less, 140% or less, 130% or less, 120% or less, 110% or less, 100% or less, 90% or less, 80% or less, 70% or less, 60% or less, 50% or less, 40% or less, 30% or less, 20% or less, or 10% or less, or may be an uncontradictory combination, as a proportion in a case in which the length of the oligonucleotide of interest is taken as 100%. Specifically, the length of the complementary oligonucleotide may be, for example, 5 to 10%, 10 to 20%, 20 to 30%, 30 to 40%, 40 to 50%, 50 to 60%, 60 to 70%, 70 to 80%, 80 to 90%, 90 to 100%, 100 to 110%, 110 to 120%, 120 to 130%, 130 to 140%, 140 to 150%, 150 to 170%, 170 to 200%, 200 to 250%, 250 to 300%, 300 to 350%, or 350 to 400% as a proportion in a case in which the length of the oligonucleotide of interest is taken as 100%. Specifically, the length of the complementary oligonucleotide may be, for example, 5 to 400%, 5 to 300%, 5 to 200%, 5 to 150%, 5 to 100%, 5 to 50%, 5 to 20%, 20 to 150%, 20 to 100%, 20 to 50%, 50 to 150%, or 50 to 100% as a proportion in a case in which the length of the oligonucleotide of interest is taken as 100%.

The phrases "complementary oligonucleotide comprises modified nucleic acid residue", "complementary oligonucleotide is modified", and "complementary oligonucleotide has modification" may be used interchangeably with each other.

Each complementary oligonucleotide may comprise or need not comprise modified nucleic acid residues. In other words, each complementary oligonucleotide may be modified or need not be modified. The modification of the nucleic acid residues is as described above. Each complementary oligonucleotide may have one kind of a modification or may have two or more kinds of modifications in combination. Each complementary oligonucleotide may comprise or need not comprise, for example, each modification described as an example above. Each nucleic acid residue included in each complementary oligonucleotide may have one kind of a modification or may have two or more kinds of modifications in combination. Only some of nucleic acid residues included in each complementary oligonucleotide may be modified nucleic acid residues, or all of the nucleic acid residues included in each complementary oligonucleotide may be modified nucleic acid residues. For example, in a case in which a complementary oligonucleotide comprises a deletion of a nucleic acid residue as a destabilizing site, the linker may be inserted into the position of the deletion of the nucleic acid residue.

The proportion of the modified nucleic acid residues in the complementary oligonucleotide (that is, the proportion of the number of the modified nucleic acid residues to the number of the nucleic acid residues included in the complementary oligonucleotide) may be, for example, 0% or more, 5% or more, 10% or more, 20% or more, 30% or more, 40% or more, 50% or more, 60% or more, 70% or more, 80% or more, 90% or more, or 95% or more, may be 100% or less, 95% or less, 90% or less, 80% or less, 70% or less, 60% or less, 50% or less, 40% or less, 30% or less, 20% or less, 10% or less, or 5% or less, or may be an uncontradictory combination thereof. Specifically, the proportion of the modified nucleic acid residues in the complementary oligonucleotide may be, for example, 0 to 5%, 5 to 10%, 10 to 20%, 20 to 30%, 30 to 40%, 40 to 50%, 50 to 60%, 60 to 70%, 70 to 80%, 80 to 90%, 90 to 95%, or 95 to 100%. The proportion, described as an example above, of the modified nucleic acid residues in the complementary oligonucleotide can also be independently applied to nucleic acid residues having an optionally selected modification (for example, any modification described as an example above). In other words, for example, the proportion of nucleic acid residues having a modification in a phosphate portion in the complementary oligonucleotide (that is, the proportion of the number of nucleic acid residues having a modification in a phosphate portion to the number of the nucleic acid residues included in the complementary oligonucleotide) may be set at the proportion, described as an example above, of the modified nucleic acid residues in the complementary oligonucleotide. For example, the proportion of nucleic acid residues having a modification of a sugar portion in the complementary oligonucleotide (that is, the proportion of the number of the nucleic acid residues having the modification of the sugar portion to the number of nucleic acid residues included in the complementary oligonucleotide) may be set at the proportion, described as an example above, of the modified nucleic acid residues in the complementary oligonucleotide. For example, the proportion of nucleic acid residues having a modification of a base portion in the complementary oligonucleotide (that is, the proportion of the number of nucleic acid residues having a modification of a base portion to the number of the nucleic acid residues included in the complementary oligonucleotide) may be set at the proportion, described as an example above, of the modified nucleic acid residues in the complementary oligonucleotide.

The number of the modified nucleic acid residues in the complementary oligonucleotide may be, for example, 0 or more, 5 or more, 10 or more, 15 or more, 20 or more, 25 or more, 30 or more, 40 or more, 50 or more, 60 or more, 80 or more, 100 or more, 120 or more, 140 or more, 160 or more, or 180 or more, may be 200 or less, 180 or less, 160 or less, 140 or less, 120 or less, 100 or less, 80 or less, 60 or less, 50 or less, 40 or less, 30 or less, 25 or less, 20 or less, 15 or less, 10 or less, or 5 or less, or may be an uncontradictory combination thereof. Specifically, the number of the modified nucleic acid residues in the complementary oligonucleotide may be, for example, 0 to 5, 5 to 10, 10 to 15, 15 to 20, 20 to 25, 25 to 30, 30 to 40, 40 to 50, 50 to 60, 60 to 80, 80 to 100, 100 to 120, 120 to 140, 140 to 160, 160 to 180, or 180 to 200. The number, described as an example above, of the modified nucleic acid residues in the complementary oligonucleotide can also be independently applied to nucleic acid residues having an optionally selected modification (for example, any modification described as an example above). In other words, for example, the number of nucleic acid residues having a modification of a phosphate portion in the complementary oligonucleotide may be set at the number, described as an example above, of the modified nucleic acid residues in the complementary oligonucleotide. For example, the number of nucleic acid residues having a modification of a sugar portion in the complementary oligonucleotide may be set at the number, described as an example above, of the modified nucleic acid residues in the complementary oligonucleotide. For example, the number of nucleic acid residues having a modification of a base portion in the complementary oligonucleotide may be set at the number, described as an example above, of the modified nucleic acid residues in the complementary oligonucleotide.

In the ligation step, complementary oligonucleotides whose number is M, the M being an integer 1 or more, are used. The number (M) of the complementary oligonucleotides is 1 or more. "Number of complementary oligonucleotides" means the number of the complementary oligonucleotides used in the ligation step. The number (M) of the complementary oligonucleotides may be 1, or may be 2 or more.

In a case in which the number (N - 1) of ligation sites is 2 or more, the complementary oligonucleotides can be independently selected for each of the ligation sites. In other words, in a case in which the number (N - 1) of ligation sites is 2 or more, destabilizing sites can be independently selected for each of the ligation sites (that is, for each of complementary oligonucleotides corresponding to the ligation sites). The destabilizing sites may be identical, or need not be identical for the ligation sites (that is, for the complementary oligonucleotides corresponding to the ligation sites). For example, the destabilizing sites may be independently selected from the aspects of the destabilizing sites, described as examples above, for each of the ligation sites (that is, for each of the complementary oligonucleotides corresponding to the ligation sites). Specifically, the destabilizing site may independently comprise, for example, the destabilizing site (A) described above (that is, a combination of an abasic site and a deletion and/or insertion of a nucleic acid residue) such as the destabilizing site (a) described above (that is, a combination of an abasic site at positions -1 to +1 and a deletion and/or insertion of a nucleic acid residue at a position other than the positions -1 to +1), the destabilizing site (B) described above (that is, a combination of a mismatch site and a deletion and/or insertion of a nucleic acid residue) such as the destabilizing site (b) described above (that is, a combination of a mismatch site at positions -1 to +1 position and a deletion and/or insertion of a nucleic acid residue at positions other than the positions -1 to +1), or the destabilizing site (c) described above (that is, a mismatch site at positions -1 to +1) for each of the ligation sites (that is, for each of the complementary oligonucleotides corresponding to the ligation sites). For example, any aspect of the destabilizing sites described as examples above may be selected as the destabilizing site of a complementary oligonucleotide corresponding to any ligation site, and another aspect of the destabilizing sites described as examples above may be selected as the destabilizing site of a complementary oligonucleotide corresponding to another ligation site. Specifically, for example, the destabilizing site of the complementary oligonucleotide corresponding to any ligation site may comprise the destabilizing site (a) or (b) described above, and the destabilizing site of the complementary oligonucleotide corresponding to the other ligation site may comprise the destabilizing site (c) described above. In particular, the destabilizing site of the complementary oligonucleotide corresponding to any ligation site may comprise the destabilizing site (a) described above, and the destabilizing site of the complementary oligonucleotide corresponding to the other ligation site may comprise the destabilizing site (c) described above.

In each ligation site, one complementary oligonucleotide may be used, or two or more complementary oligonucleotides may be used. In other words, ligation between the substrate oligonucleotides on the 5' and 3' sides of each of the ligation sites is performed in the presence of one or more complementary oligonucleotides corresponding to the ligation sites. A complementary oligonucleotide may also correspond to two or more ligation sites. In other words, a complementary oligonucleotide may be used in common for ligation between substrate oligonucleotides on the 5' and 3' sides of two or more ligation sites. In a case in which a complementary oligonucleotide corresponds to two or more ligation sites, the complementary oligonucleotide may comprise a destabilizing base sequence corresponding to each of the ligation sites. For example, in a case in which three substrate oligonucleotides A, B, and C are ligated in 5' to 3' direction in the presence of a complementary oligonucleotide, the complementary oligonucleotide may comprise a destabilizing base sequence for ligating B and C and a destabilizing base sequence for ligating A and B in 5' to 3' direction. In such a case, a third base sequence for the destabilizing base sequence for ligating B and C and a second base sequence for the destabilizing base sequence for ligating A and B may be, or need not be partly or completely duplicated.

The number (M) of complementary oligonucleotides may be less than, equal to, or more than the number (N - 1) of ligation sites. For example, in a case in which a complementary oligonucleotide is independently used in each ligation site, the number (M) of the complementary oligonucleotides may be equal to the number (N - 1) of the ligation sites. For example, in a case in which a complementary oligonucleotide corresponds to two or more ligation sites, the number (M) of the complementary oligonucleotides may be less than the number (N - 1) of the ligation sites. In a case in which the number (M) of the complementary oligonucleotides is two or more, the destabilizing sites can be independently selected for each of the complementary oligonucleotides. The destabilizing sites may be identical, or need not be identical for the complementary oligonucleotides. For example, the destabilizing sites may be independently selected from the aspects of the destabilizing sites described as examples above for each of the complementary oligonucleotides. Specifically, the destabilizing sites may independently comprise, for example, the destabilizing site (a), (b), or (c) described above for each of the complementary oligonucleotides. For example, any aspect of the destabilizing sites described as examples above may be selected as the destabilizing site of any complementary oligonucleotide, and another aspect of the destabilizing sites described as examples above may be selected as the destabilizing site of another complementary oligonucleotide. Specifically, for example, the destabilizing site of any complementary oligonucleotide may comprise the destabilizing site (a) or (b) described above, and the destabilizing site of the other complementary oligonucleotide may comprise the destabilizing site (c) described above. In particular, the destabilizing site of any complementary oligonucleotide may comprise the destabilizing site (a) described above, and the destabilizing site of the other complementary oligonucleotide may comprise the destabilizing site (c) described above.

A method of producing a complementary oligonucleotide is not particularly limited. A complementary oligonucleotide can be produced by, for example, in turn ligating nucleic acid residues included in the complementary oligonucleotide. For example, the nucleic acid residues can be ligated by a phosphoroamidite method. A complementary oligonucleotide can be produced by, for example, ligating nucleic acid fragments included in the complementary oligonucleotide. The nucleic acid fragments can be enzymatically ligated by, for example, using a complementary strand. The nucleic acid fragments may be ligated by, for example, the method of the present invention.

### <4> Ligation Step

The ligation step is a step of performing enzymatic ligation of a set of substrate oligonucleotides. The phrase "ligation of set of substrate oligonucleotides" may be used interchangeably with the phrase "ligation of substrate oligonucleotides included in set".

"Enzymatic ligation of set of substrate oligonucleotides" means that a set of substrate oligonucleotides is ligated by a ligation enzyme. In the ligation step, for example, substrate oligonucleotides on a 5' side and a 3' side may hybridize with corresponding complementary oligonucleotides to form a double-stranded structure, and the substrate oligonucleotides on the 5' side and the 3' side may be then ligated by a ligation enzyme. The ligation enzyme is not particularly limited as long as the ligation enzyme can ligate substrate oligonucleotides in the presence of a complementary oligonucleotide. Examples of the ligation enzyme include DNA ligases and RNA ligases. Particular examples of the ligation enzyme include DNA ligases. The origin of the ligation enzyme is not particularly limited. The ligation enzyme may originate from, for example, an eucaryote such as a mammal or yeast, a prokaryote such as *E. coli,* or a virus such as a phage. Examples of the DNA ligases include T3 DNA ligases, T4 DNA ligases, T7 DNA ligases, *E. coli* DNA ligases, and Taq DNA ligases. Examples of the RNA ligases include: T4 RNA ligases such as T4 RNA ligase 2; and RtcB ligases. In accordance with an aspect, a case in which the ligation enzyme is a T4 DNA ligase may be excluded from the present invention. Still more particular examples of the ligation enzyme include T3 DNA ligases. The DNA ligases may catalyze ligation between DNAs or the modified nucleic acids thereof. Thus, such a DNA ligase may be used in, for example, ligation between DNAs or the modified nucleic acids thereof (that is, ligation between a substrate oligonucleotide on a 5' side, of which the 3' end is a DNA or the modified nucleic acid thereof, and a substrate oligonucleotide on a 3' side, of which the 5' end is a DNA or the modified nucleic acid thereof). Certain DNA ligases such as T3 DNA ligases and T4 DNA ligases may catalyze ligation between RNAs or the modified nucleic acids thereof as well as ligation between DNAs or the modified nucleic acids thereof. Thus, certain DNA ligases such as T3 DNA ligases and T4 DNA ligases may be used in, for example, ligation between RNAs or the modified nucleic acids thereof (that is, ligation between a substrate oligonucleotide on a 5' side, of which the 3' end is an RNA or the modified nucleic acid thereof, and a substrate oligonucleotide on a 3' side, of which the 5' end is an RNA or the modified nucleic acid thereof). The RNA ligase may catalyze ligation between RNAs or the modified nucleic acids thereof. Thus, such an RNA ligase may be used in, for example, ligation between RNAs or the modified nucleic acids thereof (that is, ligation between a substrate oligonucleotide on a 5' side, of which the 3' end is an RNA or the modified nucleic acid thereof, and a substrate oligonucleotide on a 3' side, of which the 5' end is an RNA or the modified nucleic acid thereof).

The ligation step can be carried out in a liquid. The liquid in which the ligation step is carried out is also referred to as "reaction liquid". Specifically, the ligation step can be carried out by allowing a ligation enzyme, a substrate oligonucleotide, and a complementary oligonucleotide to coexist in the reaction liquid. For example, the ligation step may be carried out by a batch method, or may be carried out by a column method. In the case of the batch method, for example, the ligation step can be carried out by mixing a ligation enzyme, a substrate oligonucleotide, and a complementary oligonucleotide in the reaction liquid in a container. The ligation step may be carried out while standing, or may be carried out while stirring or shaking. In the case of the column method, for example, the ligation step can be carried out by passing the reaction liquid containing a substrate oligonucleotide and a complementary oligonucleotide through a column filled with an immobilized ligation enzyme. An aqueous medium such as water or a buffer can be used as the reaction liquid. The reaction liquid may contain a component useful or necessary for the ligation step, as well as the ligation enzyme, the substrate oligonucleotide, and the complementary oligonucleotide. Examples of such a component include pH buffers, Mg ions, ATP, and DTT.

Conditions under which the ligation step is carried out (for example, the pH of the reaction liquid, reaction temperature, reaction time, and the amounts and concentrations of various components used) are not particularly limited as long as the oligonucleotide of interest is generated. The conditions under which the ligation step is carried out can be set as appropriate depending on various conditions such as the structure of a substrate oligonucleotide, the structure of a complementary oligonucleotide, and the kind of a ligation enzyme. "Concentration of certain component in reaction liquid" may mean the maximum concentration of the component in the reaction liquid in the ligation step unless otherwise specified. The maximum concentration of a certain component in the reaction liquid in the ligation step may be, for example, the concentration of the component in the reaction liquid at the time of starting the ligation step.

For example, the pH of the reaction liquid may be 5 to 10, 6 to 9, or 7 to 8.

For example, the reaction temperature may be 5°C or more, 10°C or more, 15°C or more, 20°C or more, 25°C or more, 30°C or more, 35°C or more, 40°C or more, 45°C or more, 50°C or more, or 55°C or more, may be 90°C or less, 80°C or less, 70°C or less, 60°C or less, 55°C or less, 50°C or less, 45°C or less, 40°C or less, 35°C or less, 30°C or less, 25°C or less, 20°C or less, 15°C or less, or 10°C or less, or may be an uncontradictory combination thereof. Specifically, the reaction temperature may be, for example, 5 to 10°C, 10 to 15°C, 15 to 20°C, 20 to 25°C, 25 to 30°C, 30 to 35°C, 35 to 40°C, 40 to 45°C, 45 to 50°C, 50 to 55°C, or 55 to 60°C. Specifically, the reaction temperature may be, for example, 5 to 60°C, 10 to 40°C, 15 to 35°C, or 20 to 30°C. The reaction temperature may be particularly ordinary temperature (for example, about 25°C). In the ligation step, heating may be carried out, or need not be carried out. The fluctuation range of the reaction temperature through the ligation step may be, for example, 30°C or less, 25°C or less, 20°C or less, 15°C or less, 10°C or less, or 5°C or less.

For example, the reaction time may be 0.5 hours or more, 1 hour or more, 2 hours or more, 4 hours or more, 6 hours or more, 9 hours or more, 12 hours or more, 15 hours or more, 18 hours or more, 24 hours or more, or 30 hours or more, may be 36 hours or less, 30 hours or less, 24 hours or less, 18 hours or less, 15 hours or less, 12 hours or less, 9 hours or less, 6 hours or less, 4 hours or less, 2 hours or less, or 1 hour or less, or may be an uncontradictory combination thereof. Specifically, the reaction time may be, for example, 0.5 to 1 hour, 1 to 2 hours, 2 to 4 hours, 4 to 6 hours, 6 to 9 hours, 9 to 12 hours, 12 to 15 hours, 15 to 18 hours, 18 to 24 hours, 24 to 30 hours, or 30 to 36 hours. Specifically, the reaction time may be, for example, 1 to 36 hours, 3 to 30 hours, or 6 to 24 hours.

For example, the used amounts of substrate oligonucleotides on 5' and 3' sides may be allowed to be substantially equal to each other by molar ratio to adequately ligate the substrate oligonucleotides. The phrase "used amounts of substrate oligonucleotides on 5' and 3' sides are substantially equal to each other in terms of molar ratio" may mean that the used amount of a substrate oligonucleotide on a 5' side or the used amount of a substrate oligonucleotide on a 3' side, whichever is less, is 90% or more, 95% or more, 97% or more, or 99% or more, by molar ratio, of the other, and also encompasses a case in which the used amounts of the substrate oligonucleotides on the 5' and 3' sides are equal to each other by molar ratio. For example, the molar concentrations of substrate oligonucleotides on 5' and 3' sides in the reaction liquid may be allowed to be substantially equal to each other to adequately ligate the substrate oligonucleotides. The phrase "molar concentrations of substrate oligonucleotides on 5' and 3' sides in reaction liquid are substantially equal to each other" may mean that the molar concentration of a substrate oligonucleotide on a 5' side or the molar concentration of a substrate oligonucleotide on a 3' side, whichever is less, is 90% or more, 95% or more, 97% or more, or 99% or more of the other, and also encompasses a case in which the molar concentrations of the substrate oligonucleotides on the 5' and 3' sides are equal to each other.

For example, the concentration of each substrate oligonucleotide in the reaction liquid may be 1 µM or more, 2 µM or more, 5 µM or more, 10 µM or more, 20 µM or more, 50 µM or more, 100 µM or more, 200 µM or more, 500 µM or more, 1000 µM or more, 2000 µM or more, or 5000 µM or more, may be 10000 µM or less, 5000 µM or less, 2000 µM or less, 1000 µM or less, 500 µM or less, 200 µM or less, 100 µM or less, 50 µM or less, 20 µM or less, 10 µM or less, 5 µM or less, or 2 µM or less, or may be an uncontradictory combination thereof. Specifically, the concentration of each substrate oligonucleotide in the reaction liquid may be, for example, 1 to 2 µM, 2 to 5 µM, 5 to 10 µM, 10 to 20 µM, 20 to 50 µM, 50 to 100 µM, 100 to 200 µM, 200 to 500 µM, 500 to 1000 µM, 1000 to 2000 µM, 2000 to 5000 µM, or 5000 to 10000 µM. Specifically, the concentration of each substrate oligonucleotide in the reaction liquid may be, for example, 1 to 10000 µM, 10 to 10000 µM, 20 to 1000 µM, or 100 to 500 µM.

The used amount of a complementary oligonucleotide may be smaller than, by molar ratio, the used amount of the substrate oligonucleotide on the 5' side or the used amount of the substrate oligonucleotide on the 3' side, whichever is less. For example, the used amount of the complementary oligonucleotide may be, by molar ratio, 0.01% or more, 0.02% or more, 0.05% or more, 0.1% or more, 0.2% or more, 0.5% or more, 1% or more, 2% or more, 5% or more, 10% or more, 15% or more, 20% or more, 30% or more, or 40% or more, may be 50% or less, 40% or less, 30% or less, 20% or less, 15% or less, 10% or less, 5% or less, 2% or less, 1% or less, 0.5% or less, 0.2% or less, 0.1% or less, 0.05% or less, or 0.02% or less, or may be an uncontradictory combination thereof, as a proportion in a case in which the used amount of the substrate oligonucleotide on the 5' side or the used amount of the substrate oligonucleotide on the 3' side, whichever is less, is taken as 100%. Specifically, the used amount of the complementary oligonucleotide may be, for example, by molar ratio, 0.01 to 0.02%, 0.02 to 0.05%, 0.05 to 0.1%, 0.1 to 0.2%, 0.2 to 0.5%, 0.5 to 1%, 1 to 2%, 2 to 5%, 5 to 10%, 10 to 15%, 15 to 20%, 20 to 30%, 30 to 40%, or 40 to 50%, as a proportion in a case in which the used amount of the substrate oligonucleotide on the 5' side or the used amount of the substrate oligonucleotide on the 3' side, whichever is less, is taken as 100%. Specifically, the used amount of the complementary oligonucleotide may be, for example, by molar ratio, 0.01 to 50%, 0.1 to 50%, 1 to 50%, 2 to 30%, or 5 to 15% as a proportion in a case in which the used amount of the substrate oligonucleotide on the 5' side or the used amount of the substrate oligonucleotide on the 3' side, whichever is less, is taken as 100%.

The molar concentration of the complementary oligonucleotide in the reaction liquid may be less than the molar concentration of the substrate oligonucleotide on the 5' side in the reaction liquid or the molar concentration of the substrate oligonucleotide on the 3' side in the reaction liquid, whichever is less. For example, the molar concentration of the complementary oligonucleotide in the reaction liquid may be 0.01% or more, 0.02% or more, 0.05% or more, 0.1% or more, 0.2% or more, 0.5% or more, 1% or more, 2% or more, 5% or more, 10% or more, 15% or more, 20% or more, 30% or more, or 40% or more, may be 50% or less, 40% or less, 30% or less, 20% or less, 15% or less, 10% or less, 5% or less, 2% or less, 1% or less, 0.5% or less, 0.2% or less, 0.1% or less, 0.05% or less, or 0.02% or less, or may be an uncontradictory combination thereof, as a proportion in a case in which the molar concentration of the substrate oligonucleotide on the 5' side in the reaction liquid or the molar concentration of the substrate oligonucleotide on the 3' side in the reaction liquid, whichever is less, is taken as 100%. Specifically, the molar concentration of the complementary oligonucleotide in the reaction liquid may be, for example, 0.01 to 0.02%, 0.02 to 0.05%, 0.05 to 0.1%, 0.1 to 0.2%, 0.2 to 0.5%, 0.5 to 1%, 1 to 2%, 2 to 5%, 5 to 10%, 10 to 15%, 15 to 20%, 20 to 30%, 30 to 40%, or 40 to 50% as a proportion in a case in which the molar concentration of the substrate oligonucleotide on the 5' side in the reaction liquid or the molar concentration of the substrate oligonucleotide on the 3' side in the reaction liquid, whichever is less, is taken as 100%. Specifically, the molar concentration of the complementary oligonucleotide in the reaction liquid may be, for example, 0.01 to 50%, 0.1 to 50%, 1 to 50%, 2 to 30%, or 5 to 15% as a proportion in a case in which the molar concentration of the substrate oligonucleotide on the 5' side in the reaction liquid or the molar concentration of the substrate oligonucleotide on the 3' side in the reaction liquid, whichever is less, is taken as 100%.

For example, the concentration of the complementary oligonucleotide in the reaction liquid may be 0.01 µM or more, 0.02 µM or more, 0.05 µM or more, 0.1 µM or more, 0.2 µM or more, 0.5 µM or more, 1 µM or more, 2 µM or more, 5 µM or more, 10 µM or more, 20 µM or more, 50 µM or more, 100 µM or more, 200 µM or more, or 500 µM or more, may 1000 µM or less, 500 µM or less, 200 µM or less, 100 µM or less, 50 µM or less, 20 µM or less, 10 µM or less, 5 µM or less, 2 µM or less, 1 µM or less, 0.5 µM or less, 0.2 µM or less, 0.1 µM or less, 0.05 µM or less, or 0.02 µM or less, or may be an uncontradictory combination thereof. Specifically, the concentration of the complementary oligonucleotide in the reaction liquid may be, for example, 0.01 to 0.02 µM, 0.02 to 0.05 µM, 0.05 to 0.1 µM, 0.1 to 0.2 µM, 0.2 to 0.5 µM, 0.5 to 1 µM, 1 to 2 µM, 2 to 5 µM, 5 to 10 µM, 10 to 20 µM, 20 to 50 µM, 50 to 100 µM, 100 to 200 µM, 200 to 500 µM, or 500 to 1000 µM. Specifically, the concentration of the complementary oligonucleotide in the reaction liquid may, for example, 0.01 to 1000 µM, 0.1 to 1000 µM, 1 to 1000 µM, 2 to 100 µM, or 10 to 50 µM.

For example, the concentration of the ligation enzyme in the reaction liquid may be 5 U/µL or more, 10 U/µL or more, 20 U/µL or more, 50 U/µL or more, 100 U/µL or more, 200 U/µL or more, or 500 U/µL or more, may be 1000 U/µL or less, 500 U/µL or less, 200 U/µL or less, 100 U/µL or less, 50 U/µL or less, 20 U/µL or less, or 10 U/µL or less, or may be an uncontradictory combination thereof. Specifically, the concentration of the ligation enzyme in the reaction liquid may be, for example, 5 to 10 U/µL, 10 to 20 U/µL, 20 to 50 U/µL, 50 to 100 U/µL, 100 to 200 U/µL, 200 to 500 U/µL, or 500 to 1000 U/µL. Specifically, the concentration of the ligation enzyme in the reaction liquid may be, for example, 5 to 1000 U/µL, 10 to 500 U/µL, or 20 to 200 U/µL.

For example, the activity of the ligation enzyme may be defined as follows.

In other words, for example, the amount of a ligation enzyme by which 50% of 100 ng of HindIII digestion λ DNA is ligated in a reaction buffer having suitable composition (for example, 66 mM Tris-HCl, 10 mM MgCl₂, 1 mM ATP, 1 mM DTT, 7.5% polyethylene glycol (PEG 6000), pH 7.6 (at 25°C), total reaction capacity of 20 µL) for 1 minute at 25°C may be defined as 1 unit [U].

For example, the amount of the ligation enzyme by which 2 × 10⁻⁵ nmol of each substrate oligonucleotide is ligated in the presence of a sufficient amount (for example, molar concentration 10 times that of substrate oligonucleotide) of a complementary oligonucleotide in a reaction buffer having suitable composition (for example, 66 mM Tris-HCl, 10 mM MgCl₂, 1 mM ATP, 1 mM DTT, 7.5% polyethylene glycol (PEG 6000), pH 7.6 (at 25°C)) for 1 minute at 25°C may be defined as 1 unit [U].

After the start of the ligation step, the ligation enzyme, the substrate oligonucleotides, the complementary oligonucleotide, and/or additional components may be additionally supplied to the reaction liquid, singly or in optional combination. Each of these components may be supplied once or plural times, or may be continuously supplied. The reaction conditions may be uniform from the start to end of the ligation step, or may vary during the ligation step. The phrase "reaction conditions vary during ligation step" is not limited to a case in which the reaction conditions vary in terms of time, and encompasses a case in which the reaction conditions vary spatially. Examples of the case in which the reaction conditions vary spatially include a case in which reaction conditions such as reaction temperature and the concentration of the ligation enzyme vary depending on a position on a flow path when the ligation step is carried out by a column method.

In the case of ligating three or more substrate oligonucleotides (that is, the number of ligation sites is two or more), the order and timing of the ligation of the substrate oligonucleotides are not particularly limited. In the case of ligating the three or more substrate oligonucleotides, for example, the substrate oligonucleotides may be ligated simultaneously or in turn. The phrase "three or more substrate oligonucleotides are ligated simultaneously" may mean that ligation of all the ligation sites of the substrate oligonucleotides is simultaneously started. The phrase "three or more substrate oligonucleotides are ligated in turn" may mean that ligation of some ligation sites of the substrate oligonucleotides is started, and ligation of the remaining ligation sites is started during proceeding or after completion of the ligation of said some ligation sites. In other words, in the case of ligating the three or more substrate oligonucleotides, for example, the ligation of the substrate oligonucleotides may be started simultaneously or in turn. For example, in the case of ligating three substrate oligonucleotides A, B, and C in 5' to 3' direction, it is acceptable that A, B, and C are simultaneously ligated, it is acceptable that A and B are ligated in advance, and C and a product obtained by ligating A and B are then ligated, or it is acceptable that B and C are ligated in advance, and A and a product obtained by ligating B and C are then ligated. Specifically, for example, in the case of ligating the three substrate oligonucleotides A, B, and C in the 5' to 3' direction, it is acceptable that A, B, and C are simultaneously ligated, it is acceptable that ligation of A and B is started in advance, and ligation of B and C (also including ligation of C and a product obtained by ligating A and B depending on the degree of proceeding of the ligation of A and B) is then started during the proceeding or after completion of the ligation of A and B, or it is acceptable that ligation of B and C is started in advance, and ligation of B and A (also including ligation of A and a product obtained by ligating B and C depending on the degree of proceeding of the ligation of B and C) is then started during the proceeding or after completion of the ligation of B and C. For example, by starting the ligation step in the presence of a complementary oligonucleotide for ligating A, B, and C, or A and B, and a complementary oligonucleotide for ligating B and C, ligation of A, B, and C can be simultaneously started, and thus, A, B, and C can be simultaneously ligated. For example, by starting the ligation step in the absence of C and/or in the absence of a complementary oligonucleotide for ligating B and C, and supplying C and/or the complementary oligonucleotide for ligating B and C to a reaction liquid after the start of the ligation step, ligation of A and B is started in advance, ligation of B and C (also including ligation of C and a product obtained by ligating A and B depending on the degree of proceeding of the ligation of A and B) can be then started during the proceeding or after completion of the ligation of A and B, and thus, A, B, and C can be ligated in turn. For example, by starting the ligation step in the absence of A and/or in the absence of a complementary oligonucleotide for ligating A and B, and supplying A and/or the complementary oligonucleotide for ligating A and B to a reaction liquid after the start of the ligation step, ligation of B and C is started in advance, ligation of B and A (also including ligation of A and a product obtained by ligating B and C depending on the degree of proceeding of the ligation of B and C) can be then started during the proceeding or after completion of the ligation of B and C, and thus, A, B, and C can be ligated in turn.

In the case of ligating three or more substrate oligonucleotides, ligation of the substrate oligonucleotides may correspond to performance of the method of the present invention as a whole. For example, in the case of ligating three substrate oligonucleotides A, B, and C in 5' to 3' direction, the ligation of A, B, and C (also including ligation of A and a product obtained by ligating B and C, and ligation of C and a product obtained by ligating A and B depending on the degree of proceeding of the ligation step) may correspond to performance of the method of the present invention, in which A, B, and C are used as substrate oligonucleotides, as a whole.

In the case of ligating three or more substrate oligonucleotides, ligation of the substrate oligonucleotides may independently correspond to performance of the method of the present invention, for ligation of each ligation site. For example, in the case of ligating three substrate oligonucleotides A, B, and C in 5' to 3' direction, ligation of A and B (also including ligation of A and a product obtained by ligating B and C depending on the degree of proceeding of the ligation step) may correspond to performance of the method of the present invention, in which A and B are used as substrate oligonucleotides, and ligation of B and C (also including ligation of C and a product obtained by ligating A and B depending on the degree of proceeding of the ligation step) may correspond to performance of the method of the present invention, in which B and C are used as substrate oligonucleotides.

By carrying out the ligation step in such a manner, the oligonucleotide of interest is generated, and thus, the reaction liquid containing the oligonucleotide of interest is provided.

The amount of the generated oligonucleotide of interest may be more than the used amount of the complementary oligonucleotide by molar ratio. In other words, the oligonucleotide of interest, of which the amount is more than the used amount of the complementary oligonucleotide by molar ratio, may be generated by catalytically repeatedly using the complementary oligonucleotide in the ligation step. For example, the amount of the generated oligonucleotide of interest may be, by molar ratio, more than 100%, 110% or more, 120% or more, 130% or more, 150% or more, 170% or more, 200% or more, 250% or more, or 300% or more as a proportion in a case in which the used amount of the complementary oligonucleotide is taken as 100%. "Used amount of complementary oligonucleotide" to be targeted for comparison may mean the used amount of complementary oligonucleotide in each ligation point in a case in which the number (N - 1) of the ligation sites is two or more. For example, "used amount of complementary oligonucleotide" to be targeted for comparison may be the minimum used amount of complementary oligonucleotides in ligation points, the maximum used amount of complementary oligonucleotides in the ligation points, or the mean value of the used amounts of complementary oligonucleotides in the ligation points in a case in which the number (N - 1) of the ligation sites is two or more. For optional ligation points, "used amount of complementary oligonucleotides" in ligation points targeted for comparison may mean the total used amount of the complementary oligonucleotides in a case in which the number of the complementary oligonucleotides used in the ligation points is two or more.

For optional ligation points, the amount of ligation products in the ligation points may be more than the used amount of complementary oligonucleotides in the ligation points by molar ratio. In other words, for the optional ligation points, the ligation products of which the amount is more than the used amount of the complementary oligonucleotides by molar ratio may be generated by catalytically repeatedly using the complementary oligonucleotides in the ligation step. For example, for the optional ligation points, the amount of the generated ligation products in the ligation points may be, by molar ratio, more than 100%, 110% or more, 120% or more, 130% or more, 150% or more, 170% or more, 200% or more, 250% or more, or 300% or more as a proportion in a case in which the used amount of the complementary oligonucleotides in the ligation points is taken as 100%. For the optional ligation points, "amount of ligation products" in the ligation points may mean the total amount of all the generated ligation products that are generated through ligation in the ligation points in a case in which the number (N - 1) of the ligation sites is two or more. For example, "amount of generated ligation product in ligation point between A and B" may mean the total amount the generated ligation product of A and B and the generated ligation products of A, B, and C in the case of ligating three substrate oligonucleotides A, B, and C in 5' to 3' direction. For optional ligation points, "used amount of complementary oligonucleotides" in ligation points targeted for comparison may mean the total used amount of the complementary oligonucleotides in a case in which the number of the complementary oligonucleotides used in the ligation points is two or more.

Generation of the oligonucleotide of interest can be confirmed by, for example, a known method used in detection or identification of a compound. Examples of such methods include HPLC, LC/MS, GC/MS, and NMR. These methods may be used singly, or in combination of two or more kinds thereof as appropriate. In a case in which a complementary oligonucleotide hybridizes with the oligonucleotide of interest, the complementary oligonucleotide and the oligonucleotide of interest may be separated, or need not be separated from each other as appropriate. For example, the separation can be performed by heating. For example, the heating temperature may be 40°C or more, 50°C or more, or 60°C or more, may be 100°C or less, 90°C or less, or 80°C or less, or may be a combination thereof. The oligonucleotide of interest can be collected from the reaction liquid as appropriate. The collection of the oligonucleotide of interest can be carried out by, for example, a known method used in separation and purification of a compound. Examples of such methods include: chromatographies such as ion exchange chromatography, reversed-phase chromatography, affinity chromatography, and gel filtration chromatography; electrophoreses such as polyacrylamide gel electrophoresis (PAGE); and membrane purification. These methods may be used singly, or in combination of two or more kinds thereof as appropriate. The oligonucleotide of interest may be purified to a desired degree. The oligonucleotide of interest may be modified so that, for example, the modification aspects, described as examples above, of the oligonucleotide of interest are achieved.

### Examples

The present invention is further specifically described with reference to nonlimiting Examples below. Unless otherwise specified, nucleic acid residues included in oligonucleotides used in Examples are unmodified DNA residues.

### [Example 1] Ligation Reaction of Oligonucleotide Using Complementary Strand into Which Abasic Site Was Introduced, or Complementary Strand into Which Abasic Site and Loop Structure Were Introduced

Five kinds of complementary strands in total of one kind of a complementary strand into which only an abasic site was introduced, three kinds of complementary strands into which abasic sites and loop structures were introduced (three patterns in which the loop structures were inserted into 5' sides, 3' sides, or both thereof), and one kind of a completely complementary strand, into which any destabilizing site was not introduced, as a control, were used as complementary strands (Figure 1A and Table 1). The abasic sites were introduced into residues complementary with ligation sites, and the loop structures were introduced into positions apart from the ligation sites.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared.

The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. After 1 hour, 3 hours, and 16 hours, a part of the reaction liquid was collected, and diluted with 10 mM of EDTA to stop the reaction.

The product was quantified by HPLC using ACQUITY UPLC OST C18 Column (1.7 µm, 2.1 × 50 mm). In HPLC, analyses were performed by linear gradients set forth in Table 2, using an eluent A comprising 100 mM of hexafluoroisopropanol, 8 mM of triethylamine, and 0.004% of phosphoric acid, and an eluent B comprising 10% of methanol as mobile phases under conditions of a column temperature of 60°C, a detection wavelength of 254 nm, an injection amount of 10 µL, and a flow rate of 0.4 mL/min. A product sample was analyzed in a similar manner, and a product of interest was confirmed to be obtained by enzyme reaction. The amount of the product was calculated from ratio of the peak area of the product to the total of the peak areas of the substrates and the product in an LC chart.

The results are indicated in Figure 1B. The amount of the product obtained 16 hours after the reaction was improved to 7.8 µM by introducing the abasic site, and further improved to 9.9 to 12.3 µM by introducing the abasic site and inserting the loop structure, compared with 2.0 µM in the case of using the completely complementary strand as the control. In other words, the complementary strands into which the destabilizing sites such as the abasic sites and the loop structures were introduced were confirmed to have catalytic action (that is, repeated utilization in ligation reaction of oligonucleotides). Moreover, the combination of the abasic site and the loop structure was confirmed to be effective at improving the efficiency of the ligation reaction of the oligonucleotides.

**[Table 1]**

| Table 1 Oligonucleotides Used | | |
|---|---|---|
| Application | Base sequence | SEQ ID NO: |
| Product sample | TCTTGGTTACATGAAATCCC | 1 |
| 5'-End substrate | TCTTGGTTAC | 2 |
| 3'-End substrate | pho-ATGAAATCCC | 3 |
| Complementary strand (control: completely complementary) | GGATTTCATGTAACCAAG | 4 |
| Complementary strand (only abasic site) | GGATTTCA(Ab)GTAACCAAG | 5 |
| Complementary strand (abasic site + loop structure (5' side)) | GGATTAATTTCA(Ab)GTAACCAAG | 6 |
| Complementary strand (abasic site + loop structure (3' side)) | GGATTTCA(Ab)GTAACCGGCCAAG | 7 |
| Complementary strand (abasic site +loop structure (5' side and 3' side)) | GGATTAATTTCA(Ab)GTAACCGGCCAAG | 8 |

| | | |
|---|---|---|
| pho: indicating modification of 5' end with phosphate group. (Ab): indicating abasic site. Residues enclosed in rectangle: indicating inserted residues. | | |

**[Table 2]**

| Table 2 Gradient | | |
|---|---|---|
| Time (min) | A% | B% |
| 0.00 | 95 | 5 |
| 8.00 | 75 | 25 |
| 8.10 | 10 | 90 |
| 10.50 | 10 | 90 |
| 10.60 | 95 | 5 |
| 14.00 | 95 | 5 |

### [Example 2] Ligation Reaction of Oligonucleotides with Modified Sugar Portions

The catalytic action of a complementary strand into which a destabilizing site was introduced was investigated for an oligonucleotide in which the position 2' of a sugar portion was modified. Two kinds of oligonucleotides in which a sugar portion with 1 residue or 5 residues was substituted with 2'-O-methoxyethyl were used as 5'-end substrates and 3'-end substrates, respectively (Table 3). In total, three kinds of complementary strands of a completely complementary strand as a control, a complementary strand into which an abasic site was introduced, and a complementary strand into which an abasic site and a loop structure were introduced were used as complementary strands (Table 3).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 0.25 hour, 1 hour, 4 hours, and 24 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 2. In the case of using the completely complementary strand as the control, the yield in each of two kinds of substrate patterns was 10% or less, 24 hours after the reaction. In contrast, in the case of using the complementary strand into which the abasic site was introduced, and in the case of using the complementary strand into which the abasic site and the loop structure were introduced, a yield of 38 to 51% was obtained 24 hours after the reaction. In other words, the catalytic action of the complementary strand into which the destabilizing sites such as the abasic site and the loop structure were introduced was also confirmed in the ligation reaction of the oligonucleotide in which the sugar portion was modified. The yields obtained 4 hours after the reaction were 21% (in a case in which the total number of modifications of sugar portions in a substrate was two) and 13% (in a case in which the total number of modifications of sugar portions in a substrate was ten) in the case of using the complementary strand into which the abasic site was introduced, and were 30% (in a case in which the total number of modifications of sugar portions in a substrate was two) and 29% (in a case in which the total number of modifications of sugar portions in a substrate was ten) in the case of using the complementary strand into which the abasic site and the loop structure were introduced. In other words, the combination of the abasic site and the loop structure was also confirmed to be effective at improving the efficiency of the ligation reaction of the oligonucleotides in the ligation reaction of the oligonucleotides with the modified sugar portions.

**[Table 3]**

| Table 3 Oligonucleotides Used | | |
|---|---|---|
| Application | Base sequence | SEQ ID NO: |
| 5'-End substrate (modification type ①) | TCT(m)TGGTTAC | 75 |
| 3'-End substrate (modification type ①) | pho-ATGAAATC(m)CC | 76 |
| 5'-End substrate (modification type ②) | T(m)C(m)T(m)T(m)G(m)GTTAC | 77 |
| 3'-End substrate (modification type ②) | pho-ATGAAA(m)T(m)C(m)C(m)C(m) | 78 |
| Complementary strand (control: completely complementary) | GGATTTCATGTAACCAAG | 4 |
| Complementary strand (abasic site) | GGATTTCA(Ab)GTAACCAAG | 5 |
| Complementary strand (abasic site + loop structure (5' side and 3' side)) | GGATTAATTTCA(Ab)GTAACCGGCCAAG | 8 |

| | | |
|---|---|---|
| pho: indicating modification of 5' end with phosphate group. (m): indicating modification with 2'-O-methoxyethyl group. (Ab): indicating abasic site. Residues enclosed in rectangle: indicating inserted residues. | | |

### [Example 3] Ligation Reaction of Oligonucleotides with Modified Phosphate Portions

The catalytic action of a complementary strand into which a destabilizing site was introduced was investigated for an oligonucleotide with a modified phosphate portion. OligoDNAs (PS-DNAs) in which all the phosphate ester bonds between nucleotides were substituted with thiophosphate ester bonds were used as a 5'-end substrate and a 3'-end substrate, respectively (Table 4). In total, four kinds of complementary strands of a completely complementary strand as a control, a complementary strand into which an abasic site was introduced, a complementary strand into which a loop structure was introduced, and a complementary strand into which an abasic site and a loop structure were introduced were used as complementary strands (Table 4).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 24 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 3. The yield obtained after 24 hours was 13% in the case of using the completely complementary strand as the control, and was improved to 43% in the case of using the complementary strand into which the abasic site was introduced and improved to 24% in the case of using the complementary strand into which the loop structure was introduced. In other words, the catalytic action of the complementary strand into which the destabilizing sites such as the abasic site and the loop structure were introduced was also confirmed in the ligation reaction of the oligonucleotide in which the phosphate portion was modified. The yield obtained after 24 hours was further improved to 50% in the case of using the complementary strand into which the abasic site and the loop structure were introduced. In other words, the combination of the abasic site and the loop structure was also confirmed to be effective at improving the efficiency of the ligation reaction of the oligonucleotides in the ligation reaction of the oligonucleotides with the modified phosphate portions.

**[Table 4]**

| Table 4 Oligonucleotides Used | | |
|---|---|---|
| Application | Base sequence | SEQ ID NO: |
| 5'-End substrate (PS-DNA type) | T^C^T^T^G^G^T^T^A^C | 79 |
| 3'-End substrate (PS-DNA type) | pho-A^T^G^A^A^A^T^C^C^C | 80 |
| Complementary strand (completely complementary) | GGATTTCATGTAACCAAG | 4 |
| Complementary strand (abasic site) | GGATTTCA(Ab)GTAACCAAG | 5 |
| Complementary strand (loop structure (5' side and 3' side)) | GGATTAATTTCATGTAACCGGCCAAG | 9 |
| Complementary strand (abasic site + loop structure (3' side)) | GGATTTCA(Ab)GTAACCGGCCAAG | 7 |

| | | |
|---|---|---|
| pho: indicating modification of 5' end with phosphate group. (Ab): indicating abasic site. ^: indicating substitution of phosphate group with thiophosphate group. Residues enclosed in rectangle: indicating inserted residues. | | |

### [Example 4] Ligation Reaction of Oligonucleotide Using Complementary Strand into Which Loop Structure Was Introduced

The catalytic action of a complementary strand into which a loop structure was inserted was investigated. A position (position -6 or position +6) apart by 6 residues from a ligation site toward a 5' side or a 3' side was selected as a position into which the loop structure was inserted (Table 5). The number of nucleic acid residues included in the loop structure was set at 4.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 16 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 4. In the case of using the complementary strand in which the loop structure was inserted at any position, the yield was increased with prolonging reaction time. The yield obtained after 16 hours was 3.8% in the case of using a completely complementary strand as a control, and was 10.7% in the case of using the complementary strand in which the loop structure was inserted into the 5' side, and 6.7% in the case of using the complementary strand in which the loop structure was inserted into the 3' side. In other words, the catalytic action of the complementary strand into which the destabilizing site such as the loop structure was introduced was confirmed.

**[Table 5]**

| Table 5 Oligonucleotides Used | | |
|---|---|---|
| Application | Base sequence | SEQ ID NO: |
| 5'-End substrate | TCTTGGTTAC | 2 |
| 3'-End substrate | pho-ATGAAATCCC | 3 |
| Complementary strand (control: completely complementary) | GGATTTCATGTAACCAAG | 4 |
| Complementary strand (loop structure (5' side)) | GGATTAATTTCATGTAACCAAG | 10 |
| Complementary strand (loop structure (3' side)) | GGATTTCATGTAACCGGCCAAG | 11 |

| | | |
|---|---|---|
| pho: indicating modification of 5' end with phosphate group. Residues enclosed in rectangle: indicating inserted residues. | | |

### [Example 5] Ligation Reaction of Oligonucleotide Using Complementary Strand into Which Deletion of Nucleic Acid Residue Was Introduced

The catalytic action of a complementary strand into which a deletion of nucleic acid residue was inserted was investigated. Seven kinds in total of complementary strands of a completely complementary strand as a control, three kinds of complementary strands (#3 to #1) in which 4 to 6 residues, 5 to 6 residues, and 6 residue were deleted, respectively, from a ligation site as an origin toward a 5' side, and three kinds of complementary strands (#6 to #4) in which 4 to 6 residues, 5 to 6 residues, and 6 residue were deleted, respectively, from the ligation site as an origin toward a 3' side were used as complementary strands (Table 6 and Figure 5A). When the complementary strands and substrates hybridize with each other, loop structures can be formed in the substrates by deleting the nucleic acid residues of the complementary strands (Figure 5A).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 16 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 5B. In the case of using each of the complementary strands into which the deletions of the nucleic acid residues were introduced, the yield obtained after 16 hours was more than the yield (5.1%) in the case of using the completely complementary strand as the control. In other words, the catalytic action of a complementary strand was confirmed even in the case of selecting a deletions of a nucleic acid residue as a destabilizing site that was introduced into the complementary strand. When the positions of the deleted residues were set from the ligation site toward the 5' side, the yield obtained after 16 hours was the most (10.5%) in a case in which the number of the deleted residue was 1, and the yield obtained after 16 hour was the least (6.2%) in a case in which the number of the deleted residues was 3. When the deleted residues were set from the ligation site toward the 3' side, the yield obtained after 16 hours was the most (14.1%) in a case in which the number of the deleted residues was 2, and the yield obtained after 16 hour was the least (9.2%) in a case in which the number of the deleted residue was 1.

**[Table 6]**

| Table 6 Oligonucleotides Used | | | | |
|---|---|---|---|---|
| Experiment number | Application | Base sequence | Deleted nucleic acid residues | SEQ ID NO: |
| | 5'-End substrate | TCTTGGTTAC | | 2 |
| | 3'-End substrate | pho-ATGAAATCCC | | 3 |
| | Complementary strand (control: completely complementary) | GGATTTCATGTAACCAAG | - | 4 |
| #1 | Complementary strand (deletion of 1 residue) | GGA-TTCATGTAACCAAG | T | 12 |
| #2 | Complementary strand (deletion of 2 residues) | GGA--TCATGTAACCAAG | TT | 13 |
| #3 | Complementary strand (deletion of 3 residues) | GGA---CATGTAACCAAG | TTT | 14 |
| #4 | Complementary strand (deletion of 1 residue) | GGATTTCATGTAAC-AAG | C | 15 |
| #5 | Complementary strand (deletion of 2 residues) | GGATTTCATGTAA--AAG | CC | 16 |
| #6 | Complementary strand (deletion of 3 residues) | GGATTTCATGTA---AAG | ACC | 17 |

| | | | | |
|---|---|---|---|---|
| pho: indicating modification of 5' end with phosphate group. "-" in #1 to #6: indicating nucleic acid residue deleted in comparison with the case of the completely complementary strand. | | | | |

### [Example 6] Examination of Number of Nucleic Acid Residues Included in Loop Structure

The influence of the number of nucleic acid residues included in a loop structure on the catalytic action of a complementary strand was evaluated. A position (position -6 or position +6) apart by 6 residues from a ligation site toward a 5' side or a 3' side was selected as a position into which the loop structure was inserted, and the number of the nucleic acid residues included in the loop structure was set at 1 to 8 (Table 7).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 0.2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 16 hours under the HPLC conditions described in Example 1, and the yield of the product was calculated. In the present example, the molar concentration of each of the complementary strands was 1/100 of the molar concentration of each substrate, and therefore, the maximum yield was 1% in a case in which each complementary strand was supposed to be used only once in the ligation reaction.

The results are set forth in Table 7. **In** the case of using the complementary strand into which the loop structure with each of the numbers of the residues was inserted, a higher yield was obtained than that in the case of using a completely complementary strand as a control. **In** other words, the catalytic action of the complementary strand was confirmed regardless of the number of the nucleic acid residues included in the loop structure introduced into the complementary strand. **In** particular, a high effect was obtained in a case in which the number of the nucleic acid residues included in the loop structure was 2 or more.

**[Table 7]**

| Table 7 Oligonucleotides Used, And Yields | | | |
|---|---|---|---|
| Application | Base sequence | Yield (%) | SEQ ID NO: |
| 5'-End substrate | TCTTGGTTAC | | 2 |
| 3'-End substrate | pho-ATGAAATCCC | | 3 |
| Complementary strand (control: completely complementary) | GGATTTCATGTAACCAAG | 0.57 | 4 |
| Complementary strand (loop structure (5' side), insertion of 1 residue) | GGATTTTCATGTAACCAAG | 1.1 | 18 |
| Complementary strand (loop structure (5' side), insertion of 2 residues) | GGATATTTCATGTAACCAAG | 1.1 | 19 |
| Complementary strand (loop structure (5' side), insertion of 3 residues) | GGATTATTTCATGTAACCAAG | 1.3 | 20 |
| Complementary strand (loop structure (5' side), insertion of 4 residues) | GGATTAATTTCATGTAACCAAG | 1.3 | 10 |
| Complementary strand (loop structure (5' side), insertion of 5 residues) | GGATTGAATTTCATGTAACCAAG | 1.3 | 21 |
| Complementary strand (loop structure (5' side), insertion of 6 residues) | GGATTGCAATTTCATGTAACCAAG | 1.4 | 22 |
| Complementary strand (loop structure (5' side), insertion of 7 residues) | GGATTGCCAATTTCATGTAACCAAG | 1.4 | 23 |
| Complementary strand (loop structure (5' side), insertion of 8 residues) | GGATTGCGCAATTTCATGTAACCAAG | 1.4 | 24 |
| Complementary strand (loop structure (3' side), insertion of 1 residue) | GGATTTCATGTAACCCAAG | 0.69 | 25 |
| Complementary strand (loop structure (3' side), insertion of 2 residues) | GGATTTCATGTAACCGCAAG | 1.1 | 26 |
| Complementary strand (loop structure (3' side), insertion of 3 residues) | GGATTTCATGTAACCGCCAAG | 1.3 | 27 |
| Complementary strand (loop structure (3' side), insertion of 4 residues) | GGATTTCATGTAACCGGCCAAG | 1.3 | 11 |
| Complementary strand (loop structure (3' side), insertion of 5 residues) | GGATTTCATGTAACCGGACCAAG | 1.2 | 28 |
| Complementary strand (loop structure (3' side), insertion of 6 residues) | GGATTTCATGTAACCGGTACCAAG | 1.3 | 29 |
| Complementary strand (loop structure (3' side), insertion of 7 residues) | GGATTTCATGTAACCGGTAACCAAG | 1.1 | 30 |
| Complementary strand (loop structure (3' side), insertion of 8 residues) | GGATTTCATGTAACCGGTTAACCAAG | 1.1 | 31 |

| | | | |
|---|---|---|---|
| pho: indicating modification of 5' end with phosphate group. Residues enclosed in rectangle: indicating inserted residues. | | | |

### [Example 7] Examination of Insertion Position of Loop Structure

The influence of the insertion position of a loop structure on the catalytic action of a complementary strand was evaluated. Positions (positions -3 to -6 or positions +3 to +6) apart by 3 to 6 residues from a ligation site toward a 3' side or a 5' side were selected as positions into which the loop structure was inserted (Table 8). The number of nucleic acid residues included in the loop structure was set at 4.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 0.2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 16 hours under the HPLC conditions described in Example 1, and the yield of the product was calculated. In the present example, the molar concentration of each of the complementary strands was 1/100 of the molar concentration of each substrate, and therefore, the maximum yield was 1% in a case in which each complementary strand was supposed to be used only once in the ligation reaction.

The results are set forth in Table 8. In the case of using the complementary strand in which the loop structure was inserted into each insertion position, a higher yield was obtained than that in the case of using a completely complementary strand as a control. In other words, the catalytic action of the complementary strand was confirmed regardless of the position of the loop structure introduced into the complementary strand. In particular, in the case of inserting the loop structure into each of the 5' side and 3' side of the ligation site, a reaction rate was the highest in the case of inserting the loop structure into the position (position -5 or position +5) apart by 5 residues from the ligation site.

**[Table 8]**

| Table 8 Oligonucleotides Used, And Yields | | | |
|---|---|---|---|
| Application | Base sequence | SEQ ID NO: | Yield (%) |
| 5'-End substrate | TCTTGGTTAC | 2 | - |
| 3'-End substrate | pho-ATGAAATCCC | 3 | - |
| Complementary strand (control: completely complementary) | GGATTTCATGTAACCAAG | 4 | 2.2 |
| Complementary strand (insertion of loop structure into position -6) | GGATTAATTTCATGTAACCAAG | 10 | 4.6 |
| Complementary strand (insertion of loop structure into position -5) | GGATTTAATTCATGTAACCAAG | 32 | 5.7 |
| Complementary strand (insertion of loop structure into position -4) | GGATTTCGATGTAACCAAG | 33 | 5.0 |
| Complementary strand (insertion of loop structure into position -3) | GGATTCATGCATGTAACCAAG | 34 | 4.1 |
| Complementary strand (insertion of loop structure into position +3) | GGATTTCATGTATATAACCAAG | 35 | 5.2 |
| Complementary strand (insertion of loop structure into position +4) | GGATTTCATGTTAACCAAG | 36 | 5.4 |
| Complementary strand (insertion of loop structure into position +5) | GGATTTCATGTAACGTACCAAG | 37 | 6.2 |
| Complementary strand (insertion of loop structure into position +6) | GGATTTCATGTAACCGGCCAAG | 11 | 4.0 |

| | | | |
|---|---|---|---|
| pho: indicating modification of 5' end with phosphate group. Residues enclosed in rectangle: indicating inserted residues. | | | |

### [Example 8] Examination of Base Sequence of Nucleic Acid Residues Included in Loop Structure

The influence of the base sequence of nucleic acid residues included in a loop structure on the catalytic action of a complementary strand was evaluated. A position into which the loop structure was inserted was set at a position (position +4 or position +6) apart by 4 or 6 residues from the ligation site toward a 3' side, and the number of the nucleic acid residues included in the loop structure was set at 4, and four kinds of base sequences were set for each position (Table 9).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 0.2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 16 hours under the HPLC conditions described in Example 1, and the yield of the product was calculated. In the present example, the molar concentration of each of the complementary strands was 1/100 of the molar concentration of each substrate, and therefore, the maximum yield was 1% in a case in which each complementary strand was supposed to be used only once in the ligation reaction.

The results are set forth in Table 8. In the case of using the complementary strand into which the loop structure consisting of each base sequence was inserted, a higher yield was obtained than that in the case of using a completely complementary strand as a control. In other words, the catalytic action of the complementary strand was confirmed regardless of the base sequence of the nucleic acid residues included in the loop structure introduced into the complementary strand.

**[Table 9]**

| Table 9 Oligonucleotides Used, And Yields | | | |
|---|---|---|---|
| Application | Base sequence | SEQ ID NO: | Yield (%) |
| 5'-End substrate | TCTTGGTTAC | 2 | - |
| 3'-End substrate | pho-ATGAAATCCC | 3 | - |
| Complementary strand (control: completely complementary) | GGATTTCATGTAACCAAG | 4 | 2.1 |
| Complementary strand (loop insertion position +6) | GGATTTCATGTAACCGGCCAAG | 11 | 4.6 |
| Complementary strand (loop insertion position +6) | GGATTTCATGTAACCAACCAAG | 38 | 5.6 |
| Complementary strand (loop insertion position +6) | GGATTTCATGTAACCTTCCAAG | 39 | 5.0 |
| Complementary strand (loop insertion position +6) | GGATTTCATGTAACCCCGGAAG | 40 | 4.1 |
| Complementary strand (loop insertion position +4) | GGATTTCATGTAAGGCCCCAAG | 41 | 5.1 |
| Complementary strand (loop insertion position +4) | GGATTTCATGTAAGGAACCAAG | 42 | 5.4 |
| Complementary strand (loop insertion position +4) | GGATTTCATGTAAGGTTCCAAG | 43 | 6.2 |
| Complementary strand (loop insertion position +4) | GGATTTCATGTAACCGGCCAAG | 11 | 4.0 |

| | | | |
|---|---|---|---|
| pho: indicating modification of 5' end with phosphate group. (Ab): indicating abasic site. Residues enclosed in rectangle: indicating inserted residues. | | | |

### [Example 9] Examination of Number of Loop Structures

The influence of the number of loop structures on the catalytic action of a complementary strand was evaluated. The number of nucleic acid residues included in each loop structure was set at 4, and complementary strands into which loop structures at one to four points were inserted were designed (Table 10).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 16 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 6. In the case of using the complementary strands into which the loop structures at the one to three points were inserted, a higher yield was obtained after 16 hours than that in the case of using a completely complementary strand as a control, and a tendency for the generated amount to increase with increasing the number of the inserted loop structures was shown. In other words, the catalytic action of the complementary strand was confirmed in a case in which the number of the loop structures introduced into the complementary strand was 1 to 3. Moreover, a combination of the two or three loop structures was confirmed to be effective at improving the efficiency of the ligation reaction of the oligonucleotides. In the case of using the complementary strand into which the loop structures at the four points were inserted, a higher yield was obtained after 16 hours than that in the case of using a completely complementary strand as a control, under only one condition among the four conditions.

**[Table 10]**

| Table 10 Oligonucleotides Used | | | |
|---|---|---|---|
| Experiment number | Application | Base sequence | SEQ ID NO: |
| | 5'-End substrate | TCTTGGTTAC | 2 |
| | 3'-End substrate | pho-ATGAAATCCC | 3 |
| | Complementary strand (control: completely complementary) | GGATTTCATGTAACCAAG | 4 |
| #1 | Complementary strand (loop structure at one point) | GGATTAATTTCATGTAACCAAG | 10 |
| #2 | Complementary strand (loop structure at one point) | GGATTTCATGTAACCGGCCAAG | 11 |
| #3 | Complementary strand (loop structures at two points) | GGATTAATTTCATGTAACCGGCCAAG | 9 |
| #4 | Complementary strand (loop structures at two points) | GGATTAATTTCATGTATATAACCAAG | 44 |
| #5 | Complementary strand (loop structures at two points) | GGATTTCATGCATGTAACCGGCCAAG | 45 |
| #6 | Complementary strand (loop structures at three points) | GGATTAATTTCATGTATATAACCATGCAAG | 46 |
| #7 | Complementary strand (loop structures at three points) | GGATTAATTTCATGTATATAACCGGCCAAG | 47 |
| #8 | Complementary strand (loop structures at three points) | GGATTAATTTICATGCATGTAACCGGCCAAG | 48 |
| #9 | Complementary strand (loop structures at four points) | GGATATATTTICATGCATGTATATAACCGGCCAAG | 49 |
| #10 | Complementary strand (loop structures at four points) | GGATTAATTTCATGCATGTATATAACCATGCAAG | 50 |
| #11 | Complementary strand (loop structures at four points) | GGATTAATTTCATGCATGTATATAACCGGCAAG | 51 |
| #12 | Complementary strand (loop structures at four points) | GGATATATTTCATGCATGTATATAACCATGCAAG | 52 |

| | | | |
|---|---|---|---|
| pho: indicating modification of 5' end with phosphate group. Residues enclosed in rectangle: indicating inserted residues. | | | |

### [Example 10] Examination of Introduction Positions and Numbers of Abasic Sites

The influence of the introduction positions and number of abasic sites on the catalytic action of a complementary strand was evaluated. Complementary strands into which abasic sites at one to three points were introduced were designed (Figure 7A and Table 11).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 16 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 7B. In the case of using the complementary strand into which the abasic sites at the one or two points were introduced, a tendency for a higher yield to be obtained than that in the case of using a completely complementary strand as a control is shown after 1 hour, and higher yields were obtained after 3 hours and 16 hours than that in the case of using the completely complementary strand as the control. In other words, the catalytic action of the complementary strand was confirmed in a case in which the number of the abasic sites introduced into the complementary strand was 1 to 2. In the case of using the complementary strand in which the abasic sites were introduced into two points of complementary strand positions 9_14, higher yields were obtained after 1 hour and 3 hours than that in the case of using the complementary strand in which the abasic site was introduced into one point of a complementary strand position 9; however, the maximum yield was obtained after 16 hours in the case of using the complementary strand in which the abasic site was introduced into the one point of the complementary strand position 9 (Figure 7B). In contrast, the yield was decreased in the case of using the complementary strand into which the three points of the abasic sites were introduced.

**[Table 11]**

| Table 11 Oligonucleotides Used | | | |
|---|---|---|---|
| Application | Introduction position of abasic site | Base sequence | SEQ ID NO: |
| 5'-End substrate | | TCTTGGTTAC | 2 |
| 3'-End substrate | | pho-ATGAAATCCC | 3 |
| Complementary strand (control: completely complementary) | | GGATTTCATGTAACCAAG | 4 |
| Complementary strand (abasic site) | 9 | GGATTTCA(Ab)GTAACCAAG | 5 |
| Complementary strand (abasic sites at two points) | 9_14 | GGATTTCA(Ab)GTAA(Ab)CAAG | 53 |
| Complementary strand (abasic sites at two points) | 4_9 | GGA(Ab)TTCA(Ab)GTAACCAAG | 54 |
| Complementary strand (abasic sites at three points) | 4_9_14 | GGA(Ab)TTCA(Ab)GTAA(Ab)CAAG | 55 |

| | | | |
|---|---|---|---|
| pho: indicating modification of 5' end with phosphate group. (Ab): indicating abasic site. A number was assigned to the introduction position of an abasic site with respect to the 5' end of a complementary strand, numbered 1. | | | |

### [Example 11] Ligation Reaction of Oligonucleotide Using Complementary Strand into Which Mismatch Site Was Introduced

The catalytic action of a complementary strand into which a mismatch site was introduced was investigated. The introduction of a mismatch base pair into a ligation site was examined. The bases (T and G, respectively) of the complementary strand, paired with the base (A, position -1) on the 3' side and the base (C, position +1) on the 5' side of the ligation site, were substituted with other bases, and the substitution was evaluated (Table 12, Figure 8A).

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, and 16 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 8B. The yield obtained after 16 hours was 4.5% in the case of using a completely complementary strand as a control, and was improved to 5.3 to 28.8% in the case of using the complementary strand into which the mismatch site was introduced (Figure 8B). In other words, the catalytic action of the complementary strand was also confirmed in the case of selecting the mismatch site as a destabilizing site that was introduced into the complementary strand.

**[Table 12]**

| Table 12 Oligonucleotides Used | | |
|---|---|---|
| Application | Base sequence | SEQ ID NO: |
| 5'-End substrate | TCTTGGTTAC | 2 |
| 3'-End substrate | pho-ATGAAATCCC | 3 |
| Complementary strand (control: completely complementary) | GGATTTCATGTAACCAAG | 4 |
| Complementary strand (mismatch) | GGATTTCAAGTAACCAAG | 56 |
| Complementary strand (mismatch) | GGATTTCAGGTAACCAAG | 57 |
| Complementary strand (mismatch) | GGATTTCACCGTAACCAAG | 58 |
| Complementary strand (mismatch) | GGATTTCATATAACCAAG | 59 |
| Complementary strand (mismatch) | GGATTTCATTTAACCAAG | 60 |
| Complementary strand (mismatch) | GGATTTCATCTAACCAAG | 61 |

| | | |
|---|---|---|
| pho: indicating modification of 5' end with phosphate group. Residue enclosed in rectangle: indicating mismatch site. | | |

### [Example 12] Examination of Substrate Concentration

The influence of a substrate concentration and a complementary strand concentration on ligation reaction of a substrate was evaluated. The substrate concentration was set at 20, 100, 300, 500, or 1000 µM. The complementary strand concentration was set at a molar concentration that was 1/10 of the substrate concentration. Two kinds of complementary strands of a complementary strand into which an abasic site was introduced and a complementary strand into which an abasic site and a loop structure were introduced were used as complementary strands (Table 13).

Ligation reaction of oligonucleotides (5'-end substrates and 3'-end substrates) which were substrates was performed using each complementary strand at each substrate concentration and each complementary strand concentration, and generation of products (oligonucleotides in which the 5'-end substrates and the 3'-end substrates were ligated) was compared. The composition of the reaction liquid, other than the substrates and the complementary strands, includes 60 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 1 hour, 3 hours, 16 hours, and 40 hours under the HPLC conditions described in Example 1, and the yield of the product and the generation rate of the product per enzymatic activity were calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figures 9A and 9B. A yield of more than 10%, which was the ratio of the amount of addition of the complementary strand to that of the substrate, was obtained at each of the complementary strand concentrations and the substrate concentrations. In other words, the catalytic action of the complementary strand was confirmed regardless of the complementary strand concentration and the substrate concentration. The maximum yield was obtained at a substrate concentration of 300 µM in the case of using each complementary strand (Figure 9A). The generation rate of the product per enzyme was increased with increasing the substrate concentration (Figure 9B).

**[Table 13]**

| Table 13 Oligonucleotides Used | | |
|---|---|---|
| Application | Base sequence | SEQ ID NO: |
| 5'-End substrate | TCTTGGTTAC | 2 |
| 3'-End substrate | pho-ATGAAATCCC | 3 |
| Complementary strand (only abasic site) | GGATTTCA(Ab)GTAACCAAG | 5 |
| Complementary strand (abasic site + loop structure (5' side and 3' side)) | GGATTAATTTCA(Ab)GTAACCGGCCAAG | 8 |

| | | |
|---|---|---|
| pho: indicating modification of 5' end with phosphate group. (Ab): indicating abasic site. Residues enclosed in rectangle: indicating inserted residues. | | |

### [Example 13] Generation of Oligonucleotide Having Length of 100 Residues by Ligation Reaction of Oligonucleotides with 50 Residues

The catalytic action of a complementary strand in ligation reaction using two substrates with 50 residues was investigated. The complementary strand was designed to form a double strand in a region with 9 residues from a ligation site toward each of a 5' side and a 3' side. As destabilizing sites, an abasic site was introduced into the position of a complementary strand, paired with the base (A, position +1) of the ligation site of the 3'-end substrate, and loop structures were introduced into the 5' end side (position of the third base from the upstream side) and 3' end side (position of the third base from the downstream side) of the complementary strand, or these destabilizing sites were introduced in combination. Oligonucleotides used are set forth in Table 14.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 150 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 24 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 10. The yields after 24 hours were 9.7% in the case of using a completely complementary strand as a control and 14.0% in the case of using the complementary strand into which the abasic site was introduced, whereas the yields after 24 hours were 57.8% in the case of using the complementary strand into which the abasic site and the 5'-side loop structure were introduced, 50.5% in the case of using the complementary strand into which the abasic site and the 3'-side loop structure were introduced, and 61.8% in the case of using the complementary strand into which the abasic site and the 5'- and 3'-side loop structures were introduced. In other words, in the generation of the oligonucleotide having a length of 100 residues by the ligation reaction of the oligonucleotides with 50 residues, the catalytic action of the complementary strand was also confirmed, and the effect of combining the destabilizing sites was also confirmed.

**[Table 14]**

| Table 14 Oligonucleotides Used | | |
|---|---|---|
| Application | Base sequence | SEQ ID NO: |
| 5'-End substrate | CTGGCATAAGCGTAGCAGTCCTGGC ATAAGCGTAGCAGTCTCTTGGTTAC | 62 |
| 3'-End substrate | pho-ATGAAATCCCCTGGCATAAGCGTAG CAGTCCTGGCATAAGCGTAGCAGTC | 63 |
| Complementary strand (control: completely complementary) | GGATTTCATGTAACCAAG | 4 |
| Complementary strand (abasic site) | GGATTTCA(Ab)GTAACCAAG | 5 |
| Complementary strand (abasic site + loop structure (5' side)) | GGATTAATTTCA(Ab)GTAACCAAG | 6 |
| Complementary strand (abasic site + loop structure (3' side)) | GGATTTCA(Ab)GTAACCGGCCAAG | 7 |
| Complementary strand (abasic site + loop structure (5' side and 3' side)) | GGATTAATTTCA(Ab)GTAACCGGCCAAG | 8 |

| | | |
|---|---|---|
| pho: indicating modification of 5' end with phosphate group. (Ab):indicating abasic site. Residues enclosed in rectangle: indicating inserted residues. | | |

### [Example 14] Generation of Oligonucleotide Having Length of 100 Residues by Ligation Reaction of Oligonucleotides with 50 Residues Comprising Modified Nucleic Acids

The catalytic action of a complementary strand in ligation reaction using two substrates with 50 residues comprising a 2'-O-methoxyethyl group, a 2'-O-methyl group, a 2'-fluoro group, 5-methylcytosine, and a thiophosphate group as modified nucleic acids was investigated. The complementary strand was designed to form a double strand in a region with 9 residues from a ligation site toward each of a 5' side and a 3' side. As destabilizing sites, an abasic site was introduced into the position of a complementary strand, paired with the base (A, position +1) of the ligation site of the 3'-end substrate, and loop structures were introduced into the 5' end side (position of the third base from the upstream side) and 3' end side (position of the third base from the downstream side) of the complementary strand, or these destabilizing sites were introduced in combination. Oligonucleotides used are set forth in Table 15.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 150 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 24 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 11. The yields after 24 hours were 7.7% in the case of using a completely complementary strand as a control and 8.3% in the case of using the complementary strand into which the abasic site was introduced, whereas the yields after 24 hours were 72.4% in the case of using the complementary strand into which the abasic site and the 5'-side loop structure were introduced, 27.3% in the case of using the complementary strand into which the abasic site and the 3'-side loop structure were introduced, and 88.3% in the case of using the complementary strand into which the abasic site and the 5'- and 3'-side loop structures were introduced. **In** other words, in the generation of the oligonucleotide having a length of 100 residues by the ligation reaction of the oligonucleotides with 50 residues comprising the modified nucleic acids, the catalytic action of the complementary strand was also confirmed, and the effect of combining the destabilizing sites was also confirmed.

**[Table 15]**

| Table 15 Oligonucleotides Used | | |
|---|---|---|
| Application | Base sequence | SEQ ID NO: |
| 5'-End substrate | CT(m)G^GCA(M)TAAGCG(F)TAGCA(m)GT ^CCT(M)GGCATAA(F)GCG^TA(m)GCAGTC U(F)C(M)U(F)T(m)G(m)GTTAC | 64 |
| 3'-End substrate | pho- ATGAAA(m)T(m)C(F)C(M)C(F)CTGG^CAT( m)AAG5(m)GTA^GCAG(m)TC5(m)TGG(m) CATA(m)AGC^GTAG5(m)AGTC | 65 |
| Complementary strand (control: completely complementary) | GGATTTCATGTAACCAAG | 4 |
| Complementary strand (abasic site) | GGATTTCA(Ab)GTAACCAAG | 5 |
| Complementary strand (abasic site + loop structure (5' side)) | GGATTAATTTCA(Ab)GTAACCAAG | 6 |
| Complementary strand (abasic site + loop structure (3' side)) | GGATTTCA(Ab)GTAACCGGCCAAG | 7 |
| Complementary strand (abasic site +loop structure (5' side and 3' side)) | GGATTAATTTCA(Ab)GTAACCGGCCAAG | 8 |

| | | |
|---|---|---|
| (m): indicating modification by 2'-O-methoxyethyl group. (M): indicating modification by 2'-O-methyl group. (F): indicating modification by 2'-fluoro group. 5(m): indicating 5-methyldeoxycytidine modified by a 2'-O-methoxyethyl group. ^ :indicating substitution of phosphate group with thiophosphate group. pho: indicating modification of 5' end with phosphate group. (Ab):indicating abasic site. Residues enclosed in rectangle: indicating inserted residues. | | |

### [Example 15] Ligation Reaction of Three Oligonucleotides

The catalytic action of a complementary strand in ligation reaction using three substrates of a 5'-end substrate with 10 residues, a central substrate with 20 residues, and a 3'-end substrate with 10 residues was investigated. The complementary strand complementary with the 5'-end substrate and the central substrate was designed to form a double strand in a region with 9 residues from a ligation site toward each of a 5' end side and a central substrate side. As destabilizing sites, an abasic site was introduced into the position of a complementary strand, paired with the base (A, position +1) of the ligation site of the 3'-end substrate, and loop structures were introduced into the 5' end side (position of the third base from the upstream side) and 3' end side (position of the third base from the downstream side) of the complementary strand, or these destabilizing sites were introduced in combination. The complementary strand complementary with the central substrate and the 3'-end substrate was designed to form a double strand in a region with 8 residues from the ligation site toward each of the central substrate side and the 3'-end side. As a destabilizing site, the residue of the complementary strand, paired with the base (T, position -1) of the ligation site of the 5'-end substrate, was regarded as a mismatch (C). Oligonucleotides used are set forth in Table 16.

Ligation reaction of oligonucleotides (5'-end substrates, central substrates, and 3'-end substrates) which were substrates was performed in combination of each of complementary strands corresponding to two ligation sites, and generation of products (oligonucleotides in which the 5'-end substrates, the central substrates, and the 3'-end substrates were ligated) was compared. Combinations of complementary strands in cases in which completely complementary strands were used in both of ligation of a 5'-end substrate and a central substrate, and ligation of a central substrate and a 3'-end substrate were regarded as control conditions. As examination of complementary strands into which destabilizing sites were introduced, four kinds of complementary strands in which an abasic site was introduced into the position of a complementary strand, paired with the base (A, position +1) of the ligation site of a 3'-end substrate, a loop structure was introduced into the 5'-end side (position of the third base from the upstream side) of a complementary strand, a loop structure was introduced into the 3'-end side (position of the third base from the downstream side) of a complementary strand, and these destabilizing sites were introduced in combination were used in ligation of a 5'-end substrate and a central substrate, a complementary strand in which a mismatch was introduced was used in ligation of a central substrate and a 3'-end substrate, and four kinds in total of reaction conditions in which the complementary strands were combined were examined. The composition of the reaction liquid includes 20 µM of each substrate, 2 µM of a complementary strand, 150 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 72 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 12. The yield was also increased with prolonging reaction time in the ligation reaction using the three substrates. The yields after 72 hours were 1.6% under the control condition, 19.3% under the condition using the complementary strand into which the abasic site was introduced in the ligation of the 5'-end substrate and the central substrate, and using the complementary strand into which the mismatch was introduced in the ligation of the central substrate and the 3'-end substrate, 27.5% under the condition using the complementary strand introduced into the abasic site and the loop structure (5'-end side) in the ligation of the 5'-end substrate and the central substrate, and using the complementary strand into which the mismatch was introduced in the ligation of the central substrate and the 3'-end substrate, 24.4% under the condition using the complementary strand introduced into the abasic site and the loop structure (3'-end side) in the ligation of the 5'-end substrate and the central substrate, and using the complementary strand into which the mismatch was introduced in the ligation of the central substrate and the 3'-end substrate, and 33.9% under the condition using the complementary strand introduced into the abasic site and the loop structures (5'-end side and 3'-end side) in the ligation of the 5'-end substrate and the central substrate, and using the complementary strand into which the mismatch was introduced in the ligation of the central substrate and the 3'-end substrate. **In** other words, the catalytic action of the complementary strand was also confirmed in the ligation reaction of the three oligonucleotides.

**[Table 16]**

| Table 16 Oligonucleotides Used | | |
|---|---|---|
| Application | Base sequence | SEQ ID NO: |
| 5'-End substrate | TCTTGGTTAC | 2 |
| Central substrate | pho-ATGAAATCCCGCCTCAGTCT | 66 |
| 3'-End substrate | pho-GCTTCGCACC | 67 |
| Complementary strand (5'-end substrate/central substrate, control: complete complementary) | GGATTTCATGTAACCAAG | 4 |
| Complementary strand (5'-end substrate/central substrate, abasic site) | GGATTTCA(Ab)GTAACCAAG | 5 |
| Complementary strand (5'-end substrate/central substrate, abasic site + loop structure (5' side)) | GGATTAATTTCA(Ab)GTAACCAAG | 6 |
| Complementary strand (5'-end substrate/central substrate, abasic site + loop structure (3' side)) | GGATTTCA(Ab)GTAACCGGCCAAG | 7 |
| Complementary strand (5'-end substrate/central substrate, abasic site + loop structure (5' side and 3' side)) | GGATTAATTTCA(Ab)GTAACCGGCCAAG | 8 |
| Complementary strand (central substrate /3'-end substrate, control: complete complementary) | GGTGCGAAGCAGACTGAGGC | 68 |
| Complementary strand (central substrate /3'-end substrate, mismatch) | TGCGAAGCCGACTGAG | 69 |

| | | |
|---|---|---|
| pho: indicating modification of 5' end with phosphate group. (Ab):indicating abasic site. Residue enclosed in rectangle: indicating mismatch site. Underlined residues: indicating inserted residues. | | |

### [Example 16] Ligation Reaction of Three Oligonucleotides Comprising Modified Nucleic Acids

The catalytic action of a complementary strand in ligation reaction using three substrates of a 5'-end substrate with 10 residues, a central substrate with 20 residues, and a 3'-end substrate with 10 residues, comprising a 2'-O-methoxyethyl group, a 2'-O-methyl group, a 2'-fluoro group, 5-methylcytosine, and a thiophosphate group as modified nucleic acids, was investigated. The complementary strand complementary with the 5'-end substrate and the central substrate was designed to form a double strand in a region with 9 residues from a ligation site toward each of a 5' end side and a central substrate side. As destabilizing sites, an abasic site was introduced into the position of a complementary strand, paired with the base (A, position +1) of the ligation site of the 3'-end substrate, and loop structures were introduced into the 5' end side (position of the third base from the upstream side) and 3' end side (position of the third base from the downstream side) of the complementary strand, or these destabilizing sites were introduced in combination. The complementary strand complementary with the central substrate and the 3'-end substrate was designed to form a double strand in a region with 8 residues from the ligation site toward each of the central substrate side and the 3'-end side. As a destabilizing site, the residue of the complementary strand, paired with the base (T, position -1) of the ligation site of the 5'-end substrate, was regarded as a mismatch (C). Oligonucleotides used are set forth in Table 17.

Ligation reaction of oligonucleotides (5'-end substrates, central substrates, and 3'-end substrates) which were substrates was performed in combination of each of complementary strands corresponding to two ligation sites, and generation of products (oligonucleotides in which the 5'-end substrates, the central substrates, and the 3'-end substrates were ligated) was compared. Combinations of complementary strands in cases in which completely complementary strands were used in both of ligation of a 5'-end substrate and a central substrate, and ligation of a central substrate and a 3'-end substrate were regarded as control conditions. As examination of complementary strands into which destabilizing sites were introduced, four kinds of complementary strands in which an abasic site was introduced into the position of a complementary strand, paired with the base (A, position +1) of the ligation site of a 3'-end substrate, a loop structure was introduced into the 5'-end side (position of the third base from the upstream side) of a complementary strand, a loop structure was introduced into the 3'-end side (position of the third base from the downstream side) of a complementary strand, and these destabilizing sites were introduced in combination were used in ligation of a 5'-end substrate and a central substrate, a complementary strand in which a mismatch was introduced was used in ligation of a central substrate and a 3'-end substrate, and four kinds in total of reaction conditions in which the complementary strands were combined were examined. The composition of the reaction liquid includes 20 µM of each substrate, 2 µM of a complementary strand, 150 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 24 hours under the HPLC conditions described in Example 1, and the yield of the product was calculated. **In** the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 13. The yield was also increased with prolonging reaction time in the ligation reaction using the three substrates comprising the modified nucleic acids. The yields after 24 hours were 1.6% under the control condition, 13.0% under the condition using the complementary strand into which the abasic site was introduced in the ligation of the 5'-end substrate and the central substrate, and using the complementary strand into which the mismatch was introduced in the ligation of the central substrate and the 3'-end substrate, 22.8% under the condition using the complementary strand introduced into the abasic site and the loop structure (5'-end side) in the ligation of the 5'-end substrate and the central substrate, and using the complementary strand into which the mismatch was introduced in the ligation of the central substrate and the 3'-end substrate, 21.1% under the condition using the complementary strand introduced into the abasic site and the loop structure (3'-end side) in the ligation of the 5'-end substrate and the central substrate, and using the complementary strand into which the mismatch was introduced in the ligation of the central substrate and the 3'-end substrate, and 22.5% under the condition using the complementary strand introduced into the abasic site and the loop structures (5'-end side and 3'-end side) in the ligation of the 5'-end substrate and the central substrate, and using the complementary strand into which the mismatch was introduced in the ligation of the central substrate and the 3'-end substrate. In other words, the catalytic action of the complementary strand was also confirmed in the ligation reaction of the three oligonucleotides comprising the modified nucleic acids.

**[Table 17]**

| Table 17 Oligonucleotides Used | | |
|---|---|---|
| Application | Base sequence | SEQ ID NO: |
| 5'-End substrate | T(m)^C(M)T(m)T(m)G(F)GTTAC | 70 |
| Central substrate | pho- ATGAAA(m)T(m)C(F)5(m)C(M)G(m) C(F)5(m)T(m)5(m)AGTCT | 71 |
| 3'-End substrate | pho-GCTTCG(m)5(m)A(F)^C(M)5(m) | 72 |
| Complementary strand (5'-end substrate/central substrate, control: complete complementary) | GGATTTCATGTAACCAAG | 4 |
| Complementary strand (5'-end substrate/central substrate, abasic site) | GGATTTCA(Ab)GTAACCAAG | 5 |
| Complementary strand (5'-end substrate/central substrate, abasic site + loop structure (5' side)) | GGATTAATTTCA(Ab)GTAACCAAG | 6 |
| Complementary strand (5'-end substrate/central substrate, abasic site + loop structure (3' side)) | GGATTTCA(Ab)GTAACCGGCCAAG | 7 |
| Complementary strand (5'-end substrate/central substrate, abasic site + loop structure (5' side and 3' side)) | GGATTAATTTCA(Ab)GTAACCGGCCAAG | 8 |
| Complementary strand (central substrate /3'-end substrate, control: complete complementary) | GGTGCGAAGCAGACTGAGGC | 68 |
| Complementary strand (central substrate /3'-end substrate, mismatch) | TGCGAAGCCGACTGAG | 69 |

| | | |
|---|---|---|
| (m): indicating modification by 2'-O-methoxyethyl group. (M): indicating modification by 2'-O-methyl group. (F): indicating modification by 2'-fluoro group. 5(m): indicating 5-methyldeoxycytidine into which a 2'-O-methoxyethyl group was introduced. ^ :indicating substitution of phosphate group with thiophosphate group. pho: indicating modification of 5' end with phosphate group. (Ab):indicating abasic site. Residue enclosed in rectangle: indicating mismatch site. Underlined residues: indicating inserted residues. | | |

### [Example 17] Ligation Reaction of Oligonucleotides Including Modified Nucleic Acid and RNA, and Having Length of 20 Residues

The catalytic action of a complementary strand in ligation reaction using two substrates with 10 residues, including RNA and a 2'-O-methyl group as a modified nucleic acid, was investigated. The complementary strand was designed to form a double strand in a region with 9 residues from a ligation site toward each of a 5' side and a 3' side. As destabilizing sites, an abasic site was introduced into the position of a complementary strand, paired with the base (A, position +1) of the ligation site of the 3'-end substrate, and loop structures were introduced into the 5' end side (position of the third base from the upstream side) and 3' end side (position of the third base from the downstream side) of the complementary strand, or these destabilizing sites were introduced in combination. Oligonucleotides used are set forth in Table 18.

Ligation reaction of oligonucleotides (5'-end substrates and 3'-end substrates) which were substrates was performed using each complementary strand, and generation of products (oligonucleotides in which the 5'-end substrates and the 3'-end substrates were ligated) was compared. The composition of the reaction liquid includes 20 µM of each substrate, 2 µM of a complementary strand, 150 units/µL of T3 DNA ligase (New England Biolabs), 66 mM of Tris-HCl, 10 mM of MgCl₂, 1 mM of ATP, 1 mM of DTT, and 7.5% of polyethylene glycol (PEG 6000) at pH 7.6 (at 25°C), and the reaction was performed at 25°C. The product was quantified after 72 hours under the HPLC conditions described in Example 1, and the yield of the product was calculated. In the present example, the molar concentration of each of the complementary strands was 1/10 of the molar concentration of each substrate, and therefore, the maximum yield was 10% in a case in which each complementary strand was supposed to be used only once in the ligation reaction.

The results are indicated in Figure 14. The yields after 72 hours were 17.5% in the case of using a completely complementary strand as a control and 18.9% in the case of using the complementary strand into which the abasic site was introduced, whereas the yields after 72 hours were 33.0% in the case of using the complementary strand into which the abasic site and the 5'-side loop structure were introduced, 20.8% in the case of using the complementary strand into which the abasic site and the 3'-side loop structure were introduced, and 11.0% in the case of using the complementary strand into which the abasic site and the 5'- and 3'-side loop structures were introduced. In other words, in the ligation reaction of the oligonucleotides including the modified nucleic acid and the RNA, the catalytic action of the complementary strand was also confirmed, and the effect of combining the destabilizing sites was also confirmed.

**[Table 18]**

| Table 18 Oligonucleotides Used | | |
|---|---|---|
| Application | Base sequence | SEQ ID NO: |
| 5'-End substrate | U(M)C(M)U(M)U(M)G(M)guuac | 73 |
| 3'-End substrate | pho-augaaA(M)U(M)C(M)C(M)C(M) | 74 |
| Complementary strand (control: completely complementary) | GGATTTCATGTAACCAAG | 4 |
| Complementary strand (abasic site) | GGATTTCA(Ab)GTAACCAAG | 5 |
| Complementary strand (abasic site + loop structure (5' side)) | GGATTAATTTCA(Ab)GTAACCAAG | 6 |
| Complementary strand (abasic site + loop structure (3' side)) | GGATTTCA(Ab)GTAACCGGCCAAG | 7 |
| Complementary strand (abasic site + loop structure (5' side and 3' side)) | GGATTAATTTCA(Ab)GTAACCGGCCAAG | 8 |

| | | |
|---|---|---|
| Capital letter: indicating DNA residue. Small letter: indicating RNA residue. (M): indicating modification by 2'-O-methyl group. pho: indicating modification of 5' end with phosphate group. (Ab): indicating abasic site. Residues enclosed in rectangle: indicating inserted residues. | | |

### Industrial Applicability

Oligonucleotides can be efficiently ligated by the present invention.

## Claims

1. A method of producing an oligonucleotide of interest, the method comprising a step of performing enzymatic ligation of N single-stranded substrate oligonucleotides, the N being an integer 2 or more, in presence of M single-stranded complementary oligonucleotides, the M being an integer 1 or more, to generate the oligonucleotide of interest,
wherein ligation between the substrate oligonucleotides on 5' and 3' sides of each of ligation sites is performed in presence of the one or more complementary oligonucleotides corresponding to the ligation sites,
wherein each of the complementary oligonucleotides comprises a base sequence in which a destabilizing site is introduced into a first base sequence,
wherein the first base sequence is a base sequence consisting of a second base sequence and a third base sequence that are ligated in 5' to 3' direction,
wherein the second base sequence is a complementary sequence of a partial sequence on a 5' side of the substrate oligonucleotide on the 3' side, or a complementary sequence of a full-length sequence of the substrate oligonucleotide on the 3' side,
wherein the third base sequence is a complementary sequence of a partial sequence on a 3' side of the substrate oligonucleotide on the 5' side, or a complementary sequence of a full-length sequence of the substrate oligonucleotide on the 5' side, and
wherein the destabilizing site is a moiety that lowers stability of a hybrid formed between each of the complementary oligonucleotide and the oligonucleotide of interest.

2. The method according to claim 1, wherein the method excludes cases (1) to (5) described below:
(1) a case in which the N is 2, and the destabilizing site consists of an abasic site at position -1;
(2) a case in which the N is 2, and the destabilizing site consists of a substitution of a linker between positions -1 and +1 with a nucleic acid residue;
(3) a case in which the N is 2, and the destabilizing site consists of a substitution of a nucleic acid residue at position -1 with a linker;
(4) a case in which the N is 2, and the destabilizing site consists of a combination of an abasic site at position -1 and an abasic site at position +4; and
(5) a case in which the N is 2, and the destabilizing site consists of a combination of an abasic site at position -1 and a mismatch site at position +4.

3. The method according to claim 1 or 2, wherein the destabilizing site comprises an abasic site, a mismatch site, an insertion of a nucleic acid residue, a deletion of a nucleic acid residue, an insertion of a linker, a deletion of a linker, a substitution of a linker, or a combination thereof.

4. The method according to any one of claims 1 to 3, wherein the destabilizing site comprises an insertion of a nucleic acid residue without a deletion of a linker and/or a deletion of a nucleic acid residue without an insertion of a linker.

5. The method according to any one of claims 1 to 4, wherein the destabilizing site consists of one to twenty sets of destabilizing sites.

6. The method according to any one of claims 1 to 5, wherein the destabilizing site consists of one to eight sets of destabilizing sites.

7. The method according to any one of claims 1 to 6, wherein the destabilizing site consists of one to four sets of destabilizing sites.

8. The method according to any one of claims 1 to 7, wherein the destabilizing site consists of destabilizing sites whose number of sets is 30% or less as a proportion with respect to a length of the first base sequence.

9. The method according to any one of claims 1 to 8, wherein the destabilizing site comprises one to eight abasic sites.

10. The method according to any one of claims 1 to 9, wherein the destabilizing site comprises one to two abasic sites.

11. The method according to any one of claims 1 to 10, wherein the destabilizing site comprises abasic sites whose number is 30% or less as a proportion with respect to the length of the first base sequence.

12. The method according to any one of claims 1 to 11, wherein the destabilizing site comprises one to eight mismatch sites.

13. The method according to any one of claims 1 to 12, wherein the destabilizing site comprises one to three mismatch sites.

14. The method according to any one of claims 1 to 13, wherein the destabilizing site comprises mismatch sites whose number is 30% or less as a proportion with respect to the length of the first base sequence.

15. The method according to any one of claims 1 to 14, wherein the destabilizing site comprises deletions of nucleic acid residues whose number is 50% or less as a proportion with respect to the length of the first base sequence.

16. The method according to any one of claims 1 to 15, wherein the destabilizing site comprises one to eight sets of deletions of nucleic acid residues.

17. The method according to any one of claims 1 to 16, wherein the destabilizing site comprises one to three sets of deletions of nucleic acid residues.

18. The method according to claim 16 or 17, wherein each set of the deletions of the nucleic acid residues consists of one to three deletions of nucleic acid residues.

19. The method according to any one of claims 1 to 18, wherein the destabilizing site comprises insertions of nucleic acid residues whose number is 200% or less as a proportion with respect to the length of the first base sequence.

20. The method according to any one of claims 1 to 19, wherein the destabilizing site comprises one to eight sets of insertions of nucleic acid residues.

21. The method according to any one of claims 1 to 20, wherein the destabilizing site comprises one to four sets of insertions of nucleic acid residues.

22. The method according to claim 20 or 21, wherein each set of the insertions of the nucleic acid residues consists of one to ten insertions of nucleic acid residues.

23. The method according to any one of claims 20 to 22, wherein in a case in which each set of the insertions of the nucleic acid residues consists of two or more insertions of nucleic acid residues, a part or all of the nucleic acid residues have self-complementarity.

24. The method according to any one of claims 1 to 23, wherein the destabilizing site comprises one to eight insertions of linkers.

25. The method according to any one of claims 1 to 24, wherein the destabilizing site comprises one to eight deletions of linkers.

26. The method according to any one of claims 1 to 25, wherein the destabilizing site comprises one to eight substitutions of linkers.

27. The method according to any one of claims 1 to 26, wherein the destabilizing site comprises (a), (b), or (c) described below:
(a) a combination of an abasic site at positions -1 to +1 and a deletion and/or insertion of a nucleic acid residue at a position other than the positions -1 to +1;
(b) a combination of a mismatch site at positions -1 to +1 and a deletion and/or insertion of a nucleic acid residue at a position other than the positions -1 to +1; or
(c) a mismatch site at positions -1 to +1.

28. The method according to claim 27, wherein the destabilizing sites (a), (b), and (c) are (a1), (b1), and (c1) described below, respectively:
(a1) a combination of an abasic site at positions -1 to +1 and one or two sets of deletions of nucleic acid residues and/or one or two sets of insertions of nucleic acid residues at positions other than the positions -1 to +1;
(b1) a combination of a mismatch site at positions -1 to +1 and one or two sets of deletions of nucleic acid residues and/or one or two sets of insertions at positions other than the positions -1 to +1; and
(c1) a mismatch site at positions -1 to +1.

29. The method according to claim 27, wherein the destabilizing sites (a), (b), and (c) are (a2), (b2), and (c2) described below, respectively:
(a2) a combination of an abasic site at positions -1 to +1, one set of insertions of nucleic acid residues at negative positions other than the position -1, and one set of insertions of nucleic acid residues at positive positions other than the position +1;
(b2) a combination of a mismatch site at positions -1 to +1, one set of insertions of nucleic acid residues at negative positions other than the position -1, and one set of insertions of nucleic acid residues at positive positions other than the position +1; and
(c2) a mismatch site at positions -1 to +1.

30. The method according to any one of claims 1 to 29, wherein the destabilizing site lowers a melting temperature of the hybrid by 1 to 60°C.

31. The method according to any one of claims 1 to 30, wherein the oligonucleotide of interest has a length of 10 to 200 residues.

32. The method according to any one of claims 1 to 31, wherein each of the substrate oligonucleotides has a length of 5 to 50 residues.

33. The method according to any one of claims 1 to 32, wherein each of the second and third base sequences has a length of 5 to 50 residues.

34. The method according to any one of claims 1 to 33, wherein a length of each of the complementary oligonucleotides is 5 to 300% as a proportion in a case in which the length of the oligonucleotide of interest is taken as 100%.

35. The method according to any one of claims 1 to 34, wherein the length of each of the complementary oligonucleotides is 5 to 300% as a proportion in a case in which a total of the lengths of the substrate oligonucleotides on the 5' side and the 3' side is taken as 100%.

36. The method according to any one of claims 1 to 35, wherein five or more residues of nucleic acid residues included in the second base sequence remain, and five or more residues of nucleic acid residues included in the third base sequence remain in each of the complementary oligonucleotides.

37. The method according to any one of claims 1 to 36, wherein 50% or more of the nucleic acid residues included in the second base sequence remain, and 50% or more of the nucleic acid residues included in the third base sequence remain in each of the complementary oligonucleotides.

38. The method according to any one of claims 1 to 37, wherein three or more consecutive residues of the nucleic acid residues included in the second base sequence remain, and three or more consecutive residues of the nucleic acid residues included in the third base sequence remain in each of the complementary oligonucleotides.

39. The method according to any one of claims 1 to 38, wherein the oligonucleotide of interest consists of a DNA residue, an RNA residue, a nucleic acid residue subjected to a modification, or a combination thereof.

40. The method according to any one of claims 1 to 39, wherein the oligonucleotide of interest is subjected to a modification.

41. The method according to any one of claims 1 to 40, wherein any one or more of the complementary oligonucleotides are subjected to a modification.

42. The method according to any one of claims 39 to 41, wherein the modification comprises a modification of a phosphate portion, a modification of a sugar portion, a modification of a base portion, or a combination thereof.

43. The method according to claim 42, wherein the modification of the phosphate portion comprises phosphorothioation, boranophosphation, insertion of a linker, or a combination thereof.

44. The method according to claim 42 or 43, wherein the modification of the sugar portion comprises 2'-MOE, 2'-OMe, 2'-F, 4'-thio- 2'-OMe, crosslinkage between positions 2' and 4' of a sugar portion, a modification of a 5' end of an oligonucleotide, a modification of a 3' end of an oligonucleotide, or a combination thereof.

45. The method according to any one of claims 1 to 44, wherein the N is 2.

46. The method according to any one of claims 1 to 45, wherein the N is 3 or more.

47. The method according to any one of claims 1 to 46, wherein the N is 10 or less.

48. The method according to any one of claims 1 to 47, wherein the M is less than N - 1.

49. The method according to any one of claims 1 to 47, wherein the M is N - 1.

50. The method according to any one of claims 1 to 47, wherein the M is more than N - 1.

51. The method according to any one of claims 1 to 50, wherein the step is carried out at 5 to 60°C.

52. The method according to any one of claims 1 to 51, wherein a used amount of each of the complementary oligonucleotides is 50% or less, by molar ratio, of a used amount of the substrate oligonucleotide on the 5' side or a used amount of the substrate oligonucleotide on the 3' side, whichever is less.

53. The method according to any one of claims 1 to 52, wherein a concentration of each of the substrate oligonucleotides in a reaction liquid in the step is 1 to 10000 µM.

54. The method according to any one of claims 1 to 53, wherein an enzyme used in the enzymatic ligation is a T3 DNA ligase.
